# EUROPEAN PATENT APPLICATION

(11) **EP 2 589 397 A1**
(43) Date of publication of application: **08.05.2013**
(21) Application number: 11800655.0
(22) Date of filing: 20.06.2011
(51) Int. Cl.: A61M 5/142, A61M 5/32, A61M 37/00

(54) **DRUG INJECTION APPARATUS AND DRUG CONTAINER**

(30) Priority: 28.07.2010 JP 2010169518; 30.06.2010 JP 2010150494; 30.06.2010 JP 2010150493
(71) Applicant: Terumo Kabushiki Kaisha, Tokyo 151-0072 (JP)
(72) Inventor: YOSHINO Keisuke, Ashigarakami-gun Kanagawa 259-0151 (JP); KOYAMA Miyuki, Ashigarakami-gun Kanagawa 259-0151 (JP)
(74) Representative: TBK
(86) International application number: PCT/JP2011/064061
(87) International publication number: WO 2012/002190

(57) **Abstract**

The present invention provides a drug injection apparatus in which it is possible to press a flexible drug container to discharge a drug from an injection needle while stably holding the drug injection apparatus in a state where the injection needle has been stuck into a living body. The drug injection apparatus of the present invention includes a drug container, a needle tube, a needle-holding portion, and a drug container mount. The needle tube has a first needle tip and a second needle tip, and the central portion of the needle tube is held by the needle-holding portion. The second needle tip projects from the drug container mount. The drug container has a container body, a seal member and a protective plate. The container body has a contact portion to be penetrated by the second needle tip and a pressing portion that faces the contact portion, wherein the pressing portion is to be pressed toward the side of the contact portion so as to deform. The seal member is attached to a portion of the contact portion to be penetrated by the second needle tip, so that the seal member closely contacts the circumferential surface of the needle tube in a liquid-tight manner. The protective plate is provided to avoid the pressing portion from being penetrated by the second needle tip inserted into the container body.

## Description

### Technical Field

The present invention relates to a flexible drug container having a drug enclosed therein, and a drug injection apparatus having the drug container.

### Background Art

In recent years, a prefilled syringe having a drug previously filled therein becomes widely used. With such a prefilled syringe, when administering a drug, since it is not necessary to draw a drug from a vial into a syringe, not only the time required for performing administration can be reduced, but also waste of the drug can be reduced.

Further, instead of the prefilled syringe, an injection apparatus using a flexible drug container has been developed (see Patent Documents 1 and 2). When using such an injection apparatus, first, the drug container is brought into communication with an injection needle, and then the drug container is pressed to thereby discharge the drug enclosed in the drug container to the outside from the injection needle.

[Patent Document 1] Japanese Unexamined Patent Application Publication No. 2001-137343
[Patent Document 2] Japanese Unexamined Patent Application Publication No. H10-509335

### Disclosure of the Invention

### Problems to be Solved by the Invention

However, in the injection apparatus disclosed in Patent Documents 1 and 2, since the flexible drug container is deformable in a direction substantially perpendicular to the extending direction of the injection needle, the direction in which the injection needle is stuck into a living body is different from the direction in which the drug container is pressed. Thus, when administering drug, particularly when performing administration with one hand, it is difficult to discharge the drug enclosed in the drug container from the injection needle while stably holding the injection apparatus in a state where the injection apparatus has been stuck into the living body.
Particularly, in the case where the drug is administered to a site such as the intradermal layer where back-pressure is high, since puncture depth of the injection needle is not stable, it is difficult to administer the drug to the target site, and therefore desired effect of the administration of the drug can not be achieved.

In view of the aforesaid problems, it is an object of the present invention to provide a drug injection apparatus in which it is possible to press the flexible drug container to discharge the drug from the injection needle while stably holding the drug injection apparatus in a state where the injection needle has been stuck into a living body.

### Means for Solving the Problems

To achieve the aforesaid object, a drug injection apparatus according to an aspect of the present invention comprises: a drug container having a drug filled therein, a needle tube, a needle-holding portion, and a drug container mount. The needle tube has a first needle tip to be stuck into a living body and a second needle tip to be inserted into the drug container, and a central portion of the needle tube is held by the needle-holding portion. The drug container mount is arranged on the second needle tip side of the needle-holding portion, and the second needle tip projects from the drug container mount.
The drug container comprises a container body, a seal member, and a penetration avoiding portion. The container body has a contact portion to be penetrated by the second needle tip and to be brought into contact with the drug container mount, and a pressing portion that faces the contact portion, wherein the pressing portion is to be pressed toward the side of the contact portion so as to deform. The seal member is attached to a portion of the contact portion to be penetrated by the second needle tip, and is adapted to be brought into close contact with the circumferential surface of the needle tube in a liquid-tight manner. The penetration avoiding portion is adapted to avoid the pressing portion from being penetrated by second needle tip inserted into the container body.

In the aforesaid drug injection apparatus, when the second needle tip of the needle tube is penetrated through the contact portion of the container body of the drug container, the inside of the container body will be brought into communication with the inside of the needle tube. Further, when the pressing portion of the container body is pressed toward the side of the contact portion, the drug filled in the container body will be discharged to the outside from the first needle tip of the needle tube. Thus, the direction in which the container body is pressed is identical to the direction in which the first needle tip of the needle tube is stuck into the living body. As a result, it is possible to press the drug container to discharge the drug from the first needle tip while stably holding the drug injection apparatus in a state where the first needle tip has been stuck into the living body.

Further, due to the presence of the penetration avoiding portion, the case where the second needle tip penetrated through the contact portion is penetrated through the pressing portion is avoided. Thus, it is possible to not only prevent the case where the discharge of the drug from the first needle tip stops, but also prevent the case where the second needle tip is stuck into the fingertip or the like that presses the pressing portion.

A drug injection apparatus according to another aspect of the present invention comprises a drug container, a needle tube, a needle-holding portion, and a drug container mount. The needle tube has a first needle tip to be stuck into a living body and a second needle tip to be inserted into the drug container. The needle-holding portion holds a central portion of the needle tube. The drug container mount is arranged on the second needle tip side of the needle-holding portion, and the drug container is to be mounted on the drug container mount. Further, the drug container comprises a container body, a needle puncture portion, and a seal member. In the container body, a liquid chamber for being filled with the drug is formed by joining a sheet-like first member and a sheet-like second member. The needle puncture portion projects outward from the second member. The second needle tip is to be inserted into the needle puncture portion. The seal member is attached to an end portion of the needle puncture portion, and is adapted to be penetrated by the second needle tip. In the drug container, the first member is pressed toward the second member, and thereby the drug filled in the liquid chamber is discharged through the needle tube penetrated through the seal member and inserted into the needle puncture portion.

In the aforesaid drug injection apparatus, the first member is pressed toward the second member to thereby discharge the drug filled in the liquid chamber of the container body. Thus, the administration of the drug to the living body can be easily performed. Further, when discharging the drug, since the second needle tip is located in the needle puncture portion, the first member pressed toward the side of the second member will not be penetrated by the second needle tip.

A drug container according to further another aspect of the present invention comprises a container body, a needle puncture portion, and a seal member. In the container body, a liquid chamber for being filled with a drug is formed by joining a sheet-like first member and a sheet-like second member. The needle puncture portion projects from the second member. A needle tube is to be inserted into the needle puncture portion. The seal member is attached to an end portion of the needle puncture portion and is adapted to be penetrated by the needle tube. In the drug container, the first member is pressed toward the second member, and thereby the drug filled in the liquid chamber is discharged through the needle tube penetrated through the seal member and inserted into the needle puncture portion.

In the aforesaid drug container, the first member is pressed toward the second member to thereby discharge the drug filled in the liquid chamber of the container body. Thus, the administration of the drug can be easily performed. Further, when discharging the drug, since the needle tip of the needle tube is located in the needle puncture portion, the first member pressed toward the side of the second member will not be penetrated by the needle tip of the needle tube.

### Advantages of the Invention

With the aforesaid drug injection apparatus, it is possible to press the flexible drug container to discharge the drug from the injection needle while stably holding the drug injection apparatus in a state where the injection needle has been stuck into the living body.

### Brief Description of Drawings

FIG. 1 is a cross-sectional view showing the configuration of a drug injection apparatus according to a first embodiment of the present invention;
FIG. 2 is a cross-sectional view showing a state where a drug container is mounted on an injection needle assembly of the drug injection apparatus of the first embodiment, and a first needle tip is stuck into a living body;
FIG. 3 is a cross-sectional view showing a state where a second needle tip of a needle tube of the drug injection apparatus of the first embodiment has been penetrated through a contact portion of the drug container;
FIG. 4 is a cross-sectional view showing a state where drug administration of the drug injection apparatus of the first embodiment has been completed;
FIG. 5 is a cross-sectional view showing the configuration of a drug injection apparatus according to a second embodiment of the present invention;
FIG. 6 is a cross-sectional view showing a state where a drug container is mounted on an injection needle assembly of the drug injection apparatus of the second embodiment, and a first needle tip is stuck into a living body;
FIG. 7 is a cross-sectional view showing a state where a second needle tip of the injection needle assembly of the drug injection apparatus of the second embodiment has been penetrated through a contact portion of the drug container;
FIG. 8 is a cross-sectional view showing a state where drug administration of the drug injection apparatus of the second embodiment has been completed;
FIG. 9 is a cross-sectional view showing the configuration of a drug injection apparatus according to a third embodiment of the present invention;
FIG. 10 is a cross-sectional view showing a state where a drug container is mounted on an injection needle assembly of the drug injection apparatus of the third embodiment;
FIG. 11 is a cross-sectional view showing a state where a first needle tip of the drug injection apparatus of the third embodiment is stuck into a living body;
FIG. 12 is a cross-sectional view showing a state where drug administration of the drug injection apparatus of the third embodiment has been completed;
FIG. 13 is a cross-sectional view showing the configuration of a drug injection apparatus according to a fourth embodiment of the present invention;
FIG. 14 is an exploded perspective view of a drug container used in the drug injection apparatus of the fourth embodiment.
FIG. 15 is a cross-sectional view showing the usage state of the drug injection apparatus of the fourth embodiment, in a state where a first needle tip is stuck into a living body;
FIG. 16 is a cross-sectional view showing the usage state of the drug injection apparatus of the fourth embodiment, in a state where a second needle tip is inserted into a drug container;

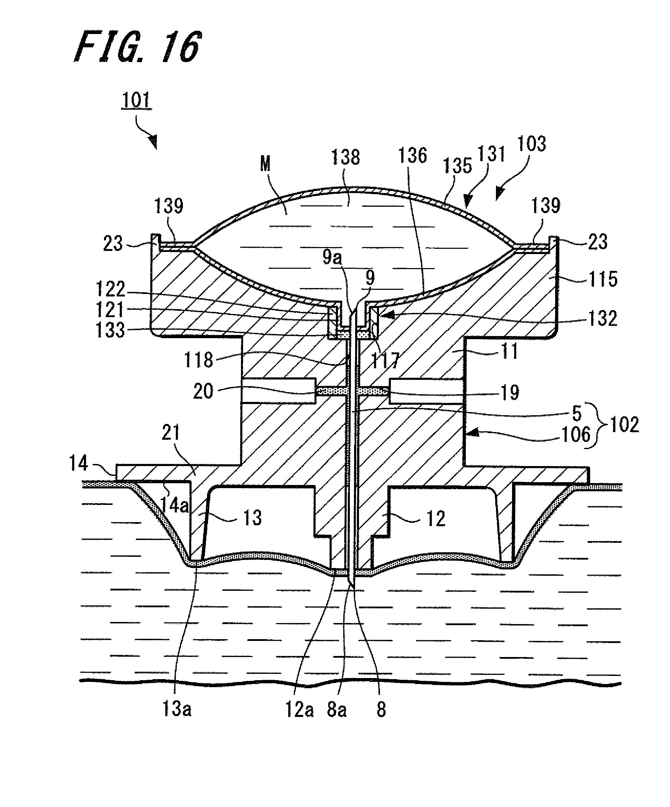

FIG. 16 is a cross-sectional view showing the usage state of the drug injection apparatus of the fourth embodiment, in a state where drug administration has been completed;

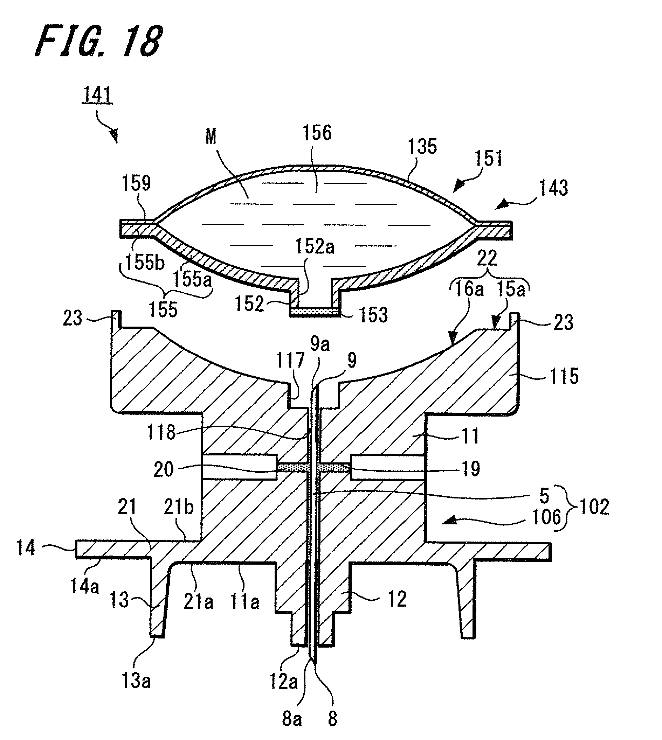

FIG. 18 is a cross-sectional view showing the configuration of a drug injection apparatus according to a fifth embodiment of the present invention;

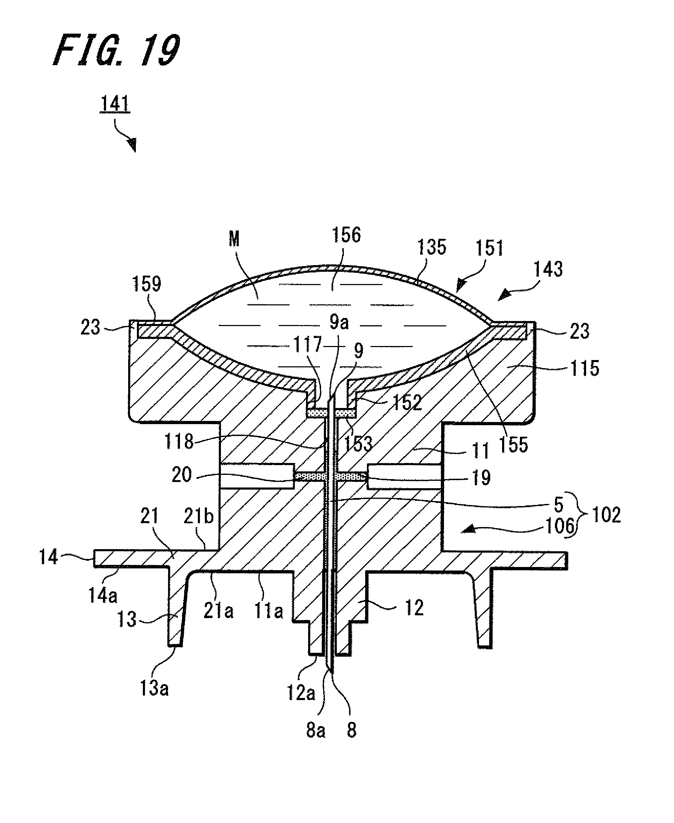

FIG. 19 is a cross-sectional view showing the usage state of the drug injection apparatus of the fifth embodiment, in a state where a second needle tip is inserted into a drug container;

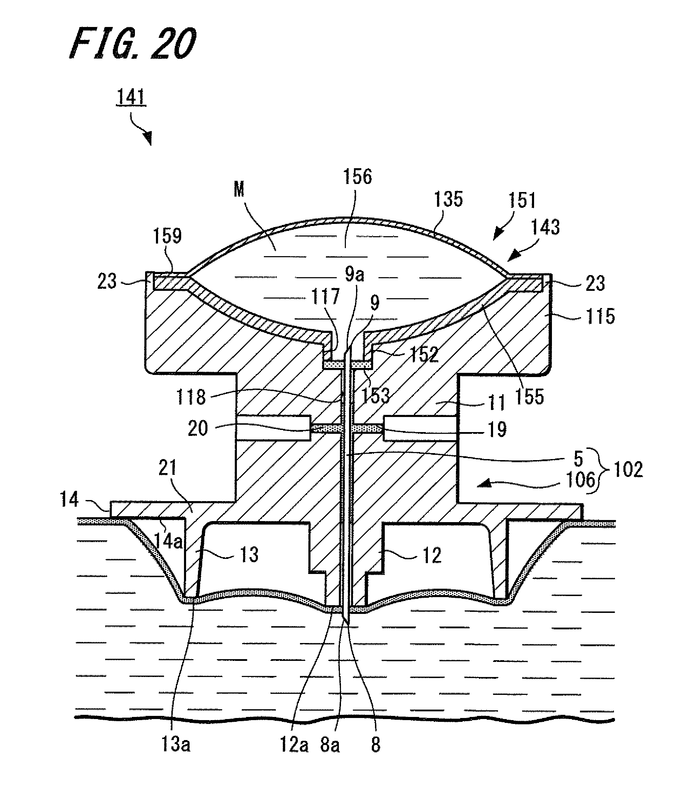

FIG. 20 is a cross-sectional view showing the usage state of the drug injection apparatus of the fifth embodiment, in a state where a first needle tip is stuck into a living body;

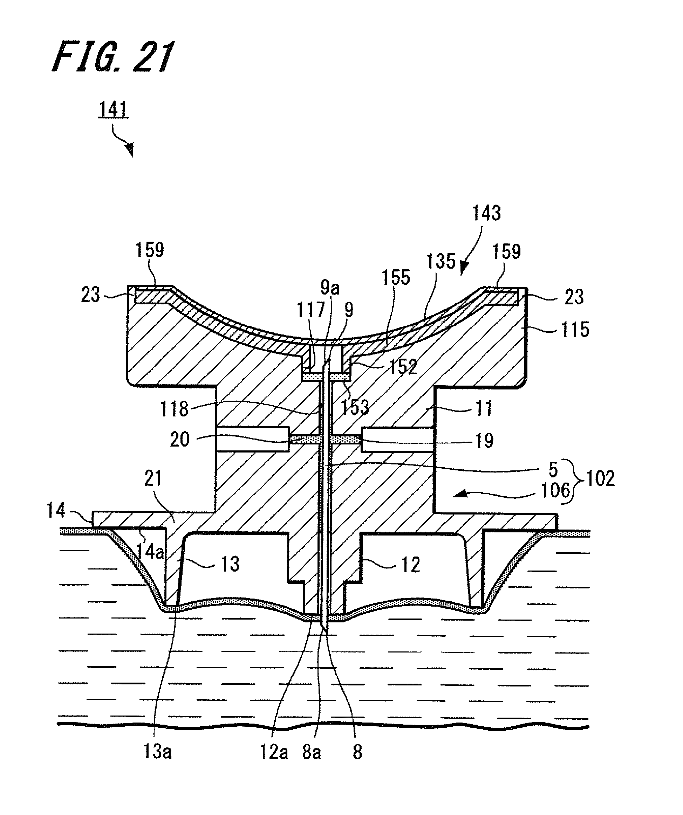

FIG. 21 is a cross-sectional view showing the usage state of the drug injection apparatus of the fifth embodiment, in a state where drug administration has been completed;

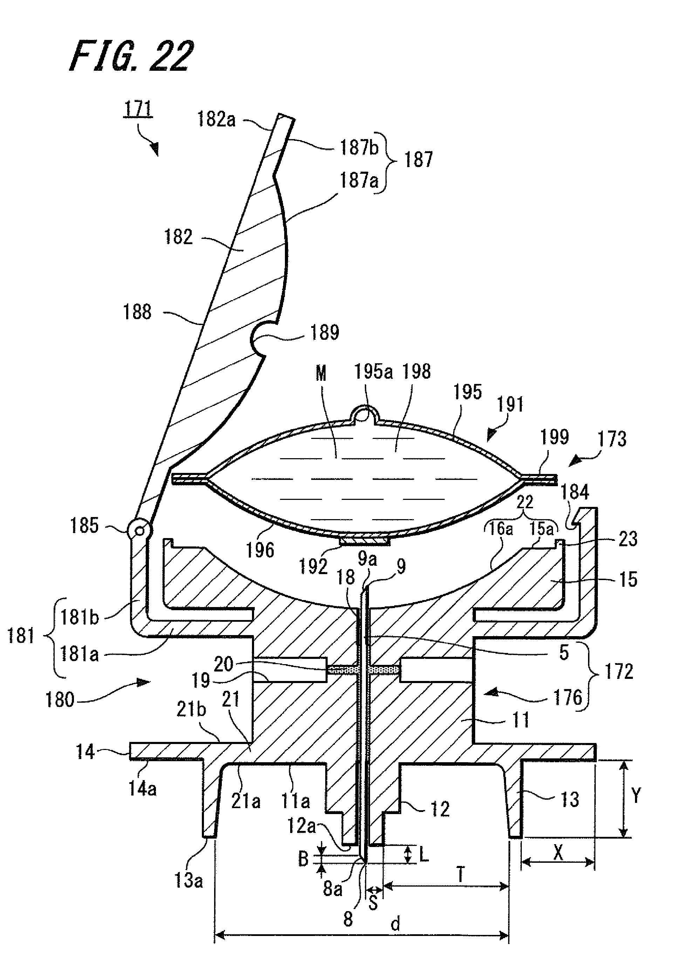

FIG. 22 is a cross-sectional view showing the configuration of a drug injection apparatus according to a sixth embodiment of the present invention;

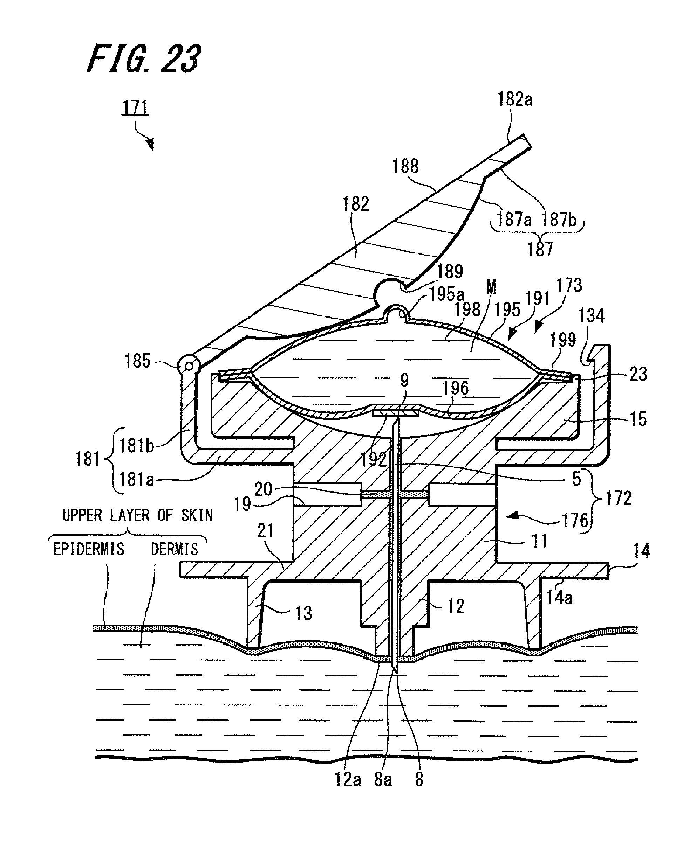

FIG. 23 is a cross-sectional view showing the usage state of the drug injection apparatus of the sixth embodiment, in a state where a first needle tip is stuck into a living body;

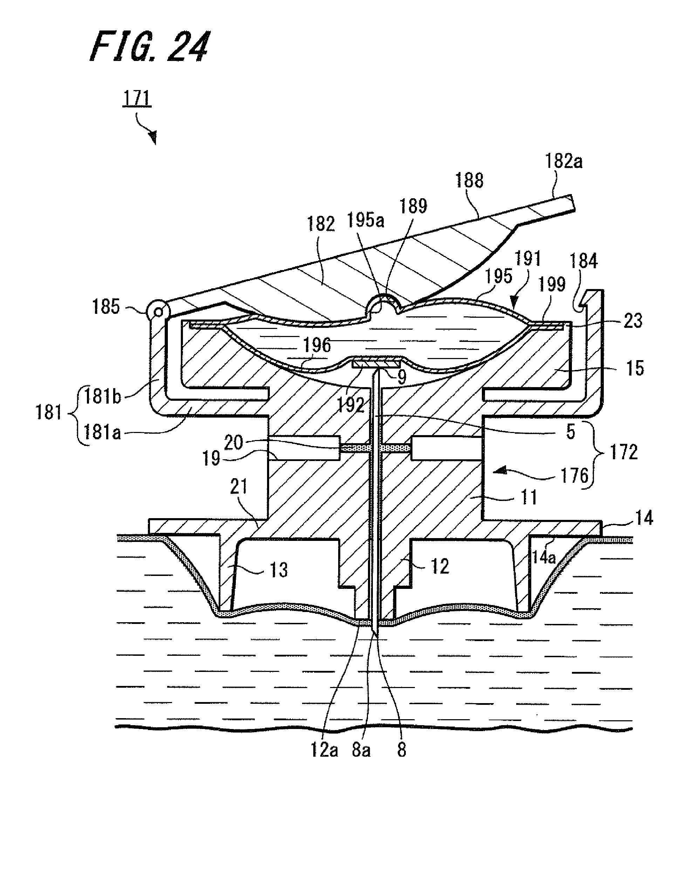

FIG. 24 is a cross-sectional view showing the usage state of the drug injection apparatus of the sixth embodiment, in a state where a second needle tip is inserted into a drug container;

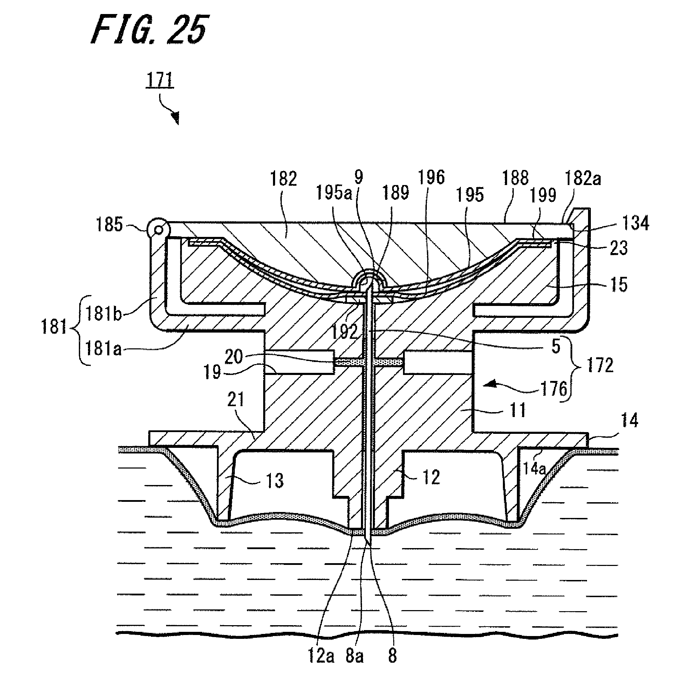

FIG. 25 is a cross-sectional view showing the usage state of the drug injection apparatus of the sixth embodiment, in a state where drug administration has been completed;

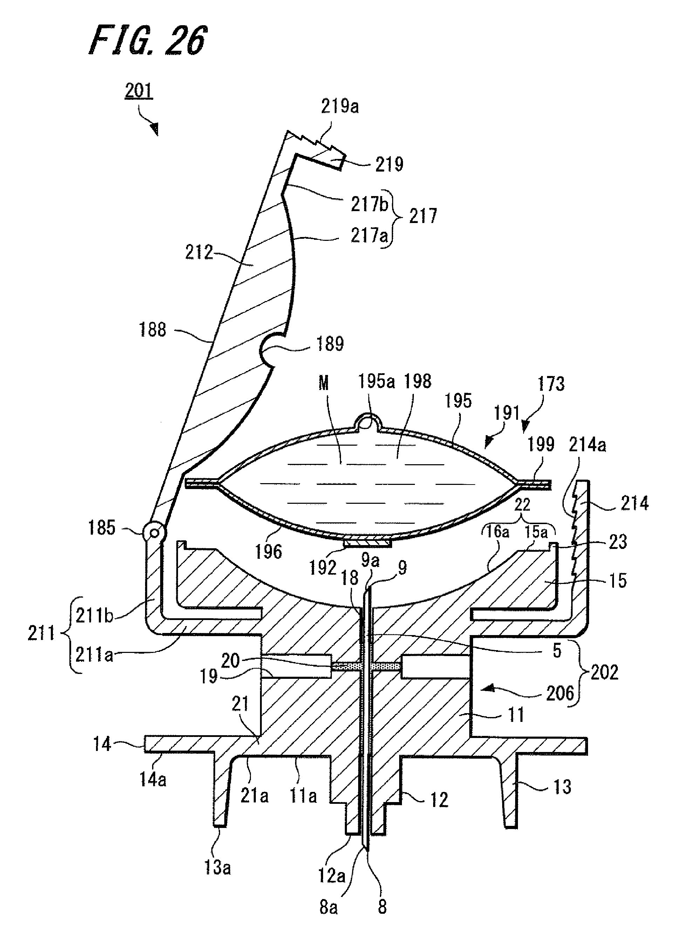

FIG. 26 is a cross-sectional view showing the configuration of a drug injection apparatus according to a seventh embodiment of the present invention;

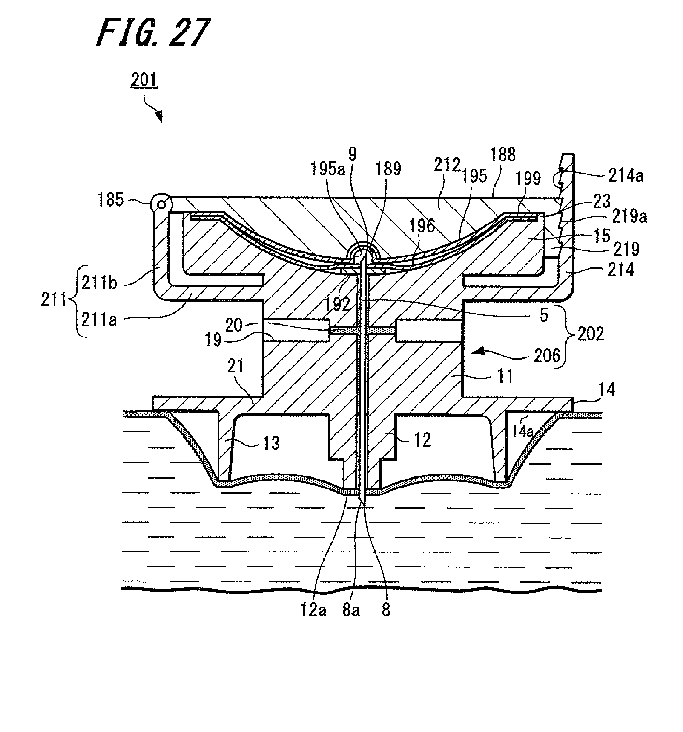

FIG. 27 is a cross-sectional view showing the usage state of the drug injection apparatus of the seventh embodiment, in a state where drug administration has been completed;

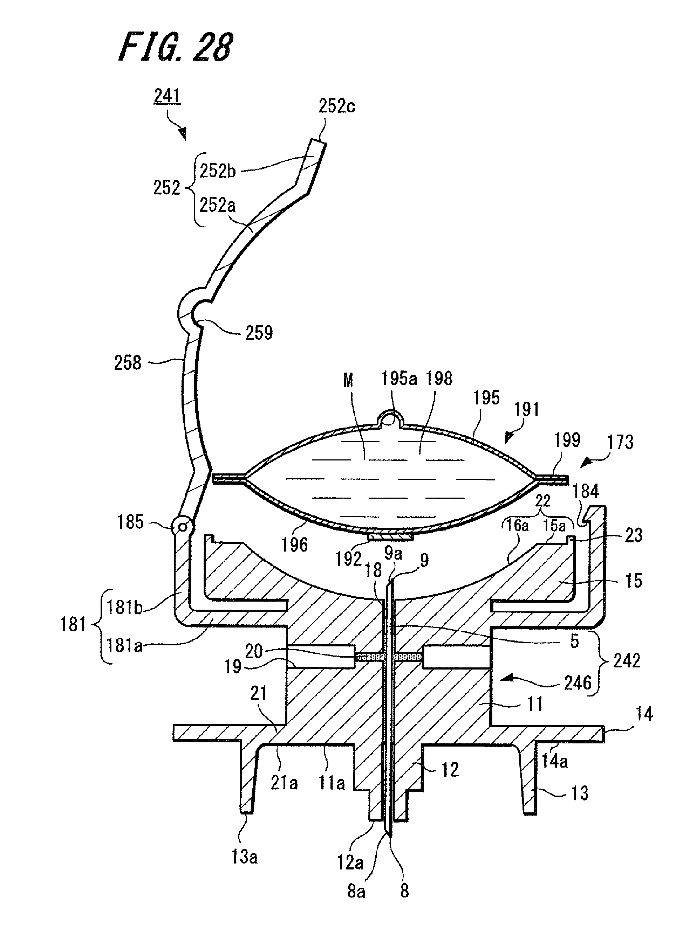

FIG. 28 is a cross-sectional view showing the configuration of a drug injection apparatus according to an eighth embodiment of the present invention;

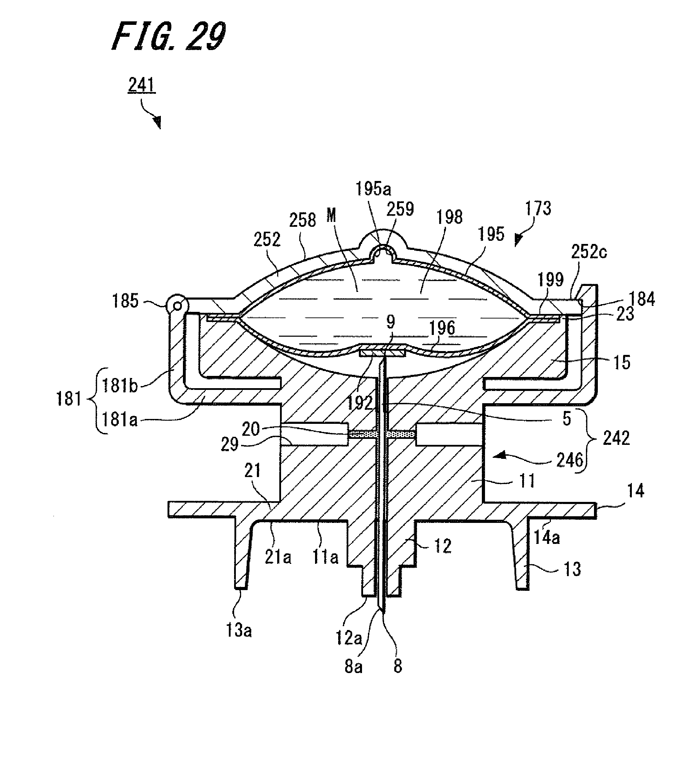

FIG. 29 is a cross-sectional view showing the usage state of the drug injection apparatus of the sixth embodiment, in a state where a drug container is set on the injection needle assembly;

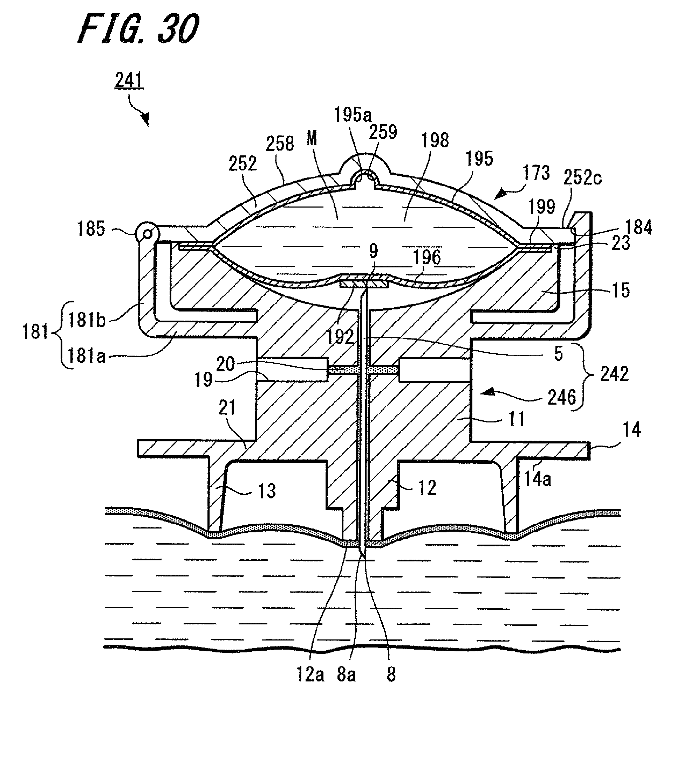

FIG. 30 is a cross-sectional view showing the usage state of the drug injection apparatus of the eighth embodiment, in a state where a first needle tip is stuck into a living body;

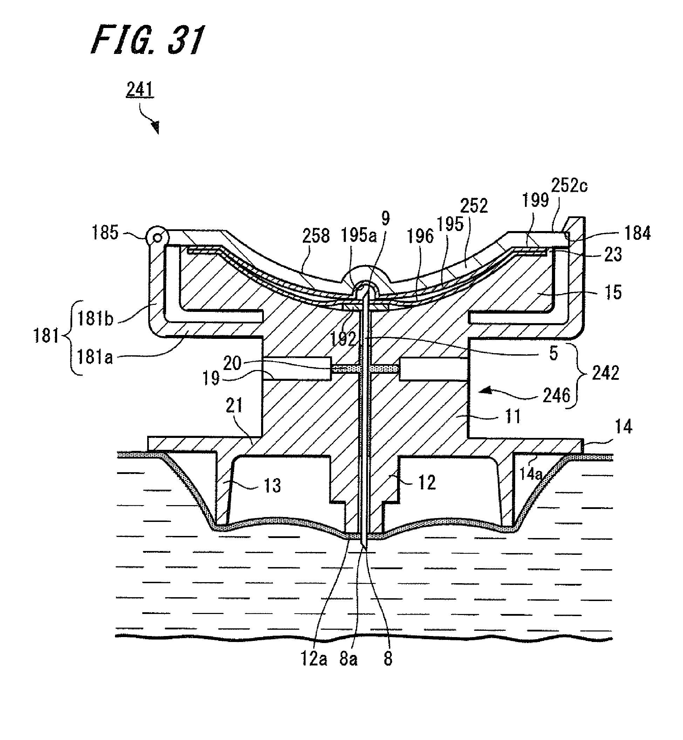

FIG. 31 is a cross-sectional view showing the usage state of the drug injection apparatus of the eighth embodiment, in a state where drug administration has been completed;

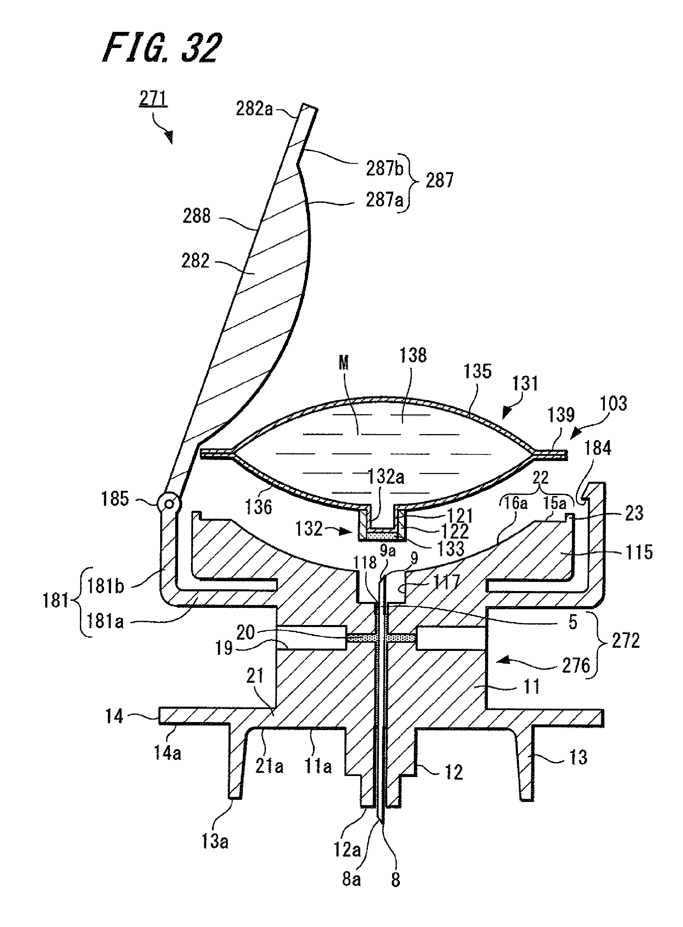

FIG. 32 is a cross-sectional view showing the configuration of a drug injection apparatus according to a ninth embodiment of the present invention;

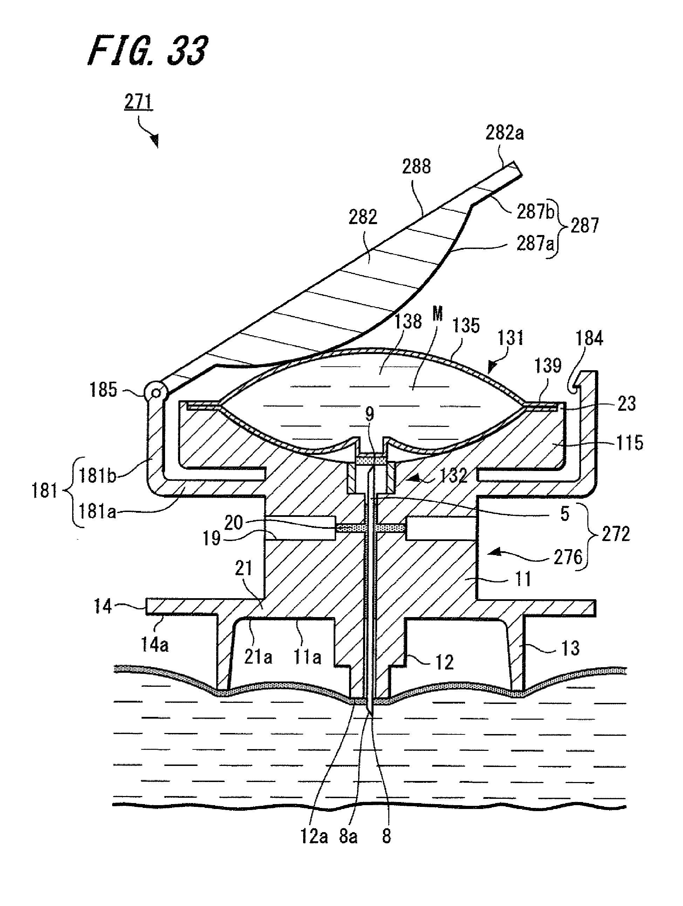

FIG. 33 is a cross-sectional view showing the usage state of the drug injection apparatus of the ninth embodiment, in a state where a first needle tip is stuck into a living body;

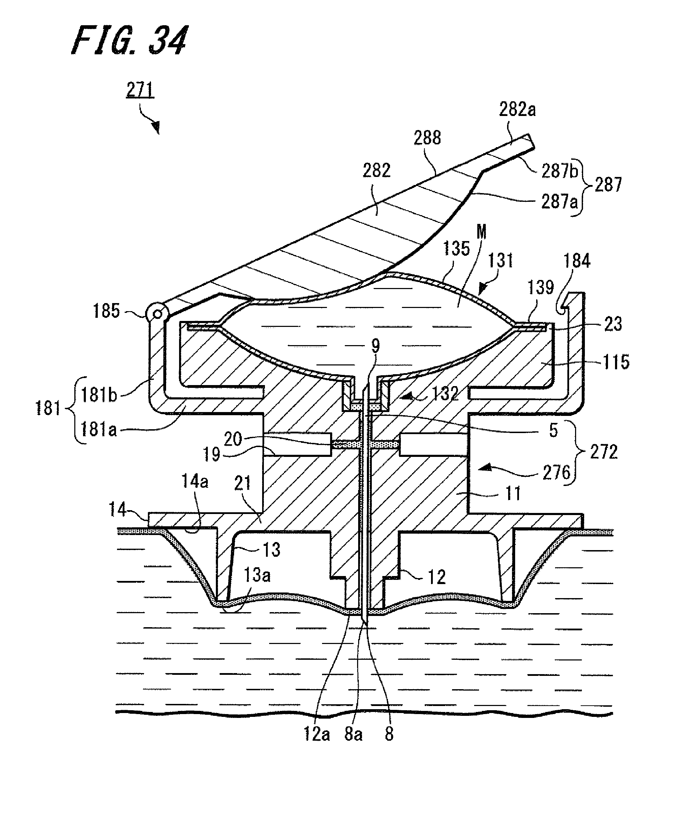

FIG. 34 is a cross-sectional view showing the usage state of the drug injection apparatus of the ninth embodiment, in a state where a second needle tip is inserted into a drug container;

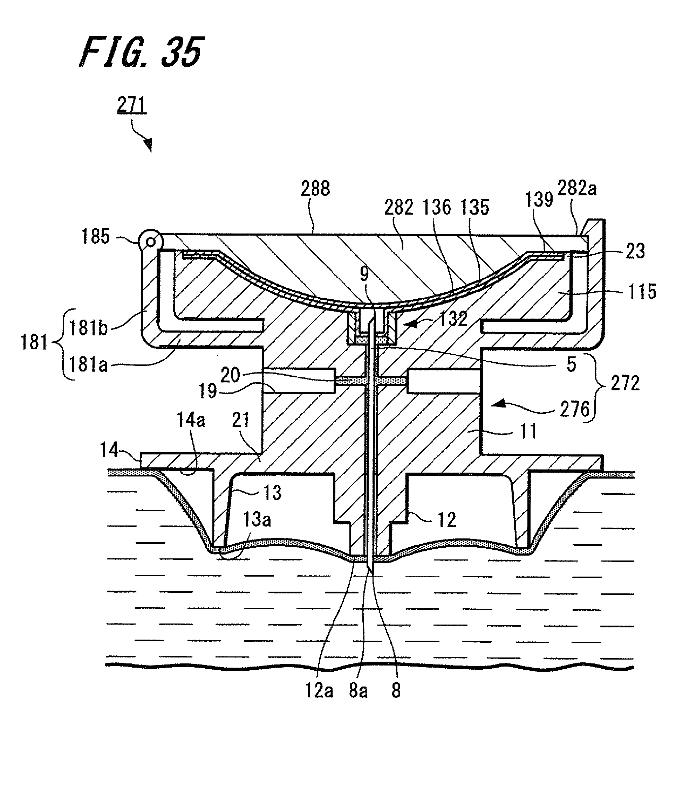

FIG. 35 is a cross-sectional view showing the usage state of the drug injection apparatus of the ninth embodiment, in a state where drug administration has been completed;

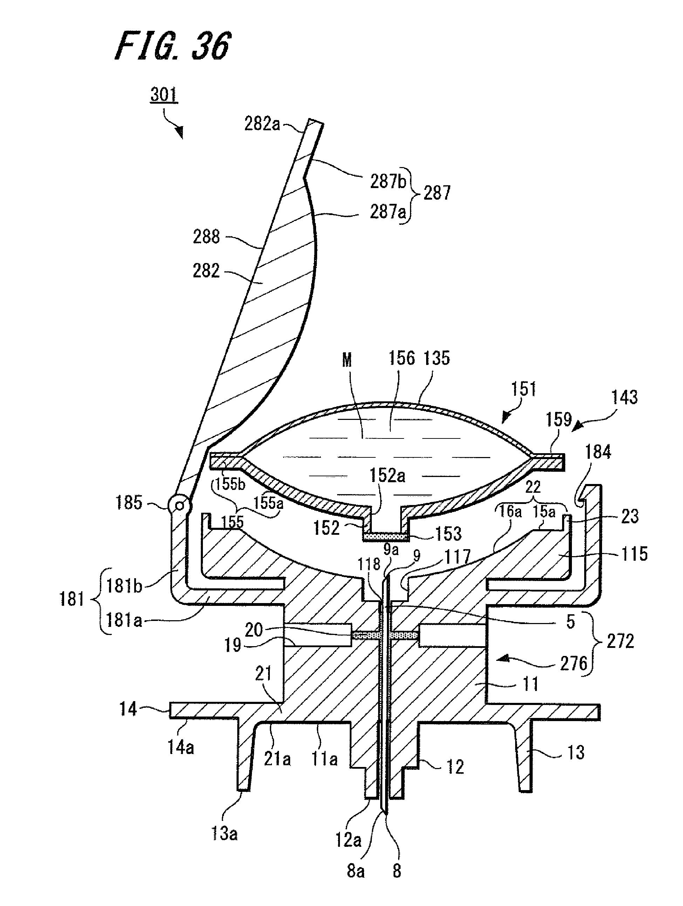

FIG. 36 is a cross-sectional view showing the configuration of a drug injection apparatus according to a tenth embodiment of the present invention;

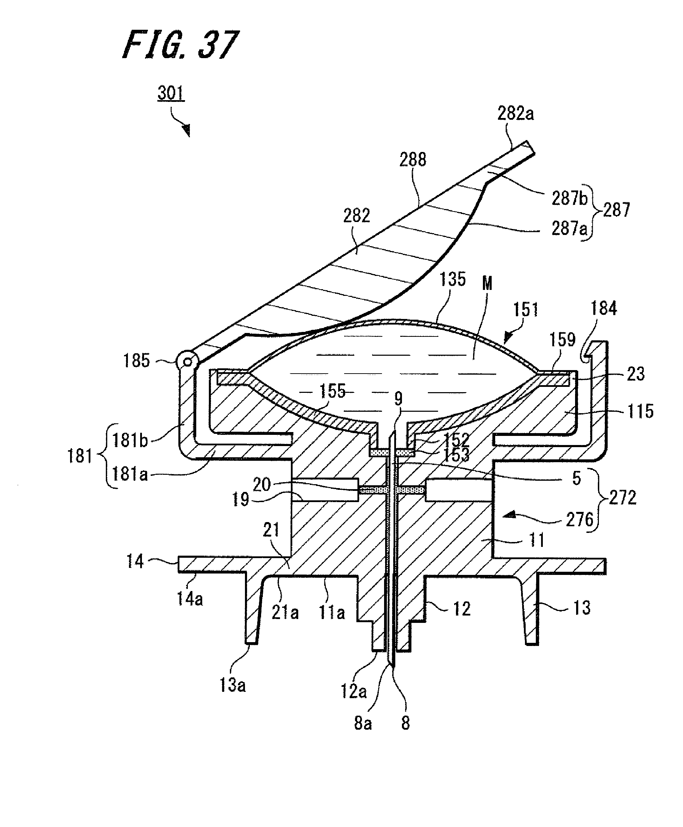

FIG. 37 is a cross-sectional view showing the usage state of the drug injection apparatus of the tenth embodiment, in a state where a second needle tip is inserted into a drug container;

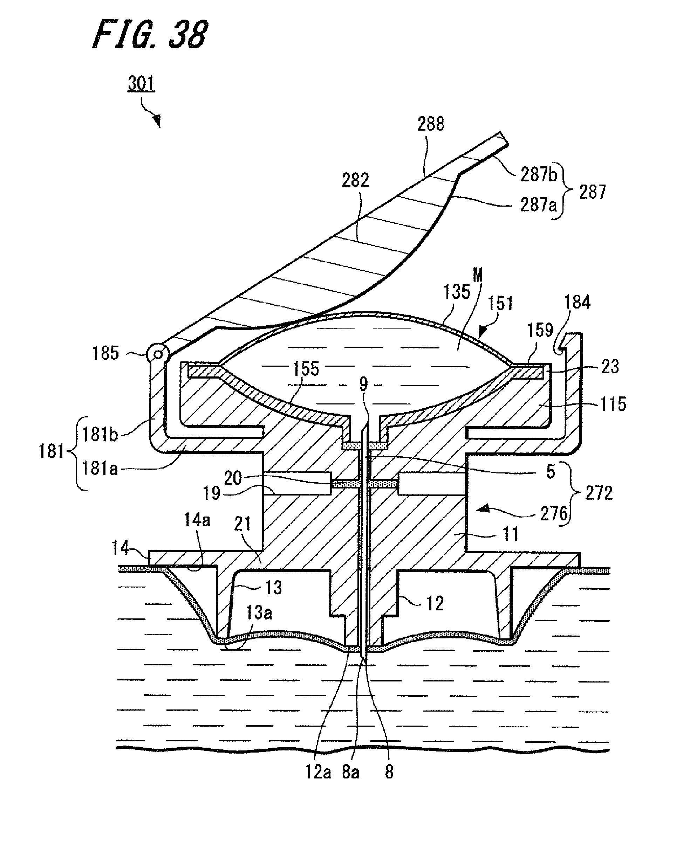

FIG. 38 is a cross-sectional view showing the usage state of the drug injection apparatus of the tenth embodiment, in a state where a first needle tip is stuck into a living body; and

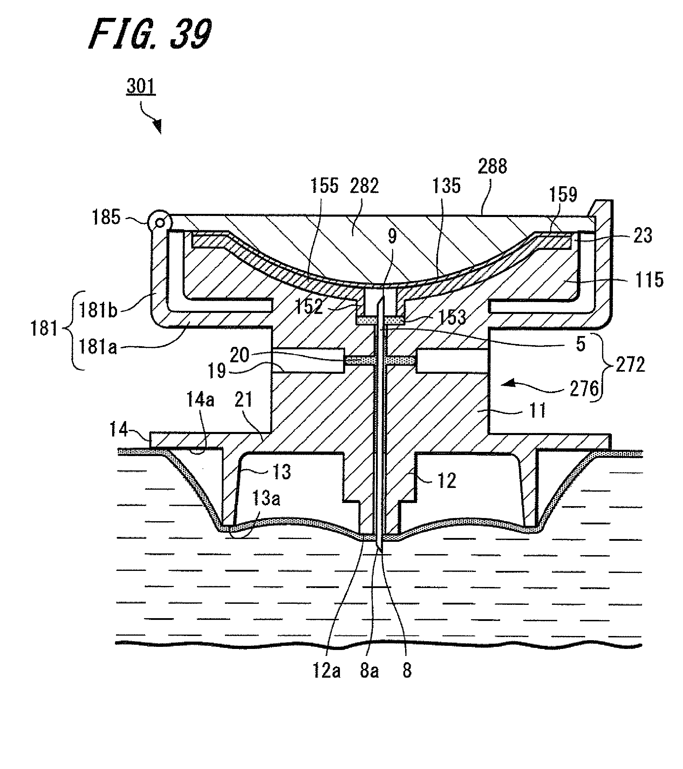

FIG. 39 is a cross-sectional view showing the usage state of the drug injection apparatus of the tenth embodiment, in a state where drug administration has been completed.

### Modes for Carrying Out the Invention

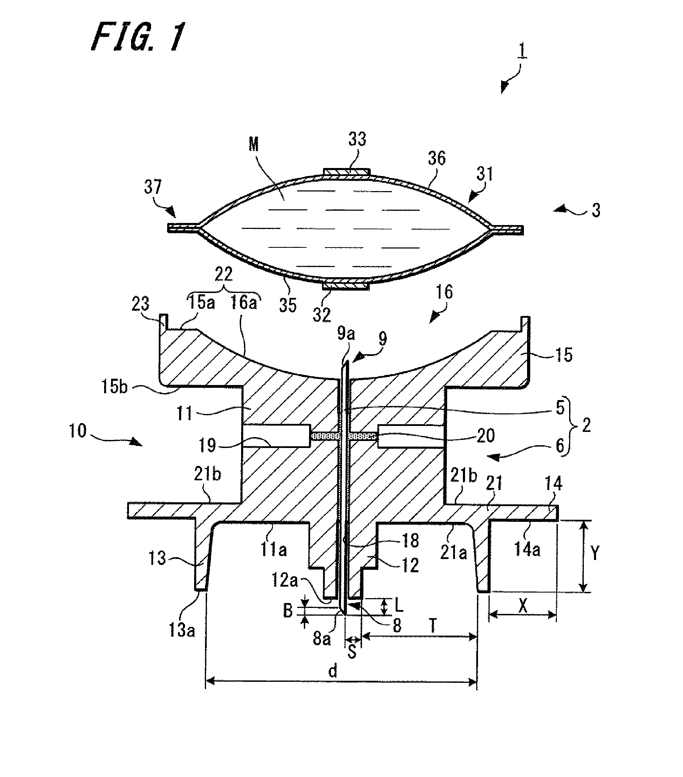

Preferred embodiments of the drug injection apparatus and the drug container of the present invention will be described below with reference to FIG. 1 to 39. Note that, in the drawings, common components are denoted by common reference numerals. Further, the present invention is not limited to the embodiments described below.

### 1. First embodiment

### [Drug injection apparatus]

First, a drug injection apparatus according to a first embodiment of the present invention will be described below with reference to FIG. 1.
FIG. 1 is a cross-sectional view showing the configuration of a drug injection apparatus 1 according to the first embodiment.

As shown in FIG. 1, the drug injection apparatus 1 includes an injection needle assembly 2, and a drug container 3 to be mounted on the injection needle assembly 2. The drug injection apparatus 1 is used to stick a needle tip into a skin from the surface of the skin to inject a drug into an upper layer of the skin.

Skin is composed of three layers: epidermis, dermis and subcutaneous tissue. The epidermis is a layer of about 50-200 µm from the skin surface, and the dermis is a layer of about 1.5-3.5 mm continuing from the epidermis. The upper layer of the skin refers to the epidermis and the dermis of the skin. Incidentally, the dose of the drug such as a vaccine to be administered to the upper layer of the skin is about 50-300 µL, preferably about 100 µL.

### [Injection needle assembly]

The injection needle assembly 2 includes a hollow needle tube 5 having a needle hole, and a needle hub 6 by which the needle tube 5 is fixed.
A needle tube of 22-33 gauge (external diameter: 0.2-0.7 mm), preferably 26-33 gauge, more preferably 30-33 gauge, in size according to ISO standard for medical needle tubes (ISO9626: 1991/Amd. 1:2001(E)) is used as the needle tube 5.

A first needle tip 8 for being stuck into a living body is provided on one end of the needle tube 5, and a second needle tip 9 for being penetrated through a contact portion 35 (which is to be described later) of the drug container 3 is provided on the other end of the needle tube 5. The first needle tip 8 has a blade face 8a. The length of the blade face 8a in the direction in which the needle tube 5 extends (referred to as "bevel length B" hereinafter) may be equal to or less than 1.4 mm (which is the minimum thickness of the upper layer of the skin of an adult), but equal to or greater than about 0.5 mm, which is the bevel length when a short bevel is formed in a needle tube of 33 gauge. In other words, it is preferred that the bevel length B is set in a range of 0.5-1.4 mm.

Further, it is further preferred that the bevel length B is equal to or less than 0.9 mm (which is the minimum thickness of the upper layer of the skin of a child). In other words, it is further preferred that the bevel length B is set in a range of 0.5-0.9 mm. Incidentally, the short bevel refers to a blade face forming an angle of 18-25° with respect to the longitudinal direction of the needle, which is generally used as an injection needle.

The second needle tip 9 has a blade face 9a. The length of the blade face 9a in the direction in which the needle tube 5 extends can be arbitrarily set, and can be set to a length equal to that of the blade face 8a of the first needle tip 8.

The material of the needle tube 5 may be, for example, stainless steel, aluminum, aluminum alloy, titanium, titanium alloy, and other metals. Further, a straight needle, a tapered needle in which at least a portion thereof is tapered or the like may be used as the needle tube 5. The tapered needle may have a configuration in which the external diameter on the side of the second needle tip 9 is larger than the external diameter on the side of the first needle tip 8, and a middle portion is tapered. Incidentally, in such a case, the shape of the first needle tip 8 may be different from the shape of the second needle tip 9.

Next, the needle hub 6 will be described below.
The needle hub 6 includes a needle-holding portion 11 for holding the central portion of the needle tube 5, an adjusting portion 12, a stabilizer 13, a guide portion 14, and a drug container mount 15.

Examples of the material of the needle hub 6 include various resins such as polyvinyl chloride, polyethylene, polypropylene, cyclic polyolefin, polystyrene, poly(4-methylpentene-1), polycarbonate, acrylate resin, acrylonitrile-butadiene-styrene copolymer, polyesters (such as polyethylene terephthalate and the like), butadiene-styrene copolymer, polyamides (such as nylon 6, nylon 6,6, nylon 6,10, nylon 12). Among the above resins, any one of polypropylene, cyclic polyolefin, polyesters, poly(4-methylpentene-1) is preferably used because of their good moldability.

The needle-holding portion 11 is formed in a substantially cylindrical shape, and has an end face 11a perpendicular to the axial direction. The adjusting portion 12 is arranged in the central portion of the end face 11a of the needle-holding portion 11, and is formed as a cylindrical projection projecting in the axial direction of the needle-holding portion 11. The axis of the adjusting portion 12 coincides with the axis of the needle-holding portion 11.

A through-hole 18, through which the needle tube 5 is inserted, is formed in the axes of the needle-holding portion 11 and the adjusting portion 12. An injection hole 19 for injecting an adhesive 20 into the through-hole 18 is formed in the needle-holding portion 11. The injection hole 19 is opened to the outer circumferential surface of the needle-holding portion 11, and is in communication with the through-hole 18. In other words, the needle tube 5 is fixed to the needle-holding portion 11 by the adhesive 20 injected from the injection hole 19 into the through-hole 18.

A ring-like flange 21 projecting outward in the radial direction of the needle-holding portion 11 is provided on the outer circumferential surface of the needle-holding portion 11. The flange 21 has a flat surface 21a and a flat surface 21b, wherein the flat surface 21a and the flat surface 21b face each other in the axial direction of the needle-holding portion 11. The flat surface 21a of the flange 21 is coplanar with the end face 11a of the needle-holding portion 11. The outer edge portion of the flange 21 serves as the guide portion 14. The guide portion 14 will be described later in more detail.

The end face of the adjusting portion 12 serves as a needle-protruding surface 12a from which the first needle tip 8 of the needle tube 5 projects. The needle-protruding surface 12a is formed as a flat surface perpendicular to the axial direction of the needle tube 5. When the needle tube 5 is stuck into the upper layer of the skin, the needle-protruding surface 12a contacts the surface of the skin, so that the insertion depth of the needle tube 5 into the upper layer of the skin is regulated. In other words, the insertion depth of the needle tube 5 into the upper layer of the skin is determined by the length of the needle tube 5 protruding from the needle-protruding surface 12a (such length will be referred to as "protruding length L" hereinafter).

The thickness of the upper layer of the skin corresponds to the depth from the surface of the skin to the dermis layer, which is generally in a range of 0.5-3.0 mm. Therefore, the protruding length L of the needle tube 5 can be set in a range of 0.5-3.0 mm.

The vaccine is generally administered to the upper arm; however, when administering the vaccine to the upper layer of the skin, it is preferred that the vaccine is administered to the portion around the shoulder where the skin is thick, particularly the portion of the deltoid muscle. Therefore, the thickness of the upper layer of the skin overlying the deltoid muscle was measured for 19 children and 31 adults. The measurements were performed by imaging the upper layer of the skin having high ultrasonic reflectivity using an ultrasonic measurement device (NP60R-UBM High Resolution Echo for Small Animal, NEPA GENE, CO., LTD.). Incidentally, since the measured values showed lognormal distribution, the range of MEAN ± 2SD was obtained by taking the geometrical mean.

The results showed that the thickness of the upper layer of skin overlying the deltoid muscle of a child was 0.9-1.6 mm. Further, the results also showed that the thickness of the upper layer of skin overlying the deltoid muscle of an adult was 1.4-2.6 mm in the distal portion, 1.4-2.5 mm in the middle portion, and 1.5-2.5 mm in the proximal portion. It can be confirmed from the above that the thickness of the upper layer of skin overlying the deltoid muscle is equal to or more than 0.9 mm for children, and is equal to or more than 1.4 mm for adults. Consequently, when performing injection on the upper layer of skin overlying the deltoid muscle, it is preferred that the protruding length L of the needle tube 5 is set in a range of 0.9-1.4 mm.

By setting the protruding length L in such a manner, it becomes possible to reliably position the blade face 8a of the first needle tip 8 in the upper layer of the skin. As a result, the needle hole (the drug outlet) opened on the blade face 8a can be positioned in the upper layer of the skin, regardless of its position in the blade face 8a. Incidentally, even when the drug outlet is positioned in the upper layer of the skin, if the first needle tip 8 is stuck into a depth deeper than the upper layer of the skin, the drug will flow into the subcutaneous layer from between the side surface of the end portion of the first needle tip 8 and the incised skin, and therefore it is important that the blade face 8a is reliably positioned in the upper layer of the skin.

Incidentally, if the needle tube is larger than 26 gauge, it will be difficult to make the bevel length B 1.0 mm or less. Accordingly, to set the protruding length L of the needle tube 5 in the preferable range (i.e., the range of 0.9-1.4 mm), it is preferred to use a needle tube smaller than 26 gauge.

The needle-protruding surface 12a is formed such that the distance S from the circumferential edge of the needle-protruding surface 12a to the outer circumferential surface of the needle tube 5 is 1.4 mm or less, preferably in a range of 0.3-1.4 mm. The distance S from the circumferential edge of the needle-protruding surface 12a to the circumferential surface of the needle tube 5 is set considering that a pressure will be applied to the blister formed by administering drug to the upper layer of the skin. In other words, the diameter of the needle-protruding surface 12a is set to sufficiently smaller than the diameter of the blister to be formed in the upper layer of the skin, so that the formation of the blister will not be obstructed. As a result, even if the needle-protruding surface 12a presses the skin around the needle tube 5, the administered drug can be prevented from being leaked out.

The stabilizer 13 is formed in a tubular shape protruded from the flat surface 21a of the flange 21 provided in the needle-holding portion 11 and having a circular tube hole. The needle tube 5 and the adjusting portion 12 are arranged in the tube hole of the stabilizer 13. In other words, the stabilizer 13 is formed in a tubular shape covering around the adjusting portion 12 through which the needle tube 5 is penetrated, and is spaced from the needle tube 5 in the outer radial direction of the needle-holding portion 11.

An end face 13a of the stabilizer 13 is located closer to the side of the second needle tip 9 of the needle tube 5 than the needle-protruding surface 12a of the adjusting portion 12. When sticking the first needle tip 8 of the needle tube 5 into the living body, first, the needle-protruding surface 12a contacts the surface of the skin, and then the end face 13a of the stabilizer 13 contacts the surface of the skin. At this time, the end face 13a of the stabilizer 13 contacts the surface of the skin, and thereby the drug injection apparatus 1 becomes stable, so that the needle tube 5 can be kept in an attitude where the needle tube 5 is substantially perpendicular to the skin.

Incidentally, the needle tube 5 can be kept in an attitude where the needle tube 5 is substantially perpendicular to the skin even if the end face 13a of the stabilizer 13 is coplanar with the needle-protruding surface 12a or located closer to the side of the first needle tip 8 of the needle tube 5 than the needle-protruding surface 12a. Incidentally, considering the raised portion of the skin formed when the stabilizer 13 is pressed against the skin, it is preferred that the distance between the end face 13a of the stabilizer 13 and the needle-protruding surface 12a in the axial direction of the needle-holding portion 11 is set to 1.3 mm or less.

Further, the inner diameter d of the stabilizer 13 is set to a value equal to or larger than the diameter of the blister formed in the skin. To be specific, the distance T between the inner wall of the stabilizer 13 and the circumferential edge of the needle-protruding surface 12a is set in a range of 4-15 mm. With such arrangement, it is possible to prevent the case where the formation of the blister is obstructed due to the pressure applied from the inner wall of the stabilizer 13 to the blister.

The distance T between the inner wall of the stabilizer 13 and the circumferential edge of the needle-protruding surface 12a is set to 4 mm or more, but without upper limit. However, if the distance T is too large, the outer diameter of the stabilizer 13 will become large, and therefore it will be difficult to bring the entire end face 13a of the stabilizer 13 into contact with the skin when the needle tube 5 is to be stuck into the skin of a slender arm, such as an arm a child. Thus, considering the slender arms of children, it is preferred that the distance T is defined to 15 mm as the maximum.

If the distance S between the circumferential edge of needle-protruding surface 12a and the outer circumferential surface of the needle tube 5 is 0.3 mm or more, the adjusting portion 12 will not enter the skin. Thus, considering the diameter (about 0.3 mm) of the needle-protruding surface 12a and the distance T (4 mm or more) between the inner wall of the stabilizer 13 and the circumferential edge of the needle-protruding surface 12a, the inner diameter d of the stabilizer 13 can be set to 9 mm or larger.

Incidentally, the shape of the stabilizer 13 is not limited to the circular tube shape, but may also be formed, for example, in a polygonal prism shape, such as a quadrangular prism shape, a hexagonal prism shape or the like, having a tube hole formed in the center thereof.

The guide portion 14 is a portion located outside of the stabilizer 13 of the flange 21. The guide portion 14 has a contact surface 14a that contacts the skin. The contact surface 14a is a portion of the flat surface 21a of the flange 21, and is a flat surface substantially parallel to the end face 13a of the stabilizer 13. By pressing the stabilizer 13 until the contact surface 14a of the guide portion 14 contacts the skin, the force for both the stabilizer 13 and the needle tube 5 to press the skin can be constantly maintained at or above a predetermined value, and thereby the protruding portion of the needle tube 5 from the needle-protruding surface 12a (corresponding to the protruding length L) can be reliably inserted into the skin.

The value of the distance Y between the contact surface 14a of the guide portion 14 and the end face 13a of the stabilizer 13 (referred to as "guide portion height") is set so that the skin can be pressed and punctured by a suitable pressing force applied from the needle tube 5 and the stabilizer 13. Thus, the pressing force applied from the needle tube 5 and the stabilizer 13 to the skin is guided by the guide portion 14, so that the first needle tip 8 (the blade face 8a) of the needle tube 5 can be reliably positioned in the upper layer of the skin, and it is possible to bring the user a sense of reassurance. Incidentally, the suitable pressing force of the needle tube 5 and the stabilizer 13 is in a range of, for example, 3N to 20N.

When the internal diameter d of the stabilizer 13 is in a range of 11-14 mm, the guide portion height Y is suitably determined based on the length X (referred to as "guide portion length" hereinafter) between the tip end face of the guide portion 14 and the outer circumferential surface of the stabilizer 13. For example, when the inner diameter d of the stabilizer 13 is 12 mm and the guide portion length X is 3.0 mm, the guide portion height Y will be set in a range of 2.3-6.6 mm.

The drug container mount 15 is formed continuously from an end portion of the needle-holding portion 11 on the opposite side to the end face 11a. The drug container mount 15 is formed in a quadrangular plate-like shape having a predetermined thickness, and the center of the drug container mount 15 coincides with the axis of the needle-holding portion 11. A surface 15a (referred to an "upper surface 15a" hereinafter) of the drug container mount 15 on the opposite side to the needle-holding portion 11 is a flat surface perpendicular to the axial direction of the needle-holding portion 11.

A recessed portion 16 is formed in the upper surface 15a of the drug container mount 15. The recessed portion 16 is formed by forming a concave spherical surface 16a whose center is a point in the axis of the needle-holding portion 11. The upper surface 15a and concave spherical surface 16a of the drug container mount 15 serve as a mounting surface 22 to be contacted by a contact portion 35 of the drug container 3.

The second needle tip 9 of the needle tube 5 projects from the central portion of the concave spherical surface 16a. The length of the portion of second needle tip 9 projecting from the concave spherical surface 16a is at least equal to a value obtained by adding the thickness of the contact portion 35 and the thickness of a seal member 32 of the drug container 3 to the bevel length of the blade face 9a. Thus, the needle tube 5 can be reliably brought into communication with the inside of the drug container 3.

The four sides of the upper surface 15a are each provided with a locking piece 23 for preventing the drug container 3 from being moved (displaced) in directions perpendicular to the axial direction of the needle-holding portion 11. The locking pieces 23 are each formed in a plate-like shape substantially vertically projecting from the upper surface 15a. Incidentally, each locking piece 23 may also be formed in a pin-like (rod-like) shape.

The lower surface 15b of the drug container mount 15 extends outward from the needle-holding portion 11 in the radial direction; and a gripping portion 10 is formed by the lower surface 15b, the flat surface 21b of the flange 21, and the outer circumferential surface of the needle-holding portion 11. The gripping portion 10 is formed in a bobbin-like shape and has a circumferentially-recessed portion. When using the drug injection apparatus 1, the user can grip the drug injection apparatus 1 by, for example, putting his (or her) index finger and middle finger on the recessed portion of the gripping portion 10 so that the needle-holding portion 11 is sandwiched by the both fingers. Since the gripping portion 10 is recessed, the fingers of the user are stabilized, so that the drug injection apparatus 1 can be easily gripped by the user.

### [Drug container]

Next, the drug container 3 will be described below.
The drug container 3 includes a container body 31, the seal member 32, and a protective plate 33, which is a first concrete example of a penetration avoiding portion.

The container body 31 is formed in a flattened bag-like shape by superimposing two sheets and joining the edge portions of the two sheets in a liquid-tight manner by means of, for example, heat fusion, ultrasonic welding or the like. The container body 31 can be produced by injection molding, extrusion molding, calender molding, blow molding, inflation molding or the like.

The container body 31 is configured by the contact portion 35, which is one sheet, and a pressing portion 36, which is the other sheet. The contact portion 35 and the pressing portion 36 are superimposed on each other and joined to each other, so that a flange piece 37 is formed in the container body 31. The internal space formed by the contact portion 35 and the pressing portion 36 becomes a liquid chamber filled with a drug M.
In the present embodiment, the container body 31 is formed by joining two quadrangular sheets so that the outline of the liquid chamber is substantially a circle, and the outline of the flange piece 37 is a quadrangle when viewed in a direction in which the contact portion 35 and the pressing portion 36 face each other.

The contact portion 35 and the pressing portion 36 face each other with the liquid chamber interposed therebetween. The second needle tip 9 of the needle tube 5 is penetrated through the contact portion 35 so as to be located inside the container body 31 (the liquid chamber). In such a state, when the pressing portion 36 is pressed toward the side of the contact portion 35, the inner surface of the pressing portion 36 will be brought close to the inner surface of the contact portion 35, so that the inner pressure of the container body 31 will be increased. As a result, the drug M enclosed in the container body 31 is discharged to the outside from the first needle tip 8 through the needle hole (not shown) of the needle tube 5.

The contact portion 35 and the pressing portion 36 are each formed of a flexible material, and can be penetrated by the second needle tip 9. Examples of the material of the contact portion 35 and pressing portion 36 include, for example, soft resins such as polypropylene (PP), polyethylene (PE), ethylene-vinyl acetate copolymer (EVA) and the like, and materials each obtained by blending styrene thermoplastic elastomer, olefinic thermoplastic elastomer and/or the like into polyethylene or polypropylene, and then softening the blend material. These materials may be used as a composite material obtained by laminating two or three layers of these materials, depending on the intended use.

The tensile elasticity of the soft resin material is preferably no more than 500 MPa, and more preferably in a range of 50-300 Mpa, from the viewpoint of handleability, liquid-dischargeability and the like. The thickness of the soft resin material is suitably determined according to its layer construction, properties (such as softness, strength, water vapor permeability, and heat resistance) and the like, instead of being particularly limited; however, generally the thickness of the soft resin material is preferably in a range of about 100-500 µm, and more preferably in a range of about 200-360 µm. Further, the inner surface of the container body 31 may be coated with a film, such as a low-density polyethylene laminated film.

Further, it is preferred that the container body 31 has water vapor barrier property. Since the container body 31 has water vapor barrier property, not only water inside the container body 31 can be prevented from being evaporated, but also water vapor outside the container body 31 can be prevented from entering the container body 31.
The water vapor permeability (as degree of the water vapor barrier property) is preferably equal to or less than 50g/m²•24hrs•40°C•90%RH, more preferably equal to or less than log/m²•24hrs•40°C•90%RH, and further preferably equal to or less than 1g/m²•24hrs•40•C•90%RH. The water vapor permeability is measured by a method described in JISK7129 (Method A).

Examples of the drug M include, but not limited to, various vaccines for preventing infectious diseases such as influenza. Incidentally, examples of the drug M also include carbohydrate injections (such as glucose and the like), electrolyte correction injections (such as sodium chloride, potassium lactate and the like), vitamins, antibiotic injections, radiopaque dye, steroid drugs, hormonal agents, protease inhibitor, fat emulsion, anticancer drugs, anesthetic drugs, stimulant drugs, narcotic drugs, heparin calcium, antibody drugs, and the like.

The drug container 3 filled with the drug M can be produced by, for example, blow fill molding method in which molding (blow), filling and sealing are performed in sequence.
In order to produce the drug container 3 filled with the drug M by blow fill molding method, first, two rectangular sheets are superimposed on each other and the long sides of the two rectangular sheets are fused to each other so as to form a substantially tubular container material. Next, one short side of the tubular container material is sealed by fusion, and the drug M is filled into the container material. Further, the other short side of the container material is sealed by fusion, and the other side is cut off. Thus, the drug container 3 filled with the drug M is formed. Incidentally, the shape of the liquid chamber can be changed by changing the shape of the fusing die.

The seal member 32 is attached to the central portion of the outer surface of the contact portion 35. The central portion of the contact portion 35 is a portion through which the second needle tip is to be penetrated. In other words, the seal member 32 is attached to the contact portion 35 in the portion through which the second needle tip is to be penetrated, and the second needle tip 9 is to be penetrated through the seal member 32 and the contact portion 35 so as to be inserted into the container body 31.

The seal member 32 comes into contact with the circumferential surface of the penetrated needle tube 5 in a liquid-tight manner. Thus, in a state where the second needle tip of the needle tube 5 is penetrated through the contact portion 35, the drug can be prevented from being leaked from between the needle tube 5 and the contact portion 35. Further, the resisting force of the seal member 32 against the insertion of the second needle tip 9 is larger than the resisting force of the living body (for example, the skin) against the insertion of the first needle tip 8. Thus, it is possible for the second needle tip 9 to be penetrated through the seal member 32 (the contact portion 35) after the first needle tip 8 has been stuck into the living body.

In order to achieve good liquid tightness between the seal member 32 and the needle tube 5, it is preferred that the seal member 32 is formed of a material having rubber elasticity. Examples of the material having rubber elasticity include natural rubber, isoprene rubber, butyl rubber, chlorinated butyl rubber, brominated butyl rubber, butadiene rubber, styrene elastomer, thermoplastic elastomer, silicone rubber, and a mixture of these materials.

The protective plate 33 is attached to the central portion of the outer surface of the pressing portion 36, and faces the seal member 32 attached to the contact portion 35. When the pressing portion 36 is pressed so as to come close to the contact portion 35, the protective plate 33 will abut the second needle tip 9 inserted into the container body 31, so that the pressing portion 36 is prevented from being penetrated by the second needle tip 9.

The protective plate 33 has such hardness that it will not be penetrated by the second needle tip 9. Examples of the material of the protective plate 33 include metals such as iron, stainless steel, and the like. The protective plate 33 may also be formed of a hard synthetic resin such as polypropylene (PP), polyethylene (PE), polystyrene (PS), ABS resin, polyvinyl chloride (PVC), polycarbonate (PC), or the like.

### [Method for using drug injection apparatus]

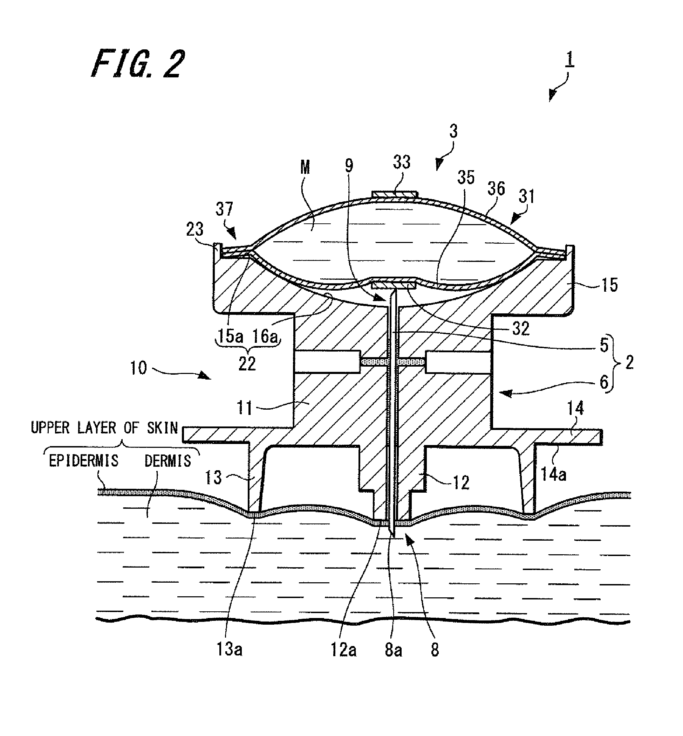

Next, a method for using the drug injection apparatus 1 will be described below with reference to FIGS. 2 to 4.

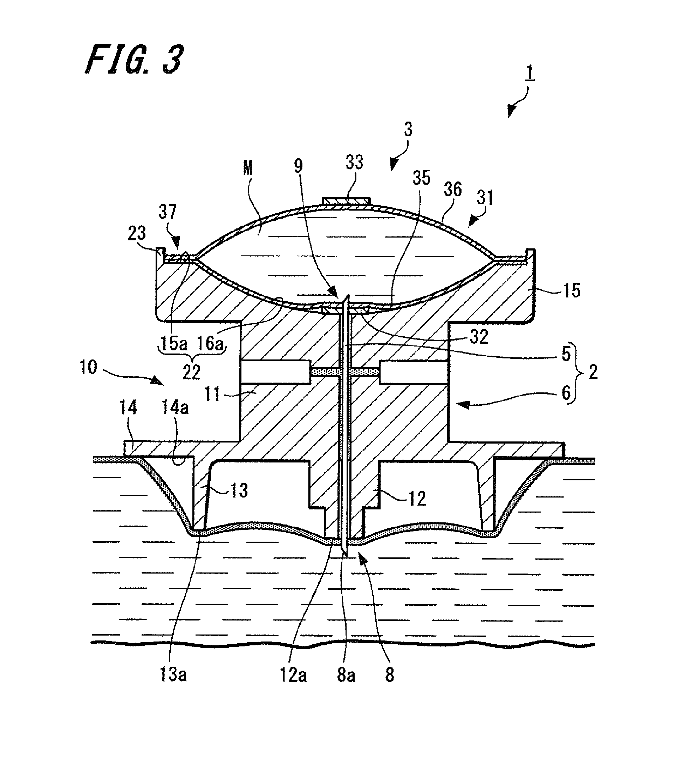

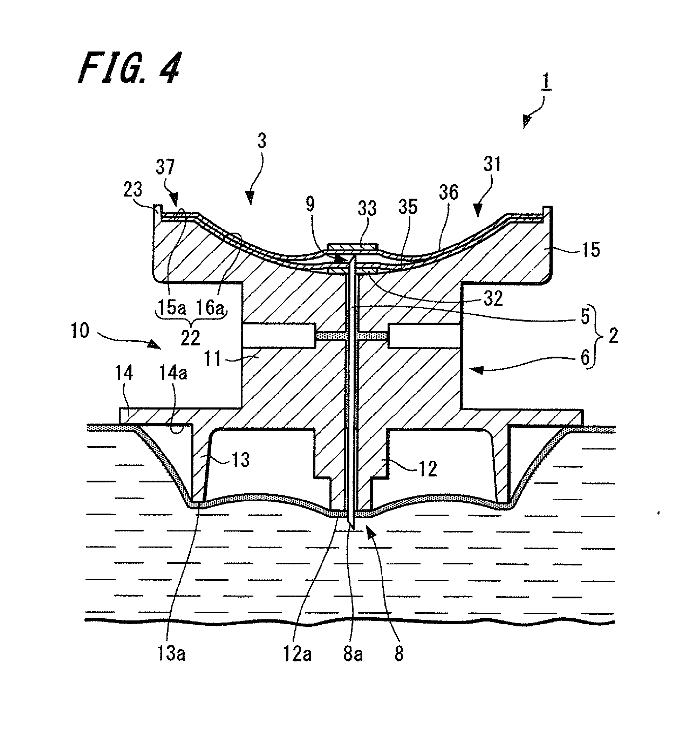

FIG. 2 is a cross-sectional view showing a state where the drug container 3 is mounted on the injection needle assembly 2, and the first needle tip 8 is stuck into a living body. FIG. 3 is a cross-sectional view showing a state where the second needle tip 9 has been penetrated through the contact portion 35 of the drug container 3. FIG. 4 is a cross-sectional view showing a state where drug administration of the drug injection apparatus 1 has been completed.

Before being used, the drug injection apparatus 1 is in a state where the injection needle assembly 2 and the drug container 3 are separated from each other (see FIG. 1). Thus, when using the drug injection apparatus 1, first, the drug container 3 is mounted on the drug container mount 15 of the injection needle assembly 2, and thereby the drug injection apparatus 1 is assembled.

The drug injection apparatus 1 includes two assembly states: one is a first assembly state where the second needle tip of the needle tube 5 abuts the seal member 32, and the other is a second assembly state where the second needle tip 9 is inserted into the container body 31 of the drug container 3. Here, a method for using the drug injection apparatus 1 in the case where the drug injection apparatus 1 is assembled in the first assembly state will be described first, and thereafter a method for using the drug injection apparatus 1 in the case where the drug injection apparatus 1 is assembled in the second assembly state will be described.

### <First assembly state>

In the case where the drug injection apparatus 1 is to be assembled in the first assembly state, the drug container 3 is caused to face the drug container mount 15 of the injection needle assembly 2, and the position of the drug container 3 is adjusted so that the four sides of the flange piece 37 of the drug container 3 are respectively parallel to the four locking pieces 23 of the drug container mount 15.

Next, the drug container 3 is mounted on the drug container mount 15. Thus, the drug injection apparatus 1 is assembled in the first assembly state. At this time, the second needle tip 9 of the needle tube 5 is abutted against the seal member 32, but not penetrated through the contact portion 35. Further, the outer surface of the contact portion 35 curves so that the central portion thereof, to which the seal member 32 is attached, is recessed toward the side of the pressing portion 36 (see FIG. 2). Further, the edge portion of the contact portion 35 contacts the upper surface 15a and the concave spherical surface 16a (i.e., the mounting surface 22) of the drug container mount 15.

Further, the flange piece 37 of the drug container 3 abuts each locking piece 23 of the drug container mount 15. Thus, the movement of the drug container 3 in directions perpendicular to the axial direction of the needle-holding portion 11 is locked, and therefore the drug container 3 can be positioned.

Next, the first needle tip 8 of the needle tube 5 is stuck into the living body. To stick the first needle tip 8 into a living body, first, the end face 13a of the stabilizer 13 is caused to face the skin of the living body. Next, the drug injection apparatus 1 is moved substantially perpendicular to the skin, and the pressing portion 36 of the drug container 3 is pressed toward the side of the contact portion 35, so that the first needle tip 8 is stuck into the skin and the end face 13a of the stabilizer 13 is pressed against the skin (see FIG. 2). At this time, the first needle tip 8 is stuck into the skin, but the second needle tip 9 does not penetrate through the seal member 32. Further, since the needle-protruding surface 12a contacts the skin so as to cause the skin to deform flatly, the first needle tip 8 of the needle tube 5 can be inserted into the skin by the protruding length L only.

Next, the pressing portion 36 of the drug container 3 is pressed until the contact surface 14a of the guide portion 14 contacts the skin. Here, the value of the height of the guide portion Y (see FIG. 1) is set so that the needle tube 5 and the stabilizer 13 can be stuck into the skin by a suitable pressing force, so as to inject the drug into the skin. Thus, the force for the stabilizer 13 to press against the skin becomes a predetermined value.

As a result, the suitable pressing force of the stabilizer 13 can be recognized by the user, and the first needle tip 8 and the blade face 8a of the needle tube 5 can be reliably positioned in the upper layer of the skin. In such a manner, the guide portion 14 becomes a mark for recognizing the suitable pressing force of the stabilizer 13, and thereby the user can use the drug injection apparatus 1 with a sense of reassurance.

Further, since the stabilizer 13 abuts the skin, the attitude of the drug injection apparatus 1 becomes stable, so that it is possible to stick the needle tube 5 straight into the skin. Further, after the first needle tip 8 has been stuck into the skin, waggling caused in the needle tube 5 can be prevented, and therefore the drug can be administered in a stable manner.

In the case of a needle tube with a very short protruding length of about 0.5 mm, for example, there is a possibility that the needle tip will not be stuck into the skin even if the needle tip is brought into contact with the skin. However, since the skin is pressed down perpendicularly by the stabilizer 13, the skin inside the stabilizer 13 is strained so as to be in a tensioned state. Thus, since the skin becomes hard to flee from the first needle tip 8 of the needle tube 5, the stabilizer 13 has an effect of making it easier for the first needle tip 8 to stick the skin.

When the contact surface 14a of the guide portion 14 contacts the skin, the second needle tip 9 will penetrate through the seal member 32 and the contact portion 35, so that the needle hole of the needle tube 5 is brought into communication with the inside (i.e., the liquid chamber) of the container body 31 (see FIG. 3). Further, the pressing portion 36 is pressed until the protective plate 33 abuts the second needle tip 9 inserted into the container body 31 (see FIG. 4). Thus, pressure is exerted on the inside of the container body 31, and thereby the drug M is injected into the upper layer of skin from the first needle tip 8 through the needle hole (not shown) of the needle tube 5.

At this time, the direction in which the needle tube 5 is stuck into the skin is identical to the direction in which the container body 31 (the pressing portion 36) is pressed. Thus, it is possible to easily press the container body 31 while stably holding the drug injection apparatus 1 in a state where the first needle tip 8 has been stuck into the living body. As a result, the drug M can be reliably administered into the upper layer of skin from the first needle tip 8, and the amount of the drug M administered to the upper layer of skin can be stabilized. Further, since the sticking operation and the subsequent injecting operation can be performed as a sequence of actions, the operation can be facilitated, and it is possible to operate the drug injection apparatus 1 with one hand.

Further, since the protective plate 33 abuts the second needle tip 9 inserted into the container body 31, the second needle tip 9 penetrated through the contact portion 35 will not penetrate through the pressing portion 36. Thus, it is possible to allow the drug M to be reliably discharged from the first needle tip 8 while preventing the second needle tip 9 from being stuck into the fingertip or the like that presses the pressing portion 36.

Further, in the present embodiment, the contact portion 35 of the drug container 3 is made of a flexible material possible to be penetrated by the second needle tip 9, and is formed so as to contact the concave spherical surface 16a of the mounting surface 22. Thus, it is possible for the entire inner surface of the pressing portion 36 of the drug container 3 to easily abut the entire inner surface of the contact portion 35, and therefore the amount of the drug remaining in the drug container 3 can be reduced.

In the present embodiment, the drug container mount 15 is formed with the concave spherical surface 16a to be contacted by the contact portion 35 of the drug container 3. However, the surface of the drug container mount to be contacted by the contact portion of the drug container may also be a conical surface.

### <Second assembly state>

Next, the method for using the drug injection apparatus 1 in the case where the drug injection apparatus 1 is assembled in the second assembly state will be described below.
In the case where the drug injection apparatus 1 is to be assembled in the second assembly state, the drug container 3 is caused to face the drug container mount 15 of the injection needle assembly 2, and the position of the drug container 3 is adjusted so that the four sides of the flange piece 37 of the drug container 3 are respectively parallel to the four locking pieces 23 of the drug container mount 15.

Next, the drug container 3 is mounted on the drug container mount 15, and the pressing portion 36 of the drug container 3 is pressed, so that the second needle tip 9 of the needle tube 5 is penetrated through the seal member 32, and thereby the needle hole of the needle tube 5 is brought into communication with the inside (i.e., the liquid chamber) of the container body 31. As a result, the drug injection apparatus 1 is assembled in the second assembly state.

Next, the needle hub 6 of the injection needle assembly 2 is gripped to cause the end face 13a of the stabilizer 13 to face the skin, and further, the drug injection apparatus 1 is moved substantially perpendicular to the skin, so that the first needle tip 8 is stuck into the skin and the end face 13a of the stabilizer 13 is pressed against the skin. At this time, since the needle-protruding surface 12a contacts the skin so as to cause the skin to deform flatly, the first needle tip 8 of the needle tube 5 can be inserted into the skin by the protruding length L only.

Next, the gripped needle hub 6 is further pressed toward the side of the skin so as to bring the contact surface 14a of the guide portion 14 into contact with the skin (see FIG. 3). Thus, the force for the stabilizer 13 to press against the skin becomes a predetermined value. As a result, the suitable pressing force of the stabilizer 13 can be recognized by the user, and the first needle tip 8 and the blade face 8a of the needle tube 5 can be reliably positioned in the upper layer of the skin.

Next, the pressing portion 36 is pressed until the protective plate 33 abuts the second needle tip 9 inserted into the container body 31 (see FIG. 4). Thus, pressure is exerted on the inside of the container body 31, and thereby the drug M is injected into the upper layer of skin from the first needle tip 8 through the needle hole (not shown) of the needle tube 5.

Similar to the first assembly state, in the second assembly state, the direction in which the needle tube 5 is stuck into the skin is also identical to the direction in which the container body 31 (the pressing portion 36) is pressed. Thus, it is possible to easily press the container body 31 while stably holding the drug injection apparatus 1 in a state where the first needle tip 8 has been stuck into the living body. As a result, the drug M can be reliably administered into the upper layer of skin from the first needle tip 8, and the amount of the drug M administered to the upper layer of skin can be stabilized.

Further, since the protective plate 33 abuts the second needle tip 9, the second needle tip 9 inserted into the container body 31 will not penetrate through the pressing portion 31 again. Thus, it is possible to prevent the second needle tip 9 from being stuck into the fingertip or the like that presses the pressing portion 36, without obstructing the discharge of the drug M.

### 2. Second embodiment

### [Drug injection apparatus]

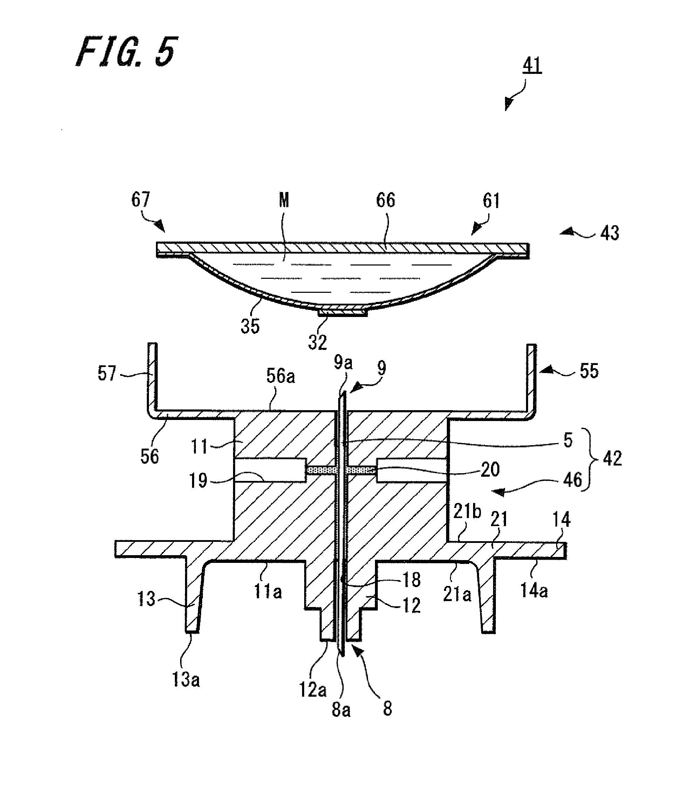

Next, a drug injection apparatus according to a second embodiment of the present invention will be described below with reference to FIG. 5.
FIG. 5 is a cross-sectional view showing the configuration of a drug injection apparatus 41 according to the second embodiment.

As shown in FIG. 5, the drug injection apparatus 41 of the second embodiment has the same configuration as that of the drug injection apparatus 1 (see FIG. 1) of the first embodiment. The drug injection apparatus 41 differs from the drug injection apparatus 1 in that a drug container 43 and a drug container mount 55 of the drug injection apparatus 41 are different from the drug container 3 and the drug container mount 15 of the drug injection apparatus 1. Thus, the description herein will be focused on the drug container 43 and the drug container mount 55; and components common to those of the drug injection apparatus 1 are denoted by the same reference numerals and the explanation thereof will not be repeated.

The drug injection apparatus 41 includes an injection needle assembly 42, and a drug container 43 to be mounted on the injection needle assembly 42. Similar to the drug injection apparatus 1, the drug injection apparatus 41 is used to stick a needle tip into a skin from the surface of the skin to inject a drug into an upper layer of the skin.

The injection needle assembly 42 includes a needle tube 5 having a hollow needle hole, and a needle hub 46 by which the needle tube 5 is fixed. The needle hub 46 includes a needle-holding portion 11 for holding the central portion of the needle tube 5, an adjusting portion 12, a stabilizer 13, a guide portion 14, and a drug container mount 55. Examples of the material of the needle hub 46 include synthetic resins such as polycarbonate, polypropylene, polyethylene and the like.

The drug container mount 55 is formed continuously from an end portion of the needle-holding portion 11 on the opposite side to the end face 11a. The drug container mount 55 is formed into a case-like shape with one face opened, and has a quadrangular bottom 56 and four side plate portions 57, wherein the opened face of the drug container mount 55 is perpendicular to the axial direction of the needle-holding portion 11. The center of the bottom 56 coincides with the axis of the needle-holding portion 11. The inner surface 56a of the bottom 56 is a flat surface perpendicular to the axial direction of the needle-holding portion 11, and serves as a mounting surface to be contacted by the contact portion 35 of the drug container 43.

The second needle tip 9 of the needle tube 5 projects from the central portion of the inner surface 56a. The length of the portion of second needle tip 9 projecting from the inner surface 56a is at least equal to a value obtained by adding the thickness of the contact portion 35 and the thickness of the seal member 32 of the drug container 43 to the bevel length of the blade face 9a. With such arrangement, the needle tube 5 can be reliably brought into communication with the inside of the drug container 43. Further, the four side plate portions 57 are engaged with a flange piece 67 (which is to be described later) of the drug container 43, so that the movement (displacement) of the drug container 43 in directions perpendicular to the axial direction of the needle-holding portion 11 is inhibited.

### [Drug container]

Next, the drug container 43 will be described below.
The drug container 43 includes a container body 61 and a seal member 32.

The container body 61 includes a contact portion 35 and a pressing portion 66 which is formed in quadrangular plate-like shape having the same size as that of the bottom 56 of the drug container mount 55. The container body 61 is formed in a flattened bag-like shape by joining the edge portion of the contact portion 35 and the edge portion of the pressing portion 66 in a liquid-tight manner.

The edge portion of the contact portion 35 and the edge portion of the pressing portion 66 are joined to each other so as to form the flange piece 67 of the container body 61. The internal space formed by the contact portion 35 and the pressing portion 66 becomes a liquid chamber filled with a drug M.
Incidentally, similar to the drug container 3 of the first embodiment, the outline of the liquid chamber of the container body 61 is substantially a circle when viewed in the direction in which the contact portion 35 and the pressing portion 66 face each other.

The pressing portion 66 is formed on one face of the container body 61, and faces the contact portion 35. When being pressed so as to come close to the contact portion 35, the pressing portion 66 will abut the second needle tip 9 inserted into the container body 61 and thereby come to a stop. In other words, the pressing portion 66 not only is a part of the container body, but also serves as a penetration avoiding portion for avoiding the penetration of the second needle tip 9.

The pressing portion 66 may be formed of a material having such hardness that it will not be penetrated by the second needle tip 9. Examples of the material of the pressing portion 66 include metals, such as iron, stainless steel and the like, and hard synthetic resins, such as polypropylene (PP), polyethylene (PE), polystyrene (PS), ABS resin, polyvinyl chloride (PVC), polycarbonate (PC) and the like.

### [Method for using drug injection apparatus]

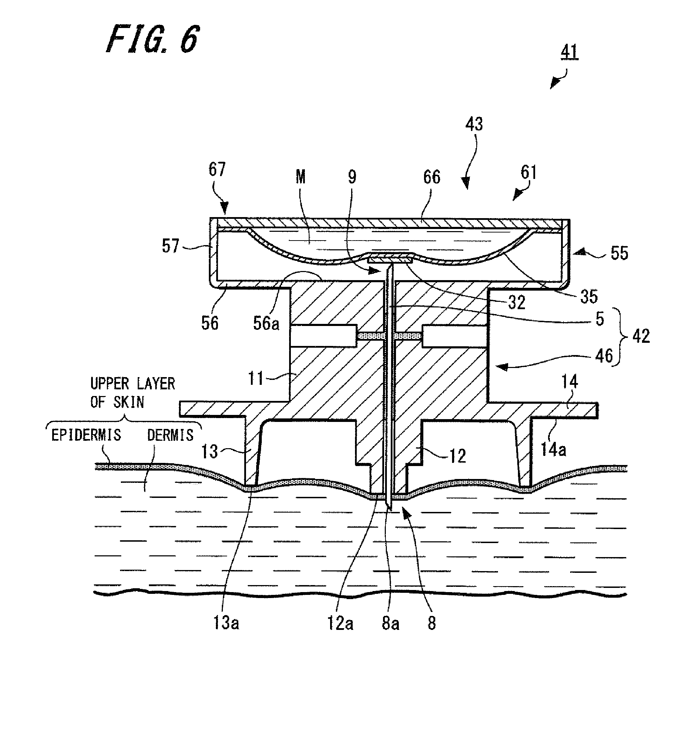

Next, a method for using the drug injection apparatus 41 will be described below with reference to FIGS. 6 to 8.

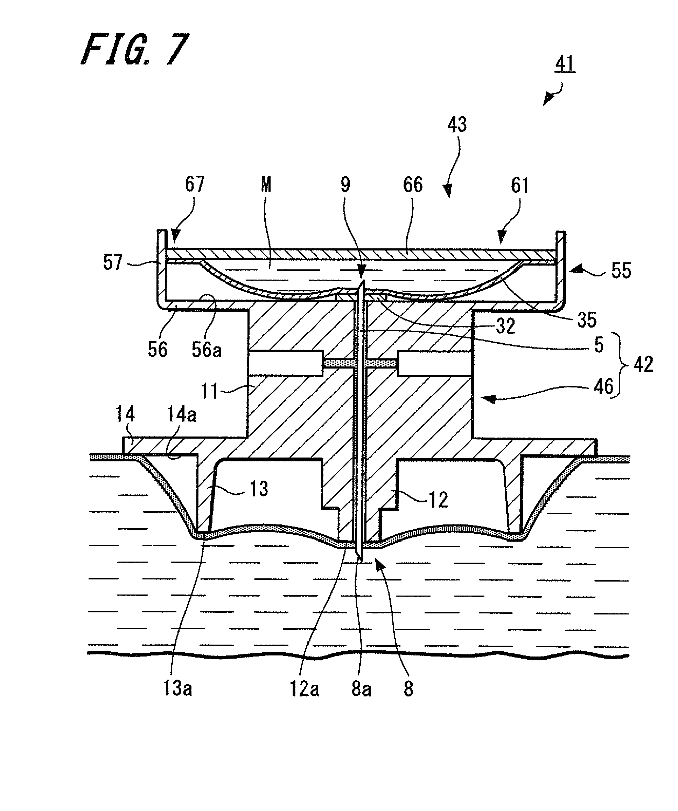

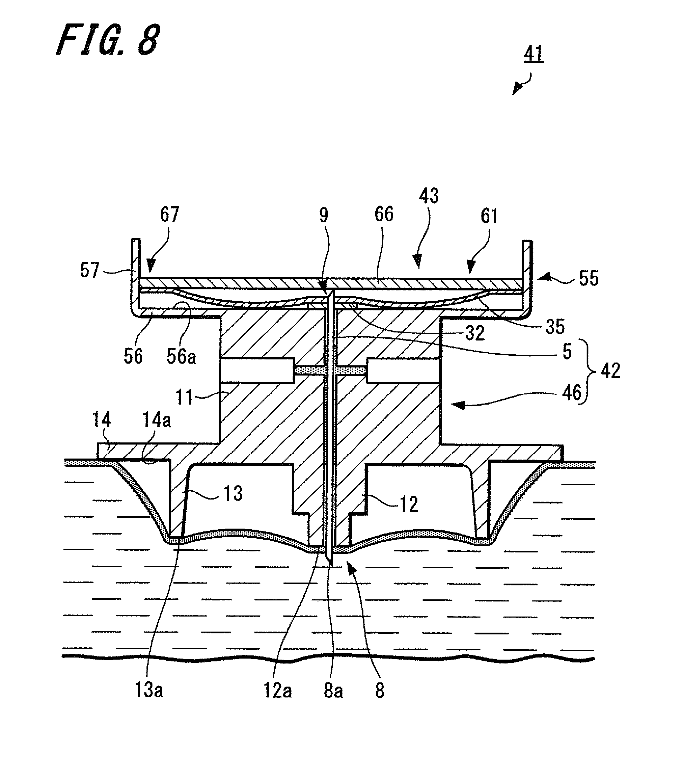

FIG. 6 is a cross-sectional view showing a state where the drug container 43 is mounted on the injection needle assembly 42, and the first needle tip 8 is stuck into a living body. FIG. 7 is a cross-sectional view showing a state where the second needle tip 9 has been penetrated through the contact portion 35 of the drug container 43. FIG. 8 is a cross-sectional view showing a state where drug administration of the drug injection apparatus 41 has been completed.

Before being used, the drug injection apparatus 41 is in a state where the injection needle assembly 42 and the drug container 43 are separated from each other (see FIG. 5). Thus, when using the drug injection apparatus 41, first, the drug container 43 is mounted on the drug container mount 55 of the injection needle assembly 42, and thereby the drug injection apparatus 41 is assembled.

Similar to the first embodiment, the drug injection apparatus 1 includes two assembly states: one is a first assembly state, and the other is a second assembly state.

### <First assembly state>

In the case where the drug injection apparatus 1 is to be assembled in the first assembly state, the drug container 43 is caused to face the drug container mount 55 of the injection needle assembly 42, and the position of the drug container 43 is adjusted so that the four sides of the flange piece 67 of the drug container 43 are respectively parallel to the four side plate portions 57 of the drug container mount 55.

Next, the drug container 43 is mounted on the drug container mount 55. Thus, the drug injection apparatus 41 is assembled in the first assembly state. At this time, the second needle tip 9 of the needle tube 5 is abutted against the seal member 32, but not penetrated through the contact portion 35. Further, the outer surface of the contact portion 35 curves so that the central portion thereof, to which the seal member 32 is attached, is recessed toward the side of the pressing portion 36 (see FIG. 6).

Further, the peripheral edge the flange piece 67 of the drug container 43 abuts the inner surface of each side plate portion 57 of the drug container mount 55. Thus, the movement of the drug container 43 in directions perpendicular to the axial direction of the needle-holding portion 11 is locked, and therefore the drug container 43 can be positioned.

Next, the drug injection apparatus 41 is moved substantially perpendicular to the skin, and the pressing portion 66 of the drug container 43 is pressed toward the side of the contact portion 35, so that the first needle tip 8 is stuck into the skin and the end face 13a of the stabilizer 13 is pressed against the skin (see FIG. 6). At this time, the first needle tip 8 is stuck into the skin, but the second needle tip 9 does not penetrate through the seal member 32. Further, since the needle-protruding surface 12a contacts the skin so as to cause the skin to deform flatly, the first needle tip 8 of the needle tube 5 can be inserted into the skin by the protruding length L only.

Next, the pressing portion 36 of the drug container 3 is pressed until the contact surface 14a of the guide portion 14 contacts the skin. Thus, the force for the stabilizer 13 to press against the skin becomes a predetermined value. As a result, the suitable pressing force of the stabilizer 13 can be recognized by the user, and the first needle tip 8 and the blade face 8a of the needle tube 5 can be reliably positioned in the upper layer of the skin. In such a manner, the guide portion 14 becomes a mark for recognizing the suitable pressing force of the stabilizer 13, and thereby the user can use the drug injection apparatus 1 with a sense of reassurance.

When the contact surface 14a of the guide portion 14 contacts the skin, the second needle tip 9 will penetrate through the seal member 32 and the contact portion 35, so that the needle hole of the needle tube 5 is brought into communication with the inside (i.e., the liquid chamber) of the container body 61 (see FIG. 7). Further, the pressing portion 36 is pressed until the pressing portion 36 abuts the second needle tip 9 inserted into the container body 31 (see FIG. 8). Thus, pressure is exerted on the inside of the container body 61, and thereby the drug M is injected into the upper layer of skin from the first needle tip 8 through the needle hole (not shown) of the needle tube 5.

At this time, the direction in which the needle tube 5 is stuck into the skin is identical to the direction in which the container body 61 (the pressing portion 66) is pressed. Thus, it is possible to easily press the container body 61 while stably holding the drug injection apparatus 41 in a state where the first needle tip 8 has been stuck into the living body. As a result, the drug M can be reliably administered into the upper layer of the skin from the first needle tip 8, and the amount of the drug M administered to the upper layer of skin can be stabilized. Further, since the sticking operation and the subsequent injecting operation can be performed as a sequence of actions, the operation can be facilitated, and it is possible to operate the drug injection apparatus 41 with one hand.

Further, the second needle tip 9 inserted into the container body 61 is abutted against but not penetrated through the pressing portion 66. Thus, it is possible to allow the drug M to be reliably discharged from the first needle tip 8 while preventing the second needle tip 9 from being stuck into the fingertip or the like that presses the pressing portion 66.

### <Second assembly state>

Next, the method for using the drug injection apparatus 41 in the case where the drug injection apparatus 41 is assembled in the second assembly state will be described below.
In the case where the drug injection apparatus 41 is to be assembled in the second assembly state, the drug container 43 is caused to face the drug container mount 55 of the injection needle assembly 42, and the position of the drug container 3 is adjusted so that the four sides of the flange piece 67 of the drug container 43 are respectively parallel to the four side plate portions 57 of the drug container mount 55.

Next, the drug container 43 is mounted on the drug container mount 55, and the pressing portion 66 of the drug container 43 is pressed, so that the second needle tip 9 of the needle tube 5 is penetrated through the seal member 32, and thereby the needle hole of the needle tube 5 is brought into communication with the inside (i.e., the liquid chamber) of the container body 61. As a result, the drug injection apparatus 41 is assembled in the second assembly state.

Next, the needle hub 46 of the injection needle assembly 42 is gripped to cause the end face 13a of the stabilizer 13 to face the skin, and further, the drug injection apparatus 1 is moved substantially perpendicular to the skin, so that the first needle tip 8 is stuck into the skin and the end face 13a of the stabilizer 13 is pressed against the skin. At this time, since the needle-protruding surface 12a contacts the skin so as to cause the skin to deform flatly, the first needle tip 8 of the needle tube 5 can be inserted into the skin by the protruding length L only.

Next, the gripped needle hub 46 is further pressed toward the side of the skin so as to bring the contact surface 14a of the guide portion 14 into contact with the skin (see FIG. 7). Thus, the force for the stabilizer 13 to press against the skin becomes a predetermined value. As a result, the suitable pressing force of the stabilizer 13 can be recognized by the user, and the first needle tip 8 and the blade face 8a of the needle tube 5 can be reliably positioned in the upper layer of the skin.

Further, the pressing portion 66 is pressed until the pressing portion 66 abuts the second needle tip 9 inserted into the container body 61 (see FIG. 8). Thus, the inner pressure of the container body 61 increases, and thereby the drug M is injected into the upper layer of the skin from the first needle tip 8 through the needle hole (not shown) of the needle tube 5.

Similar to the first assembly state, in the second assembly state, the direction in which the needle tube 5 is stuck into the skin is also identical to the direction in which the container body 61 (the pressing portion 66) is pressed. Thus, it is possible to easily press the container body 61 while stably holding the drug injection apparatus 41 in a state where the first needle tip 8 has been stuck into the living body. As a result, the drug M can be reliably administered into the upper layer of the skin from the first needle tip 8, and the amount of the drug M administered to the upper layer of skin can be stabilized.

Further, since the pressing portion 66 abuts the second needle tip 9, the second needle tip 9 inserted into the container body 61 will not penetrate through the pressing portion again. Thus, it is possible to prevent the second needle tip 9 from being stuck into the fingertip or the like that presses the pressing portion 36, without obstructing the discharge of the drug M.

In the present embodiment, the pressing portion 66 of the container body 61 is formed in a quadrangular plate-like shape. However, the present invention includes a possible configuration in which the inner surface of the of the pressing portion is formed as a spherical surface, and the mounting surface of the drug container mount is also formed as a spherical surface, wherein the curvature radius of the mounting surface of the drug container mount is equal to or larger than the curvature radius of the inner surface of the pressing portion. Further, the present invention includes a possible configuration in which the pressing portion is formed of a flexibly deformable material having hardness so as not to be penetrated by the second needle tip 9, so that when pressing the pressing portion, the pressing portion will deform according to the shape of the mounting surface.

### 3. Third embodiment

### [Drug injection apparatus]

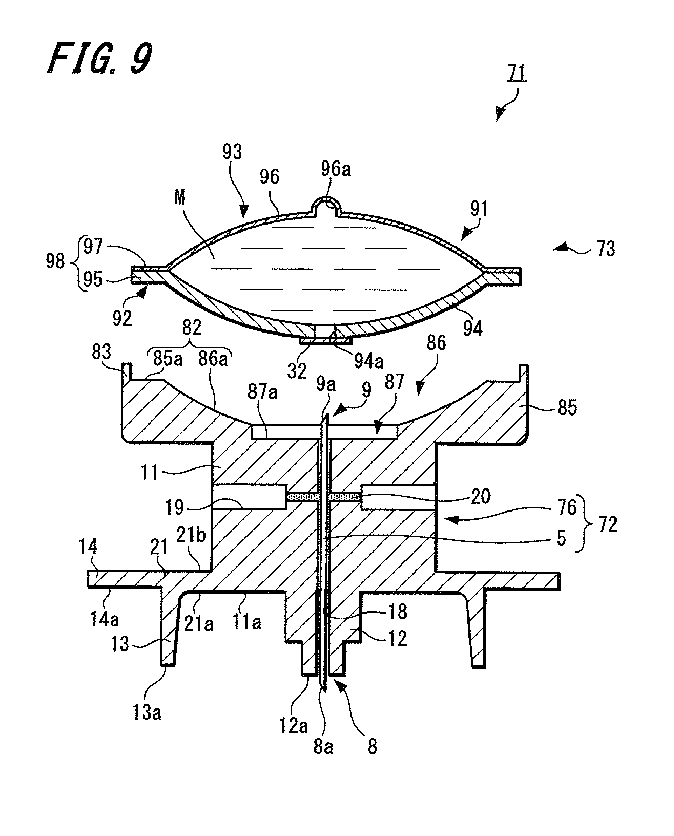

Next, a drug injection apparatus according to a third embodiment of the present invention will be described below with reference to FIG. 9.
FIG. 9 is a cross-sectional view showing the configuration of a drug injection apparatus 71 according to the third embodiment.

As shown in FIG. 9, the drug injection apparatus 71 of the third embodiment has the same configuration as that of the drug injection apparatus 1 (see FIG. 1) of the first embodiment. The drug injection apparatus 71 differs from the drug injection apparatus 1 in that a drug container 73 and a drug container mount 85 of the drug injection apparatus 71 are different from the drug container 3 and the drug container mount 15 of the drug injection apparatus 1. Thus, the description herein will be focused on the drug container 73 and the drug container mount 85; and components common to those of the drug injection apparatus 1 are denoted by the same reference numerals and the explanation thereof will not be repeated.

The drug injection apparatus 71 includes an injection needle assembly 72, and a drug container 73 to be mounted on the injection needle assembly 72. Similar to the drug injection apparatus 1, the drug injection apparatus 71 is used to stick a needle tip into a skin from the surface of the skin to inject a drug into an upper layer of the skin.

The injection needle assembly 72 includes a needle tube 5 having a hollow needle hole, and a needle hub 76 by which the needle tube 5 is fixed. The needle hub 76 includes a needle-holding portion 11 for holding the central portion of the needle tube 5, an adjusting portion 12, a stabilizer 13, a guide portion 14, and a drug container mount 85. Examples of the material of the needle hub 76 include synthetic resins such as polycarbonate, polypropylene, polyethylene and the like.

The drug container mount 85 is formed continuously from an end portion of the needle-holding portion 11 on the opposite side to the end face 11a. The drug container mount 85 is formed in a quadrangular plate-like shape having a predetermined thickness, and the center of the drug container mount 85 coincides with the axis of the needle-holding portion 11. A surface 85a (referred to an "upper surface 85a" hereinafter) of the drug container mount 85 on the opposite side to the needle-holding portion 11 is a flat surface perpendicular to the axial direction of the needle-holding portion 11.

A recessed portion 86 is formed in the upper surface 85a of the drug container mount 85. The recessed portion 86 is formed by forming a spherical surface 86a whose center is a point in the axis of the needle-holding portion 11. The upper surface 85a and spherical surface 86a of the drug container mount 85 serve as a mounting surface 82 to be contacted by a contact portion 92 of the drug container 73.

Further, a step portion 87 is formed in the spherical surface 86a of the drug container mount 85. The step portion 87 is provided in the central portion of the drug container mount 85, and has a bottom surface 87a. The second needle tip 9 of the needle tube 5 projects from the bottom surface 87a of the step portion 87. The length of the portion of second needle tip 9 projecting from the bottom surface 87a is at least equal to a value obtained by adding the thickness of the contact portion 92 and the thickness of the seal member 32 of the drug container 73 to the bevel length of the blade face 9a.

The four sides of the upper surface 85a are each provided with a locking piece 83 for preventing the drug container 73 from being moved (displaced) in directions perpendicular to the axial direction of the needle-holding portion 11. The locking pieces 83 are each formed in a plate-like shape substantially vertically projecting from the upper surface 85a. Incidentally, each locking piece 83 may also be formed in a pin-like (rod-like) shape.

### [Drug container]

Next, the drug container 73 will be described below.
The drug container 73 includes a container body 91 and a seal member 32.

The container body 91 includes a contact portion 92 and a pressing portion 93.
The contact portion 92 has a vessel-like shape, and includes a contact-side housing body 94 that forms the liquid chamber, and a joining piece 95 that is joined to the pressing portion 93. The contact-side housing body 94 has a dome-like shape bulging toward the side opposite to the pressing portion 93. The outer surface of the contact-side housing body 94 contacts the spherical surface 86a of the drug container mount 85. Thus, the curvature radius of the outer surface of the contact-side housing body 94 is substantially equal to the curvature radius of the spherical surface 86a of the drug container mount 85.

A through-hole 94a is formed in the central portion of the contact-side housing body 94, and the second needle tip 9 of the needle tube 5 is to be penetrated through the through-hole 94a. The through-hole 94a is sealed by the seal member 32 in a liquid-tight manner.

The joining piece 95 is formed by a frame projecting from the peripheral edge of the contact-side housing body 94, and has a quadrangular outline. One flat surface of the joining piece 95 contacts a joining piece 97 (which is to be described later) of the pressing portion 93 in a liquid-tight manner. The other flat surface of the joining piece 95 contacts the upper surface 85a of the drug container mount 85. Further, the peripheral edge of the joining piece 95 is engaged with the locking pieces 83 of the drug container mount 85.

The contact portion 92 has suitable rigidity, and thereby is inflexible. Here, the term of "inflexible" is defined as a property that is undeformable even if a pressing force is applied to the drug container 73. Examples of the material of the contact portion 92 include synthetic resins such as polypropylene (PP), polyethylene (PE), polystyrene (PS), ABS resin, polyvinyl chloride (PVC), polycarbonate (PC) and the like. The contact portion 92 may also be formed of a metal such as iron, stainless steel or the like.

The pressing portion 93 includes a pressing-side housing body 96 which forms the liquid chamber, and a joining piece 97 which is joined to the joining piece 95 of the contact portion 92. The pressing-side housing body 96 has a dome-like shape bulging toward the side opposite to the contact-side housing body 94. The pressing-side housing body 96 is flexible, and is formed so that it will yield so as to be reversed (i.e., be buckled) when subjected to a pressure equal to or higher than a certain level.
When the pressing-side housing body 96 is buckled, the pressing-side housing body 96 will reverse so as to become convex toward the side of the contact-side housing body 94, so that the inner surface of the pressing-side housing body 96 contacts the inner surface of the contact-side housing body 94.

A needle housing portion 96a (which is a second concrete example of the penetration avoiding portion) is provided in the central portion of the pressing-side housing body 96. The needle housing portion 96a is a recessed portion formed continuously from the inner surface of the pressing-side housing body 96, and is adapted to house the second needle tip 9 inserted into the container body 91 when the pressing-side housing body 96 is buckled.

By arranging the needle housing portion 96a in the pressing-side housing body 96, the second needle tip 9 can be prevented from penetrating through the pressing portion 93. Further, when the pressing-side housing body 96 is buckled, since the inner surface of the pressing-side housing body 96 can be caused to contact the inner surface of the contact-side housing body 94, the amount of the drug remaining in the container body 91 can be reduced.

The pressing portion 93 is formed of a material so that it will yield so as to be reversed when subjected to a pressure equal to or higher than a certain level. Examples of the material of the pressing portion 93 include metals, such as iron, stainless steel and the like, and synthetic resins, such as polypropylene (PP), polyethylene (PE), polystyrene (PS), ABS resin and the like.

The joining piece 95 of the contact portion 92 and the joining piece 97 of the pressing portion 93 are joined to each other in a liquid-tight manner by a fixing method such as adhesive, ultrasonic welding or the like. The piece 95 of the contact portion 92 and the joining piece 97 of the pressing portion 93 are superimposed on each other and joined to each other, so that a flange piece 98 is formed in the container body 91. Further, the internal space formed by the contact-side housing body 94 and the pressing-side housing body 96 becomes a liquid chamber filled with a drug M.

### [Method for using drug injection apparatus]

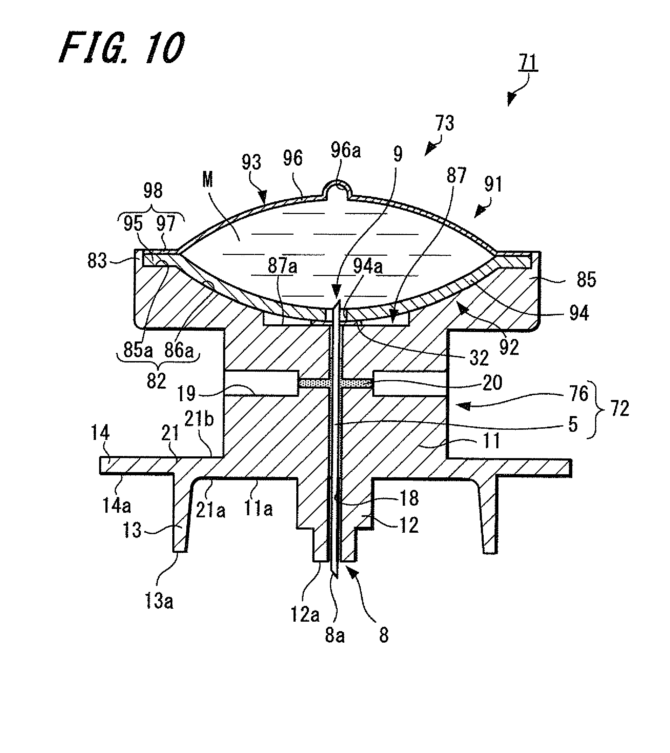

Next, a method for using the drug injection apparatus 71 will be described below with reference to FIGS. 10 to 12.

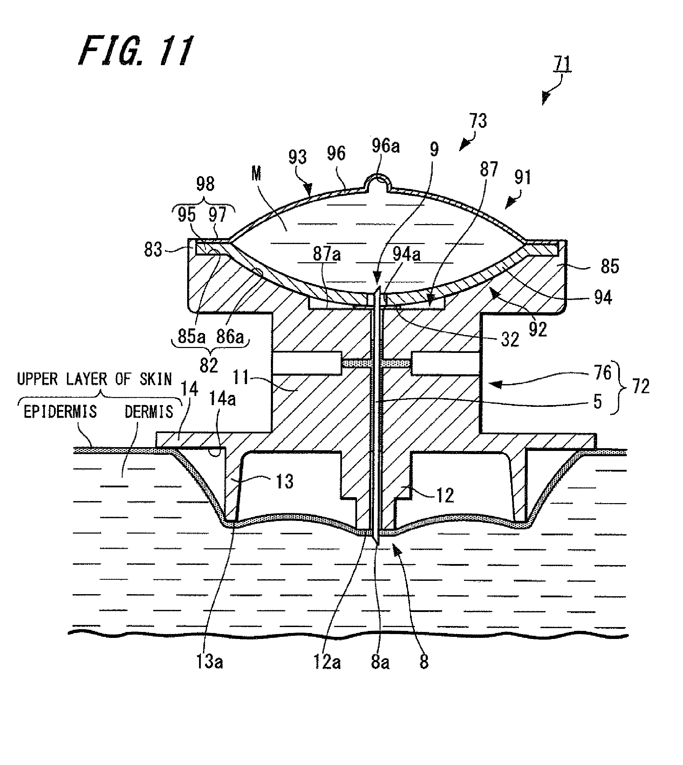

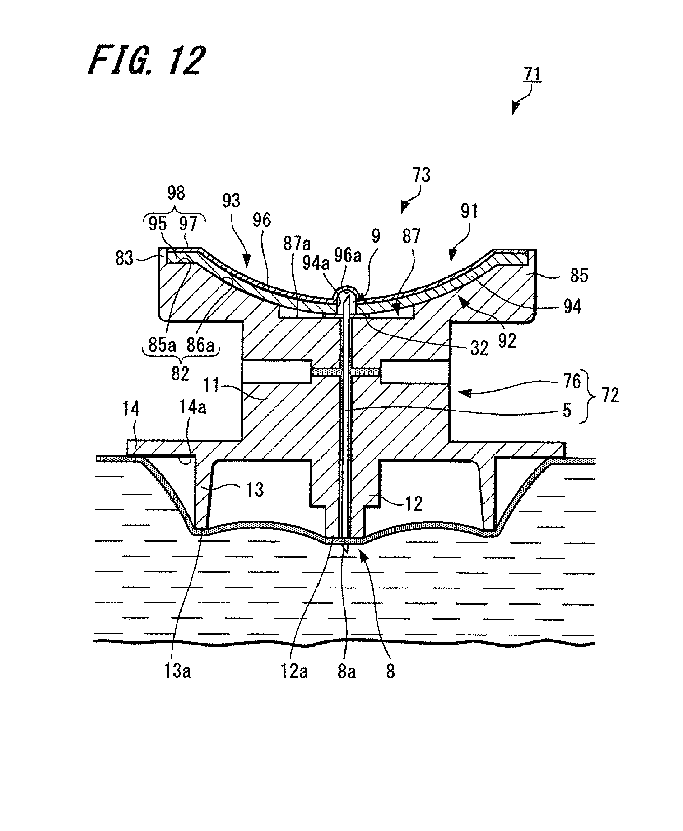

FIG. 10 is a cross-sectional view showing a state where the drug container 73 is mounted on the injection needle assembly 72. FIG. 11 is a cross-sectional view showing a state where the first needle tip 8 is stuck into a living body. FIG. 12 is a cross-sectional view showing a state where drug administration of the drug injection apparatus 71 has been completed.

Before being used, the drug injection apparatus 71 is in a state where the injection needle assembly 72 and the drug container 73 are separated from each other (see FIG. 9). Thus, when using the drug injection apparatus 71, first, the drug container 73 is mounted on the drug container mount 85 of the injection needle assembly 72, and thereby the drug injection apparatus 71 is assembled.

When assembling the drug injection apparatus 71, the drug container 73 is caused to face the drug container mount 85 of the injection needle assembly 72, and the position of the drug container 73 is adjusted so that the four sides of the flange piece 98 of the drug container 73 are respectively parallel to the four locking pieces 83 of the drug container mount 85.

Next, the drug container 73 is mounted on the drug container mount 85, so that each edge of the flange piece 98 is pressed against the upper surface 85a of the drug container mount 85. Thus, each edge of the flange piece 98 contacts the upper surface 85a of the drug container mount 85, so that the contact-side housing body 94 of the drug container 73 contacts the spherical surface 86a of the drug container mount 85. Further, the second needle tip 9 of the needle tube 5 is penetrated through the seal member 32, and thereby the needle hole of the needle tube 5 is brought into communication with the inside (i.e., the liquid chamber) of the container body 91. As a result, the assembly of the drug injection apparatus 71 is completed.

When the drug injection apparatus 71 is assembled, the four edges of the flange piece 98 of the drug container 73 respectively abut the four locking pieces 83 of the drug container mount 85. Thus, the movement of the drug container 73 in directions perpendicular to the axial direction of the needle-holding portion 11 is inhibited, and therefore the drug container 73 can be positioned.

Next, the needle hub 76 of the injection needle assembly 72 is gripped to cause the end face 13a of the stabilizer 13 to face the skin, and further, the drug injection apparatus 71 is moved substantially perpendicular to the skin, so that the first needle tip 8 is stuck into the skin and the end face 13a of the stabilizer 13 is pressed against the skin. At this time, since the needle-protruding surface 12a contacts the skin so as to cause the skin to deform flatly, the first needle tip 8 of the needle tube 5 can be inserted into the skin by the protruding length L only.

Next, the gripped needle hub 76 is further pressed toward the side of the skin so as to bring the contact surface 14a of the guide portion 14 into contact with the skin (see FIG. 11). Thus, the force for the stabilizer 13 to press against the skin becomes a predetermined value. As a result, the suitable pressing force of the stabilizer 13 can be recognized by the user, and the first needle tip 8 and the blade face 8a of the needle tube 5 can be reliably positioned in the upper layer of the skin.

Next, the pressing portion 93 (the pressing-side housing body 96) of the drug container 73 is pressed. Thus, the pressing-side housing body 96 is buckled, so that the pressing-side housing body 96 deforms (reverses) so as to become convex toward the side of the contact-side housing body 94. As a result, the drug M is injected into the upper layer of the skin from the first needle tip 8 through the needle hole (not shown) of the needle tube 5.

Similar to the drug injection apparatus 1 of the first embodiment, in the drug injection apparatus 71, the direction in which the needle tube 5 is stuck into the skin is identical to the direction in which the container body 91 (the pressing portion 93) is pressed. Thus, it is possible to easily press the container body 91 while stably holding the drug injection apparatus 71 in a state where the first needle tip 8 has been stuck into the living body. As a result, the container body 91 can be simply deformed, and the drug M can be reliably discharged to the upper layer of the skin from the first needle tip 8.

Further, when the pressing-side housing body 96 is buckled, the second needle tip 9 inserted into the container body 91 is housed in the needle housing portion 96a. Thus, the second needle tip 9 will not penetrate through the pressing portion 93. Thus, discharge of the drug M will not stop in the middle, and the second needle tip 9 will not be stuck into the fingertip or the like that presses the pressing portion 93.
Further, it is possible to cause the inner surface of the pressing-side housing body 96 of the pressing portion 93, which is buckled, to contact the inner surface of the contact-side housing body 94. As a result, the amount of the drug remaining in the drug container 91 can be reduced.

Further, after the pressing portion 93 has been reversed caused by buckling, even if pressing force applied to pressing portion 93 is reduced, a predetermined pressure will be applied to the inside of the container body 91 until the deformation of the pressing portion 93 is completed, and therefore the injection of the drug will be continued in a stable manner.

In the present embodiment, the assembly state of the drug injection apparatus 71 is a state where the second needle tip 9 of the needle tube 5 is penetrated through the seal member 32. However, similar to the drug injection apparatus 1 of the first embodiment, in the drug injection apparatus 71, the assembly state of the drug injection apparatus 71 may also be a state where the second needle tip 9 of the needle tube 5 is abutted against but not penetrated through the seal member 32.
In such a case, when pressing the pressing-side housing body 96, the first needle tip 8 of the needle tube 5 is stuck into the living body, then the second needle tip 9 is penetrated through the seal member 32, and finally the pressing-side housing body 96 yields so as to be reversed.

In the present embodiment, the spherical surface 86a is formed as a portion of the mounting surface 82 of the drug container mount 85; however, the mounting surface of the drug container mount may also be formed in any shape without conforming to the shape of the contact portion of the drug container. For example, the mounting surface of the drug container mount may also be formed as a flat surface.

In the present embodiment, the contact-side housing body 94 and the pressing-side housing body 96 of the drug container 73 are each formed in a dome-like shape. However, the contact-side housing body and the pressing-side housing body of the drug container of the present invention may also each be formed in a truncated-cone shape. In such a case, when a pressure equal to or higher than a certain level to applied to the pressing-side housing body, the pressing-side housing body will yield so as to be reversed, so that the drug will be discharged from the first needle tip of the needle tube.

In the present embodiment, the pressing portion 93 of the drug container 73 is formed so as to be buckled; however, the pressing portion of the drug container of the present invention is not such limited, but may also have the same configuration as that of, for example, the pressing portion 36 of the first embodiment. In such a case, similar to the first embodiment, a protective plate 33 needs to be attached to the pressing portion.

### 4. Fourth embodiment

### [Drug injection apparatus]

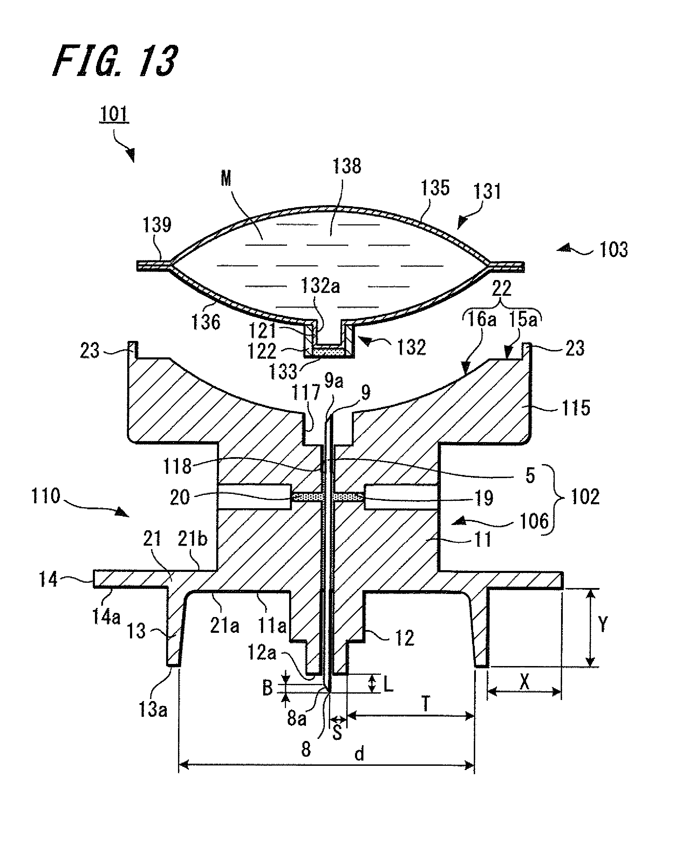

Next, a drug injection apparatus 101 according to a fourth embodiment of the present invention will be described below with reference to FIG. 13.
FIG. 13 is a cross-sectional view showing the configuration of the drug injection apparatus 101 of the fourth embodiment, and showing a state of a drug container 103 and an injection needle assembly 102 before use.

As shown in FIG. 13, the drug injection apparatus 101 includes a drug container 103 having a drug M enclosed therein, and an injection needle assembly 102 having a needle tube 5 incorporated therein. The drug injection apparatus 101 is configured in a manner in which a user directly presses the drug container 103 to cause it to deform, and thereby the drug M is administered.

In the present embodiment, the drug injection apparatus 101 is described based on an example in which the drug injection apparatus 101 is used to stick a needle tip into a skin from the surface of the skin to inject a drug into the upper layer of the skin.
In the following paragraphs, the configuration of the injection needle assembly 102 and the drug container 103, as well as a method for using the drug injection apparatus 101, will be described.

### [Injection needle assembly]

The injection needle assembly 102 includes a needle tube 5 having a hollow needle hole, and a needle hub 106 by which the needle tube 5 is fixed.

Similar to the first to third embodiments, the needle tube 5 is a double-ended needle. In other words, the needle tube 5 has a first needle tip 8 for being stuck into a living body, and a second needle tip 9 for penetrating through a seal member 133 (which is to be described later) of the drug container 103. Since the details of the needle tube 5 have been described above when discussing the first embodiment, the description will not be repeated herein.

Next, the needle hub 106 will be described below.
The needle hub 106 includes a needle-holding portion 11 for holding the central portion of the needle tube 5, a drug container mount 115 provided on one end of the needle-holding portion 11, an adjusting portion 12 provided on the other end of the needle-holding portion 11, a stabilizer 13, and a guide portion 14.

Examples of the material of the needle hub 106 include various resins identical to those of the needle hub 6 of the first embodiment.
Further, since the details of needle-holding portion 11, the adjusting portion 12, the stabilizer 13 and the guide portion 14, which configure the needle hub 106, have been described above when discussing the first embodiment, the description will not be repeated herein.

The drug container mount 115 is configured by a quadrangular plate-like member having an insertion-hole 117 formed in the central portion thereof, and is provided on one end of the needle-holding portion 11. A surface of the drug container mount 115 on the opposite side to the needle-holding portion 11 serves as a mounting surface 22 on which the drug container 103 is mounted. The mounting surface 22 includes a concave spherical surface 16a and an upper surface 15a, wherein the upper surface 15a is formed continuously from the outer edge of the concave spherical surface 16a. The diameter of the concave spherical surface 16a (which is curved in a spherical shape) is substantially equal to the diameter of a liquid chamber (which is to be described later) of the drug container 103.

Further, the insertion-hole 117 is opened to a predetermined depth from the central portion of the drug container mount 115 so that a through-hole 118 of the needle-holding portion 11 can be seen from the mounting surface 22. As will be described later, the insertion-hole 117 is an opening for being inserted by a needle puncture portion 132 of the drug container 103 when using the drug injection apparatus 101.

Further, the four sides of the edge portion of the mounting surface 22 are each provided with a locking piece 23 for determining the mounting position of the drug container 103 when mounting the drug container 103, and preventing the drug container 103 from being displaced in the surface direction of the mounting surface 22. The locking pieces 23 are each formed in a plate-like shape substantially vertically projecting from the upper surface 15a, and it is preferred that the height of the locking piece 23 is larger than the height of a flange piece 139 (which is to be described later) of the drug container 103. Incidentally, each locking piece 23 may also be formed in a pin-like (rod-like) shape.

The through-hole 118, through which the needle tube 5 is inserted, is formed in the axes of the needle-holding portion 11 and the adjusting portion 12, in a section from the bottom of the insertion-hole 117 of the drug container mount 115 to the needle-protruding surface 12a of the adjusting portion 12. An injection hole 19 for injecting an adhesive 20 into the through-hole 118 is formed in the needle-holding portion 11. The injection hole 19 is opened from the outer circumferential surface of the needle-holding portion 11 so that the injection hole 19 is brought into communication with the through-hole 118. In the needle-holding portion 11, the needle tube 5 is fixed to the needle-holding portion 11 by the adhesive 20 injected from the injection hole 19 into the through-hole 118, so that the needle tube 5 is fixed to penetrate through the needle-holding portion 11 and the adjusting portion 12.

Further, the needle tube 5 is fixed so that the first needle tip 8 of the needle tube 5 projects from the needle-protruding surface 12a of the adjusting portion 12 by a predetermined length, and the second needle tip 9 of the needle tube 5 projects in the insertion-hole 117 of the drug container mount 115 by a predetermined length but does not project above the mounting surface 22. In other words, in the present embodiment, the protruding length of the second needle tip 9 is set to a length shorter than the depth of the insertion-hole 117.

Here, the protruding length of the second needle tip 9 projecting in the insertion-hole 117 is at least equal to a value obtained by adding the thickness of a second member 136, which configures the drug container 103, and the thickness of the seal member 133 to the bevel length of the blade face 9a. Thus, the needle tube 5 can be reliably brought into communication with the inside of the drug container 103.

The surface of the drug container mount 115 on the side of the needle-holding portion 11 extends outward from the needle-holding portion 11 in the radial direction. A gripping portion 110 is formed by the surface of the drug container mount 115 on the side of the needle-holding portion 11, the flat surface 21b of the flange 21, and the outer circumferential surface of the needle-holding portion 11. The gripping portion 110 is formed in a bobbin-like shape and has a circumferentially-recessed portion. When using the drug injection apparatus 101, the user can grip the drug injection apparatus 101 by, for example, putting his (or her) index finger and middle finger on the recessed portion of the gripping portion 110 so that the needle-holding portion 11 is sandwiched by the both fingers. Since the gripping portion 110 is recessed, the fingers of the user are stabilized, so that the drug injection apparatus 101 can be easily gripped by the user.

### [Drug container]

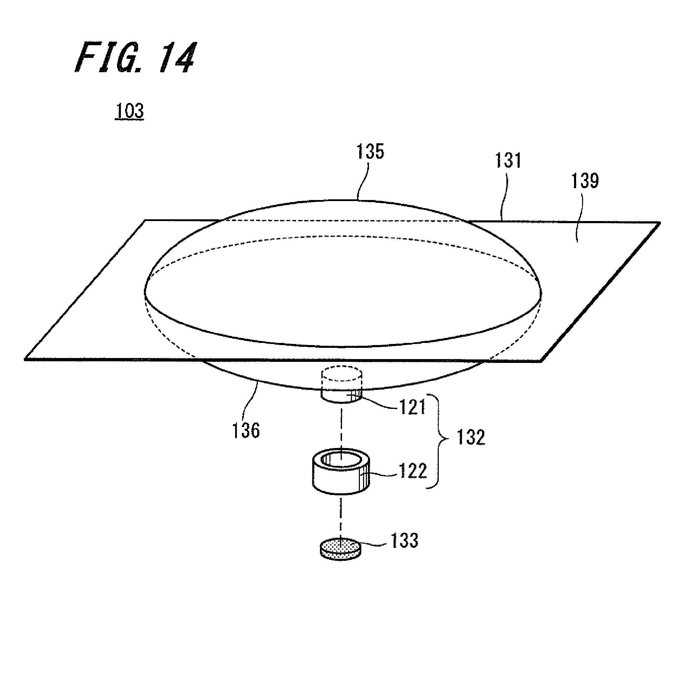

Next, the drug container 103 will be described below. FIG. 14 is an exploded perspective view of the drug container 103 used in the drug injection apparatus 101 of the present embodiment.
The drug container 103 includes a container body 131 having the drug M enclosed therein, a needle puncture portion 132 into which the needle is inserted from the outside of the drug container 103, and the seal member 133 provided on the tip end of the needle puncture portion 132.

The container body 131 includes a sheet-like first member 135 and a sheet-like second member 136, wherein the first member 135 is the side to be pressed, and the second member 136 is the side on which the needle puncture portion 132 is formed.
The first member 135 and the second member 136 are each formed of a transparent flexible material. Examples of the transparent flexible material include soft resin materials such as, for example, polypropylene (PP), polyethylene (PE), ethylene-vinyl acetate copolymer (EVA) and the like. The first member 135 and the second member 136 may also each be formed of a material obtained by blending styrene thermoplastic elastomer, olefinic thermoplastic elastomer and/or the like into polyethylene or polypropylene, and then softening the blend material.

Further, a bag-like container body 131 is formed by attaching the edge portion of the first member 135 and the edge portion of the second member 136 to each other; and the internal space of the bag-like container body 131 becomes a liquid chamber 138 to be filled with the drug M. In the present embodiment, the container body 131 is formed in a bag-like shape by joining the edge portion of the sheet-like first member 135 and the edge portion of the second member 136 in a liquid-tight manner by means of, for example, heat fusion, ultrasonic welding or the like.

By joining the first member 135 and the second member 136 to each other, the edge portion of the first member 135 and the edge portion of the second member 136, which serve as joining faces, configure a flange piece 139. The flange piece 139 is a portion to be effectively used when mounting the drug container 103 on the injection needle assembly 102.
In the present embodiment, a quadrangular sheet is used as each of the first member 135 and the second member 136, and the first member 135 and the second member 136 are joined to each other so that the outline of the liquid chamber 138 is substantially a circle when viewed in the direction perpendicular to the joining faces. Thus, the outline of the flange piece 139 is a quadrangle.

Further, a projecting portion 121 is formed in the central portion of the second member 136, wherein the projecting portion 121 projects toward the outside of the container body 131. The projecting portion 121 configures the needle puncture portion 132. The projecting portion 121 includes a tube hole that comes into communication with the inside of the container body 131, and the inner surface of the projecting portion 121 is formed continuously from the inner surface of the container body 131.

The container body 131 can be produced by injection molding, extrusion molding, calender molding, blow molding, inflation molding or the like.

The tensile elasticity and the thickness of the flexible material applied to both the first member 135 and the second member 136 may be set in the same manner as those of the contact portion 35 and the pressing portion 36 of the drug container 3 of the first embodiment. Further, the inner surface of the container body 131 may be coated with a film, such as a low-density polyethylene laminated film.

Further, it is preferred that the container body 131 formed in the aforesaid manner has water vapor barrier property. Since the container body 131 has water vapor barrier property, not only water inside the container body 131 can be prevented from being evaporated, but also outside water vapor outside the container body 131 can be prevented from entering the container body 131.
The level of the water vapor barrier property of the container body 131 may be set in the same manner as that of the container body 31 of the drug container 3 of the first embodiment.

The needle puncture portion 132 includes the projecting portion 121 formed in the second member 136, and a ring-shaped guard portion 122 surrounding the side portion of the projecting portion 121; and the needle puncture portion 132 is formed so as to project toward the direction in which the container body 131 is pressed.

The guard portion 122 is formed of an inflexible material. The inner diameter of the guard portion 122 is substantially equal to the outer diameter of the projecting portion 121 of the second member 136. The guard portion 122 is attached to the projecting portion 121 by ultrasonic welding. Here, the term of "inflexible" is defined as a property that is undeformable when a force is applied to press the drug container. The length of the guard portion 122 in the axial direction is substantially equal to the depth of the insertion-hole 117. Further, the inner diameter of the guard portion 122 is set to a value smaller than the width of the finger of the user who presses the drug container 103, but large than the outer diameter of the second needle tip 9 of the needle tube 5 because the guard portion 122 is a portion to be inserted by the second needle tip 9. Considering the deviation generated when mounting the drug container 103 on the injection needle assembly 102, it is preferred that the inner diameter of the guard portion 122 is set in a range of 2 to 8 mm.

The constituent material of the guard portion 122 may be an inflexible material (i.e., a material that does not deform when a force is applied to press the drug container 103); for example, the guard portion 122 may be formed of the same material as the constituent material of the needle hub 106.

The seal member 133 is attached to the tip end of the projecting portion 121 configured by the second member 136. Similar to the seal member of the first to third embodiments, in order to achieve good liquid tightness between the seal member 133 and the needle tube 5, it is preferred that the seal member 133 is formed of a material having rubber elasticity.

In the present embodiment, the drug injection apparatus 101 is configured so that, when using the drug injection apparatus 101, the second needle tip 9 of the needle tube 5 held by the injection needle assembly 102 penetrates into the drug container 103 from the side of the seal member. The seal member 133 has an effect of coming close contact with the circumferential surface of the needle tube 5 in a liquid-tight manner, thereby preventing the drug M inside the drug container 103 from being leaked from the gap between the second needle tip 9 and the container body 131. Further, the seal member 133 is formed of a material so as to have such a penetration resistance that, if not being pressed by a pressure of a certain level, the seal member 133 can not be penetrated by the second needle tip 9; and the penetration resistance of the seal member 133 to the second needle tip 9 is larger than the resistance of the living body to the first needle tip 8.

The drug container 103 may be produced by forming the container body 131 and then filling the drug M, or by forming the container body 131 in a stated in which the drug M has been filled. In the case when the drug container 103 is produced by forming the container body 131 in a stated in which the drug M has been filled, the container body 131 can be produced by blow fill molding method in which molding (blow), filling and sealing are performed in sequence.

### [Method for using drug injection apparatus]

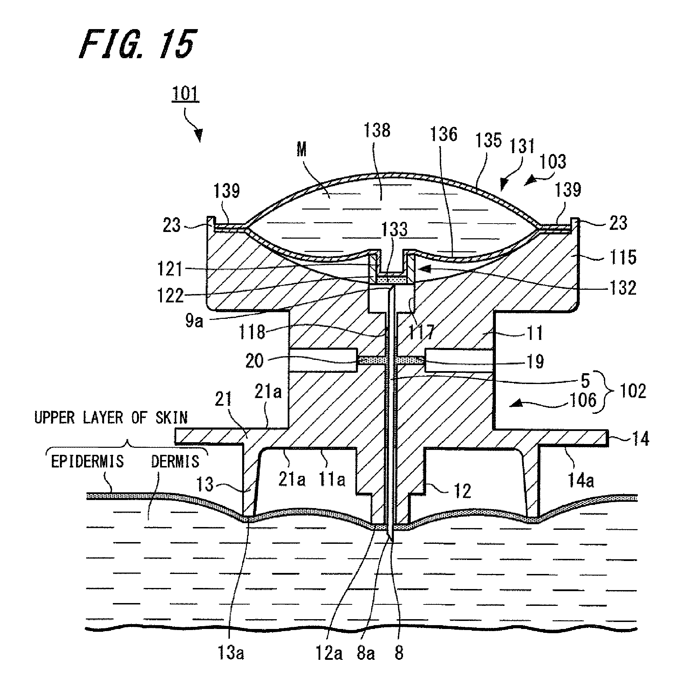

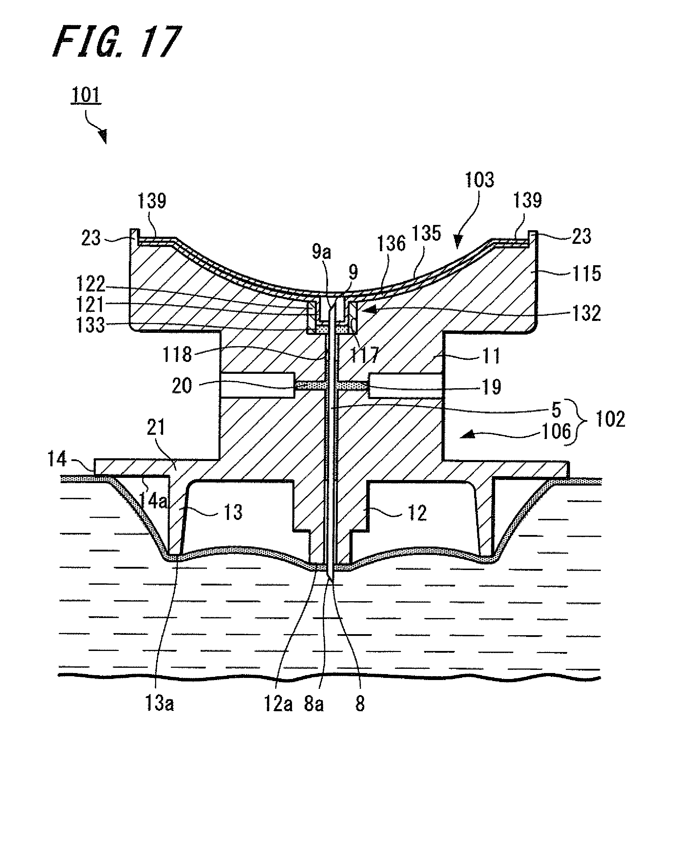

Next, a method for using the drug injection apparatus 101 of the present embodiment will be described below. FIG. 15 is a cross-sectional view showing a state where the drug container 103 is mounted on the injection needle assembly 102, and the first needle tip 8 is stuck into a living body. FIG. 16 is a cross-sectional view showing a state where the second needle tip 9 is penetrated through the seal member 133 of the drug container 103 so as to be inserted into the needle puncture portion 132. FIG. 17 is a cross-sectional view showing a state where administration of the drug M performed by the drug injection apparatus 101 has been completed.

Before being used, the drug injection apparatus 101 is in a state where the injection needle assembly 102 and the drug container 103 are separated from each other (see FIG. 13). Thus, when using the drug injection apparatus 101, first, the drug container 103 is mounted on the mounting surface 22 of the drug container mount 115 so that the seal member 133 of the drug container 103 is abutted against the second needle tip 9. Since the seal member 133 has a certain level of resistance to the force for the second needle tip 9 to try to penetrate through the seal member 133, the seal member 133 will not be penetrated by the second needle tip 9 at the stage where the drug container 103 has been mounted on the mounting surface 22. Further, at this time, since the flange piece 139 of the drug container 103 is locked by the locking pieces 23 formed on the edge of the mounting surface 22, the drug container 103 is positioned, so that the drug container 103 is prevented from being displaced in the surface direction of the mounting surface 22.
Thus, the drug container 103 is set onto the injection needle assembly 102, and thereby the preparation of the drug injection apparatus 101 is completed.

Next, the end face 13a of the stabilizer 13 is caused to face the skin while maintaining the state where the drug container 103 is mounted on the mounting surface 22, and thereby the first needle tip 8 of the needle tube 5 is caused to face the skin to be stuck.

Next, as shown in FIG. 15, the first member 135 of the drug container 103 is pressed toward the side of the second member 136, and thereby the injection needle assembly 102 is substantially perpendicularly moved toward the side of the skin, so that the first needle tip 8 is stuck into the skin and the end face 13a of the stabilizer 13 is pressed against the skin. Incidentally, in the present embodiment, the user presses the first member 135 of the drug container 103 with his (or her) finger, and thereby a pressing force occurs to the drug injection apparatus 101.

At this time, since the seal member 133 is formed of a material so that the resistance of the seal member 133 to the second needle tip 9 is larger than the resistance of the living body to the first needle tip 8, the first needle tip 8 is stuck into the living body before the second needle tip 9 penetrates through the seal member 133.

Here, the needle-protruding surface 12a of the adjusting portion 12 is coplanar with the end face 13a of the stabilizer 13. Thus, the needle-protruding surface 12a of the adjusting portion 12 can contact the skin so as to cause the skin to deform flatly, and therefore the first needle tip 8 of the needle tube 5 can be inserted into the skin by the protruding length L only.

Next, the first member 135 of the drug container 103 is further pressed toward the side of the second member 136, and thereby the drug injection apparatus 101 is pressed toward the side of the living body, so that the contact surface 14a of the guide portion 14 is brought into contact with the skin. Here, the value of the height of the guide portion Y is set so that the skin can be stuck by a suitable pressing force applied from the needle tube 5 and the stabilizer 13, so as to inject the drug into the skin. Thus, the force for the stabilizer 13 to press against the skin becomes a predetermined value. As a result, it is possible to guide the pressing force of the stabilizer 13 for the user as well as to press the stabilizer 13 against the skin with a proper pressing force, and therefore the first needle tip 8 and the blade face 8a of the needle tube 5 can be reliably positioned in the upper layer of the skin.

When the first member 135 of the container body 131 is being pressed toward the side of the second member 136 until the contact surface 14a of the guide portion 14 contacts the skin, the pressing force is larger than the resistance of the seal member 133 to the second needle tip 9. Thus, as shown in FIG. 16, the second needle tip 9 is penetrated through the seal member 133, and the needle puncture portion 132 of the drug container 103 is inserted into the insertion-hole 117 formed in the drug container mount 115. At this time, since the depth of the insertion-hole 117 is substantially equal to the protruding length of the needle puncture portion 132, the needle puncture portion 132 is inserted into insertion-hole 117 until the tip end of the needle puncture portion 132 abuts the bottom of the insertion-hole 117, and thereby the second needle tip 9 is inserted into the needle puncture portion 132. Since the protruding length of the second needle tip 9 within the insertion-hole 117 is set to a value smaller than the depth of the insertion-hole 117, the insertion length of the second needle tip 9 into the needle puncture portion 132 is smaller than the protruding length of the needle puncture portion 132, and therefore the second needle tip 9 will not project from the mounting surface 22.
By performing the above operation, the liquid passage of the drug M enclosed in the drug container 103 into the needle tube 5 is completed.

Next, the user further presses the first member 135 of the drug container 103 toward the side of the second member 136, and thereby the flexible first member 135 is caused to deform toward the side of the second member 136. Thus, the inner pressure of the container body 131 increases, and thereby the drug M enclosed in the container body 131 is discharged into the living body from the first needle tip 8 through the second needle tip 9 of the needle tube 5. Further, as shown in FIG. 17, the first member 135 is pressed until the first member 135 is brought into close contact with the second member 136, and thereby the drug M in the drug container 103 is completely discharged, so that the administration of the drug M is completed.

In the drug injection apparatus 101 of the present embodiment, the direction in which the drug injection apparatus 101 is pressed to stick the first needle tip 8 into the living body is identical to the direction in which the drug container 103 is pressed to discharge the drug M in the container body 131 from the first needle tip 8. Thus, by pressing the first member 135 of the drug container 103 toward the side of the second member 136, the drug injection apparatus 101 is pressed against the living body, and the drug M in the container body 131 is discharged from the first needle tip 8. Thus, since it is possible to discharge the drug M while stably maintaining the pressing force of the first needle tip 8 toward the living body, the user can administer the drug M in a stable and easy manner.

In the above description, although the method for using the drug injection apparatus 101 is described on a step-by-step basis, the entire operation from the step of sticking the first needle tip 8 into the living body to the step of discharging the drug M can be successively performed by pressing the drug container 103 in a direction from the first member 135 to the second member 136. Thus, actually the time for performing a sequence of operations can be reduced. Further, since operation is easy, the operation from the step of sticking the living body to the step of administering the drug can be performed by one hand.

Further, in the drug injection apparatus 101 of the present embodiment, the direction in which the first needle tip 8 is stuck into the living body is identical to the direction in which the drug container 103 is pressed to discharge the drug M, and therefore when discharging the drug, the drug injection apparatus 101 has to be operated so that the finger of the user comes close to the second needle tip 9. However, in the present embodiment, the needle puncture portion 132, which projects outward, is formed on the side of the second member 136 of the drug container 103, and when the needle puncture portion 132 is inserted into the insertion-hole 117 of the drug container mount 115, the second needle tip 9 will not project out from side of the mounting surface 22. Thus, even if the finger of the user is abutted against the mounting surface 22 through the first member 135 and the second member 136, the second needle tip 9 will not penetrate through the flexible first member 135 to stick into the finger of the user. Thus, safety of the user can be ensured.

In the present embodiment, the insertion-hole 117 possible to be inserted by the needle puncture portion 132 of the drug container 103 is formed, however, the insertion-hole 117 does not have to be formed. In the present embodiment, the needle puncture portion 132 is configured by the guard portion 122 formed of an inflexible material, and the inner diameter of the guard portion 122 is smaller than the average diameter of the finger. Thus, in the case where the first member 135 is pressed toward the side of the second member 136 to discharge the drug M, the discharge of the drug M is completed at the time when the finger abuts the surface of the inflexible needle puncture portion 132 on the side of the container body 131. At this time, since the insertion length of the second needle tip 9 is smaller than the length of a tube hole 132a of the needle puncture portion 132, the second needle tip 9 will not project above the needle puncture portion 132, and therefore accidental sticking to the user can be prevented.

In the case where the insertion-hole 117 is formed, like the case of the present embodiment, the user can press the first member 135 until his (or her) finger abuts the concave spherical surface 16a of the mounting surface 22, and therefore not only safety can be improved, but also the first member 135 can be pressed until it is brought into closer contact with the second member 136, so that the residual liquid can be reduced.

Further, in the present embodiment, since the concave spherical surface 16a of the mounting surface 22 is formed in a curved shape, when the first member 135 has been pressed toward the second member 136, the finger of the user will closely abut the concave spherical surface 16a through the flexible first member 135 and the second member 136. Thus, the drug M filled in the container body 131 can be sufficiently discharged, and therefore residual liquid can be reduced.

In the present embodiment, the drug M is filled in the drug container 103 formed of a flexible material, and the drug M can be administered by pressing the drug container 103 with a finger. Thus, the configuration of the drug injection apparatus 101 of the present embodiment is simpler than that of a conventional syringe in which the drug in an inflexible drug container is discharged by pressing a pusher. Thus, with the present embodiment, cost can be reduced compared to the conventional syringe, and the present embodiment is particularly effective to be applied to the case where the drug injection apparatus is filled with a small amount of drug.

### 5. Fifth embodiment

### [Drug injection apparatus]

Next, a drug container according to a fifth embodiment of the present invention, and a drug injection apparatus which uses the drug container will be described below.
FIG. 18 is a cross-sectional view showing the configuration of a drug injection apparatus 141 of the fifth embodiment, and showing a state of a drug container 143 and an injection needle assembly 102 before using the drug injection apparatus 141. The present embodiment differs from the first embodiment in that the configuration of the drug container 143 of the present embodiment is different from the configuration of the drug container of the first embodiment. In FIG. 18, components corresponding to those of FIG. 13 are denoted by the same reference numerals, and the explanation thereof will not be repeated.

### [Drug container]

First, the configuration of the drug container 143 will be described below. The drug container 143 of the present embodiment includes a container body 151 having the drug M enclosed therein, a needle puncture portion 152 into which the needle is inserted from the outside of the drug container 143, and a seal member 153 provided on the tip end of the needle puncture portion 152.

The container body 151 includes a sheet-like flexible first member 135, and a second member 155. The first member 135 and the second member 155 each have a quadrangular shape. Similar to the fourth embodiment, the first member 135 and the second member 155 are superimposed on each other, and the edge portion of the first member 135 and the edge portion of the second member 155 are joined to each other, so that a liquid chamber 156 is formed.

The second member 155 includes a recessed portion 155a that has a dome-like shape bulging toward the side opposite to the first member 135, and a flat portion 155b flatly formed in the outer circumferential portion of the recessed portion 155a. A surface of the recessed portion 155a on the side of the first member 135 is a concave spherical surface curved toward the side facing the first member 135 from the center toward the outer side. Further, the surface of the recessed portion 155a on the side of the mounting surface 22 is formed in so as to fit the mounting surface 22.

Further, the needle puncture portion 152 is formed in the central portion of the second member 155, wherein the needle puncture portion 152 is formed in a circular tube shape projecting toward the side opposite to the side of the first member 135. A tube hole 152a of the needle puncture portion 152 comes into communication with the inside of the container body 151 (i.e., the liquid chamber 156), and the inner surface of the needle puncture portion 152 is formed continuously from the inner surface of the second member 155. Further, the tip end of the needle puncture portion 152 is opened. The protruding length of the needle puncture portion 152 from the second member 155 is substantially equal to the depth of the insertion-hole 117 of the drug container mount 115.

Further, the inner diameter of the needle puncture portion 152 is set to a value smaller than the width of the finger of the user who presses the drug container 143, but large than the outer diameter of the second needle tip 9 of the needle tube 5 because the needle puncture portion 152 is a portion to be inserted by the second needle tip 9. Considering the deviation generated when mounting the drug container 143 on the injection needle assembly 102, it is preferred that the inner diameter of the needle puncture portion 152 is set in a range of 2 to 8 mm.
The seal member 153 is attached to the opened tip end of the needle puncture portion 152 in a liquid-tight manner.

The same material as that of the first member of the fourth embodiment may be used as the constituent material of the first member 135, and the same material as that of the needle hub 106 may be used as the constituent material of the second member 155. The container body 151 configured by the first member 135 and the second member 155 may be produced by the same method as that of the first embodiment.

In the present embodiment, the flexible first member 135 is joined to the flat portion 155b formed on the edge of the inflexible second member 155, and thereby the container body 151 is formed. Since the first member 135 is joined to the flat portion 155b of the second member 155, high joint strength can be achieved even if the material of the first member 135 is different from the material of the second member 155. Further, the edge portion of the first member 135 and the edge portion of the second member 155, which are joining faces, constitute a flange piece 159. Similar to the first embodiment, the flange piece 159 is a portion to be effectively used when mounting the drug container 143 on the injection needle assembly 102.

### [Method for using drug injection apparatus]

Next, a method for using the drug injection apparatus 141 of the present embodiment will be described below. FIG. 19 is a cross-sectional view showing a state where the drug container 143 is mounted on the injection needle assembly 102, and where the second needle tip 9 is penetrated through the seal member 153 of the drug container 143 so as to be inserted into the needle puncture portion 152. FIG. 20 is a cross-sectional view showing a state where the first needle tip 8 is stuck into a living body. FIG. 21 is a cross-sectional view showing a state where administration of the drug M performed by the drug injection apparatus 141 has been completed.

Before being used, the drug injection apparatus 141 is in a state where the injection needle assembly 102 and the drug container 143 are separated from each other (see FIG. 18). Thus, when using the drug injection apparatus 141, first, the drug container 143 is mounted on the mounting surface 22 of the drug container mount 115 so that the seal member 153 of the drug container 143 is abutted against the upper portion of the second needle tip 9 of the needle tube 5.

Further, the user presses the flange piece 159 of the drug container 143 from the side of the first member 135 with a predetermined force, and thereby the drug container 143 is moved toward the side of the mounting surface 22 so that the needle puncture portion 152 of the drug container 143 is inserted into the insertion-hole 117. Thus, as shown in FIG. 19, the second needle tip 9 is penetrated through the seal member 153 so as to be inserted into the needle puncture portion 152 of the drug container 143.
Thus, the drug container 143 is set to the injection needle assembly 102, and thereby the preparation of the drug injection apparatus 141 is completed.

At this time, since the depth of the insertion-hole 117 is substantially equal to the protruding length of the needle puncture portion 152, the tip end of the needle puncture portion 152 abuts the bottom of the insertion-hole 117, and the second member 155 of the drug container 143 also abuts the mounting surface 22. Further, since the protruding length of the second needle tip 9 within the insertion-hole 117 is set to a value smaller than the depth of the insertion-hole 117, the insertion length of the second needle tip 9 into the needle puncture portion 152 is smaller than the protruding length of the needle puncture portion 152, and therefore the second needle tip 9 will not project above the concave spherical surface 16a (see FIG. 18) of the mounting surface 22 (i.e., the second needle tip 9 will not project from inner surface of the second member 155). Further, at this time, since the flange piece 159 of the drug container 3 is locked by the locking pieces 23 formed on the peripheral edge of the mounting surface 22, the drug container 143 is positioned, so that the drug container 143 is prevented from being displaced in the surface direction of the mounting surface 22.
At this time, liquid passage of the drug M enclosed in the drug container 143 into the needle tube 5 is performed.

However, when the liquid passage of the drug M into the needle tube 5 is being performed, since the user presses the flange piece 159 of the drug container 143 to move the drug container 143 toward the side of the mounting surface 22, the liquid chamber 156 is not pressed. Thus, the drug M of the liquid chamber 156 can be prevented from being discharged from the first needle tip 8. Further, in the present embodiment, since the second member 155 of the drug container 143 is formed of an inflexible material, the drug container 143 can be simply moved toward the side of the mounting surface 22 by pressing the flange piece 159.

Next, the end face 13a of the stabilizer 13 is caused to face the skin while maintaining the state where the second needle tip 9 is inserted into the drug container 143 from the side of the seal member 153, and thereby the first needle tip 8 of the needle tube 5 is caused to face the skin to be stuck. Next, the user grips a portion other than the drug container 143 (for example, the side portion of the needle-holding portion 11) to press the drug injection apparatus 141 in the direction in which the first needle tip 8 is stuck into the living body. The injection needle assembly 102 is caused to substantially perpendicularly move toward the side of the skin, so that the first needle tip 8 is stuck into the skin, and the stabilizer is pressed until the contact surface 14a of the guide portion 14 contacts the skin.
Thus, as shown in FIG. 20, the first needle tip 8 is stuck into the living body.

In the present embodiment, liquid passage has been performed before the first needle tip 8 is stuck into the living body, but the user does not press the drug container 143 when the first needle tip 8 is stuck into the living body. In other words, the user grips a portion other than the drug container 143 to press the drug injection apparatus 141, and thereby the drug M can be prevented from being discharged first from the first needle tip 8.

Next, the user presses the flexible first member 135 toward the side of the second member 155. Thus, the inner pressure of the container body 151 increases, and thereby the drug M enclosed in the container body 151 is discharged into the living body from the first needle tip 8 through the second needle tip 9 of the needle tube 5. Further, as shown in FIG. 21, the first member 135 is pressed until the first member 135 is brought into close contact with the second member 155, and thereby the drug M in the container body 151 is completely discharged, and the administration of the drug M is completed.

In the drug injection apparatus 141 of the present embodiment, since the second member 155 of the drug container 143 mounted on the drug container mount 115 is formed of an inflexible material in a curved shape, the second member 155 is unstable on the mounting surface 22 before the second needle tip 9 penetrates through the seal member 153. Thus, liquid passage has been completed before the first needle tip 8 is stuck into the living body, and thereafter, administration of the drug M can be stably performed by sticking the first needle tip 8 into the living body.
Further, the same advantages as those of the first embodiment can be achieved.

In the present embodiment, although the mounting surface 22 of the drug container mount 115 in the injection needle assembly 102 is formed in a curved shape, the mounting surface may also be formed as a flat surface. Incidentally, in the present embodiment, since the second member 155 of the container body 151 is formed in a curved shape, when the user presses the first member 135 toward the side of the second member 155, the finger of the user will closely abut the curved surface through the flexible first member 135, and therefore the drug M filled in the container body 151 can be sufficiently discharged, so that residual liquid can be reduced. Further, since the mounting surface 22 of the drug container mount 115 is formed in a curved shape, when mounting the drug container 143 on the drug container mount 115, the drug container 143 can be more stably mounted, and therefore safety in use can be ensured.

Incidentally, in the present embodiment, in the needle hub 106, the second needle tip 9 of the needle tube 5 is held by the needle-holding portion 11 so that the second needle tip 9 does not project above the mounting surface 22 of the drug container mount 115; however, the second needle tip 9 may also project above the mounting surface 22. In the case where the second member 155, which constitutes the container body 151 of the drug container 143, is formed of an inflexible material and has a certain thickness, like the case of the present embodiment, the length of the tube hole 152a of the needle puncture portion is larger than the depth of the insertion-hole 117. In such a case, even if the second needle tip 9 projects above the mounting surface 22, the insertion length to the needle puncture portion 152 can be smaller than the length of the tube hole 152a, and therefore the identical advantages of the present embodiment can be achieved.

It is to be understood that the drug injection apparatus of present invention is not limited to the embodiments described above, and various modifications can be made without departing from the spirit and scope of the present invention.

The fourth and fifth embodiments are described based on an example in which a flexible member whose deformation is not particularly limited is used as the first member 135 of the drug containers 103, 143. However, a flexible member whose deformation is limited, such as a member that will yield so as to be reversed (i.e., be buckled) when subjected to a pressure equal to or higher than a certain level, may be used as the first member 135 of the drug containers 103, 143. In the case where a member that yields so as to be reversed is used, the pressing force necessary for causing such a member to be buckled is set to a value lager than both the pressing force necessary for performing puncture and the pressing force necessary for the second needle tip to penetrate through the seal member. Further, after such member has been reversed, even if the pressing force is reduced, a predetermined pressure will be applied to the drug in the drug container until the deformation is completed, and therefore the injection of the drug can be continued in a stable manner. Such a member may be formed of a synthetic resin, a metal or the like.

### 6. Sixth embodiment

### [Drug injection apparatus]

Next, a drug injection apparatus according to a sixth embodiment of the present invention will be described below.
FIG. 22 is a cross-sectional view showing the configuration of a drug injection apparatus 171 of the sixth embodiment, and showing a state of a drug container 173 and an injection needle assembly 172 before using the drug injection apparatus 171.

As shown in FIG. 22, the drug injection apparatus 171 includes a drug container 173 having a drug M enclosed therein, and an injection needle assembly 172 having a needle tube 5 incorporated therein; and the administration of the drug M can be performed by pressing the drug container 173 to cause it to deform.

In the present embodiment, the drug injection apparatus 171 is described based on an example in which the drug injection apparatus is used to stick a needle tip into a skin from the surface of the skin to inject a drug into an upper layer of the skin.
In the following paragraphs, the configuration of the injection needle assembly 172 and the drug container 173, as well as a method for using the drug injection apparatus 171, will be described.

### [Injection needle assembly]

The injection needle assembly 172 includes a needle tube 5 having a hollow needle hole, and a needle hub 176 by which the needle tube 5 is fixed.

Similar to the first to fifth embodiments, the needle tube 5 is a double-ended needle. In other words, the needle tube 5 has a first needle tip 8 for being stuck into a living body, and a second needle tip 9 for penetrating through a seal member 192 (which is to be described later) of the drug container 173. Since the configuration of the needle tube 5 has been described above when discussing the first embodiment, the description will not be repeated herein.

Next, the needle hub 176 will be described below.
The needle hub 176 includes a needle-holding portion 11 for holding the central portion of the needle tube 5, a drug container mount 15, a lid receiver 181, a lid 182, an adjusting portion 12, a stabilizer 13, and a guide portion 14, wherein the drug container mount 15, the lid receiver 181 and the lid 182 are provided on one end of the needle-holding portion 11, and the adjusting portion 12, the stabilizer 13 and the guide portion 14 are provided on the other end of the needle-holding portion 11.

Examples of the material of the needle hub 176 include various resins identical to those of the needle hub 6 of the first embodiment.
Further, since the details of needle-holding portion 11, the adjusting portion 12, the stabilizer 13, the guide portion 14 and the drug container mount 15, which configure the needle hub 176, have been described above when discussing the first embodiment, the description will not be repeated herein.

The lid receiver 181 includes a bottom 181a and a side peripheral portion 181b, wherein the bottom 181a is formed in ring shape projecting outward from the side surface of the needle-holding portion 11 in the radial direction, and the side peripheral portion 181b is formed in circular tube shape erected at the circumferential edge of the bottom 181a on the side of the drug container mount 15. The lid receiver 181 is formed on the drug container mount 15 side of the injection hole 19 of the needle-holding portion 11, and the diameter of the bottom 181a is larger than the diameter of the drug container mount 15. Further, the upper portion of the side peripheral portion 181b projects more toward the side on which the drug container 173 is mounted than the mounting surface 22 of the drug container mount 15. Further, a locked portion 184 for locking the lid 182 is formed in the upper portion of the side peripheral portion 181b. The locked portion 184 is formed at a position facing a hinge 185 (which is to be described later) with the drug container mount 15 interposed therebetween.

The lid 182 is formed in a circular plate shape whose outer diameter is equal to or smaller than the inner diameter of the side peripheral portion 181b of the lid receiver 181, and is rotatably attached to the lid receiver 181 by the hinge 185. The lid 182 includes an abutting surface 187 and a pressing surface 188, where the abutting surface 187 is a surface facing the mounting surface 22 of the drug container mount 15 and is also a surface abutting the drug container 173 when using the drug injection apparatus 171, and the pressing surface 188 is a surface opposite to the abutting surface 187 and is also a surface to be pressed by the user when using the drug injection apparatus 171.

The abutting surface 187 is formed so as to curve toward the side opposite to the side facing the mounting surface 22 from the central portion toward the outer side, and includes a convex spherical surface 187a and a flat surface 187b flatly formed in the outer circumferential portion of the convex spherical surface 187a, wherein the diameter of the convex spherical surface 187a is identical to the diameter of the concave spherical surface 16a of the mounting surface 22. Further, the curvature of the convex spherical surface 187a of the abutting surface 187 of the lid 182 is substantially equal to the curvature of the concave spherical surface 16a of the mounting surface 22 of the drug container mount 15. Further, the pressing surface 188 of the lid 182 is substantially flat.

Further, a recessed contact avoiding portion 189 formed continuously from the abutting surface 187 is formed in the central portion of the abutting surface 187 of the lid 182. The contact avoiding portion 189 is a portion adapted to avoid contact between the second needle tip 9 of the needle tube 5 and the abutting surface 187 of the lid 182 when using the drug injection apparatus 171. Further, the end portion of the lid 182 serves as a locking portion 182a to be locked by the locked portion 184 of the lid receiver 181 when the lid 182 is rotated toward the side of the lid receiver 181.

With such an arrangement, the lid 182 can be rotated with respect to the lid receiver 181 and drug container mount 15 so as to be closed. Further, the lid 182 is rotated toward the side of the lid receiver 181, so that the locking portion 182a formed on the end portion of the lid 182 is locked by the locked portion 184 of the lid receiver 181, and thereby the rotation of the lid 182 is stopped. Further, when the lid 182 is closed with respect to the lid receiver 181, the convex spherical surface 187a of the abutting surface 187 of the lid 182 fits the concave spherical surface 16a of the mounting surface 22 of the drug container mount 15. Further, when the lid 182 is closed with respect to the lid receiver 181, the second needle tip 9 projects above the mounting surface 22 of the drug container mount 15 is housed in the contact avoiding portion 189.

Further, the surface of the bottom 181a of the lid receiver 181 on the side of the needle-holding portion 11 extends outward from the needle-holding portion 11 in the radial direction. The surface of the lid receiver 181 on the side of the needle-holding portion 11, the flat surface 21b of the flange 21, and the outer circumferential surface of the needle-holding portion 11 form a gripping portion 180. The gripping portion 180 is formed in a bobbin-like shape and has a circumferentially-recessed portion. When using the drug injection apparatus 171, the user can grip the drug injection apparatus 171 by, for example, putting his (or her) index finger and middle finger on the recessed portion of the gripping portion 180 so that the needle-holding portion 11 is sandwiched by the both fingers. Since the gripping portion 180 is recessed, the fingers of the user are stabilized, so that the drug injection apparatus 171 can be easily gripped by the user.

### [Drug container]

The drug container 173 includes a container body 191 having the drug M enclosed therein, and a seal member 192 through which the second needle tip 9 of the needle tube 5 is to be punctured.

The container body 191 includes a sheet-like first member 195 and a sheet-like second member 196, wherein the first member 195 is the side to be pressed, and the second member 196 is the side to which the seal member 192 is attached.
The first member 195 and the second member 196 are each formed of a transparent flexible material, and examples of the transparent flexible material include soft resin materials such as, for example, polypropylene (PP), polyethylene (PE), ethylene-vinyl acetate copolymer (EVA) and the like. The first member 195 and the second member 196 may also each be formed of a material obtained by blending styrene thermoplastic elastomer, olefinic thermoplastic elastomer and/or the like into polyethylene or polypropylene, and then softening the blend material.

Further, the container body 191 is a bag-like member obtained by attaching the edge portion of the first member 195 and the edge portion of the second member 196 to each other. The internal space of the beg-like container body 191 becomes a liquid chamber 198 filled with the drug M. In the present embodiment, the container body 191 is formed in a bag-like shape by joining the edge portion of the sheet-like first member 195 and the edge portion of the sheet-like second member 196 in a liquid-tight manner by means of, for example, ultrasonic welding.

By joining the first member 195 and the second member 196 to each other, the edge portion of the first member 195 and the edge portion of the second member 196, which are joining faces, configure a flange piece 199. The flange piece 199 is a portion to be effectively used when mounting the drug container 173 on the injection needle assembly 172.
In the present embodiment, a quadrangular sheet is used as each of the first member 195 and the second member 196, and the first member 195 and the second member 196 are joined to each other so that the outline of the liquid chamber 198 is substantially a circle when viewed in the direction perpendicular to the joining face. Thus, the outline of the flange piece 199 is a quadrangle.

Further, a needle housing portion 195a is formed in the central portion of the first member 195, wherein the needle housing portion 195a projects toward the outside of the container body 191. The inner surface of the needle housing portion 195a is formed continuously from the inner surface of the container body 191. In other words, by forming the first member 195 in the first member 195, the liquid chamber 198 formed between the first member 195 and the second member 196 also becomes a shape projecting toward the side of the needle housing portion 195a. When the first member 195 is pressed toward the side of the second member 196, the second needle tip 9 inserted into the side of the seal member 192 of the second member 196 is housed in the needle housing portion 195a. Further, the needle housing portion 195a is formed in a shape so that when the lid 182 is closed with respect to the lid receiver 181, the needle housing portion 195a can be housed in the contact avoiding portion 189 formed in the lid 182.

The container body 191 can be produced by injection molding, extrusion molding, calender molding, blow molding, inflation molding or the like.

The tensile elasticity and the thickness of the flexible material applied to both the first member 195 and the second member 196 may be set in the same manner as that of the contact portion 35 and the pressing portion 36 of the drug container 3 of the first embodiment. Further, the inner surface of the container body 191 may be coated with a film, such as a low-density polyethylene laminated film.

It is preferred that the container body 191 formed in the aforesaid manner has water vapor barrier property. Since the container body 191 has water vapor barrier property, not only water inside the container body 191 can be prevented from being evaporated, but also water vapor outside the container body 191 can be prevented from entering the container body 191.
The level of the water vapor barrier property of the container body 191 may be set in the same manner as that of the container body 31 of the drug container 3 of the first embodiment.

The seal member 192 is attached to the central portion of the outer surface of the second member 196, and is formed at a position facing the needle housing portion 195a with the joining face between the first member 195 and the second member 196 as the symmetry plane. Similar to the seal members 32, 133 of the first to fifth embodiments, in order to achieve good liquid tightness between the seal member 192 and the needle tube 5, it is preferred that the seal member 192 is formed of a material having rubber elasticity.

In the present embodiment, when using the drug injection apparatus 171, the second needle tip 9 of the needle tube 5 held by the injection needle assembly 172 is penetrated through both the seal member 192 and the second member 196 so as to be inserted into the container body 191. The seal member 192 has an effect of preventing the drug M enclosed in the container body 191 from being leaked from the gap between the second needle tip 9 and the container body 191. Further, the seal member 192 is formed of a material so as to have such a penetration resistance that, if not being pressed by a pressure of a certain level, the seal member 192 can not be penetrated by the second needle tip 9; and the penetration resistance of the seal member to the second needle tip 9 is larger than the resistance of the living body to the first needle tip 8.

The drug container 173 may be produced by forming the container body 191 and then filling the drug M, or by forming the container body 191 in a stated in which the drug M has been filled. In the case when the drug container 173 is produced by forming the container body 191 in a stated in which the drug M has been filled, the container body 191 can be produced by blow fill molding method in which molding (blow), filling and sealing are performed in sequence.

### [Method for using drug injection apparatus]

Next, a method for using the drug injection apparatus 171 of the present embodiment will be described below. FIG. 23 is a cross-sectional view showing a state where the drug container 173 is mounted on the injection needle assembly 172, and the first needle tip 8 is stuck into a living body. FIG. 24 is a cross-sectional view showing a state where the second needle tip 9 is penetrated through the seal member 192 of the drug container 173 so as to be inserted into the needle puncture portion 191. FIG. 25 is a cross-sectional view showing a state where administration of the drug M performed by the drug injection apparatus 171 has been completed.

Before being used, the drug injection apparatus 171 is in a state where the injection needle assembly 172 and the drug container 173 are separated from each other (see FIG. 22). Thus, when using the drug injection apparatus 171, first, the drug container 173 is mounted on the mounting surface 22 of the drug container mount 15 so that the seal member 192 of the drug container 173 is abutted against the second needle tip 9. Since the seal member 192 has a certain level of resistance to the force for the second needle tip 9 to try to penetrate through the seal member 192, the seal member 192 will not be penetrated by the second needle tip 9 at the stage where the drug container 173 has been mounted on the mounting surface 22. Further, at this time, since the flange piece 199 of the drug container 173 is locked by the locking pieces 23 formed on the edge of the mounting surface 22, the drug container 173 is positioned, so that the drug container 173 is prevented from being displaced in the surface direction of the mounting surface 22.
By performing the above operation, the drug container 173 is set to the injection needle assembly 172, and thereby the preparation of the drug injection apparatus 171 is completed.

Next, the lid 182 is rotated toward the side of the lid receiver 181, so that the abutting surface 187 of the lid 182 is abutted against the first member 195 of the drug container 173. Next, the user grips the drug injection apparatus 171 to cause the end face 13a of the stabilizer 13 to face the skin and cause the first needle tip 8 of the needle tube 5 to come close to the skin to be stuck.

Next, as shown in FIG. 23, the pressing surface 188 of the lid 182 is pressed to further rotate the lid 182 toward the side of the lid receiver 181, and thereby the injection needle assembly 172 is substantially perpendicularly moved toward the side of the skin, so that the first needle tip 8 is stuck into the skin and the end face 13a of the stabilizer 13 is pressed against the skin.

At this time, since the seal member 192 is formed of a material so that the penetration resistance of the seal member 192 to the second needle tip 9 is larger than the puncture resistance of the living body to the first needle tip 8, the first needle tip 8 is stuck into the living body before the second needle tip 9 is penetrated into the seal member 192.

Here, the needle-protruding surface 12a of the adjusting portion 12 is coplanar with the end face 13a of the stabilizer 13. Thus, the needle-protruding surface 12a of the adjusting portion 12 can contact the skin so as to cause the skin to deform flatly, and the first needle tip 8 of the needle tube 5 can be inserted into the skin by the protruding length L only.

Next, as shown in FIG. 24, the user presses the pressing surface 188 of the lid 182 to further rotate the lid 182 toward the side of the lid receiver 181, and thereby the drug injection apparatus 171 is pressed toward the side of the living body, so that the contact surface 14a of the guide portion 14 is brought into contact with the skin. Here, the value of the height of the guide portion Y is set so that the skin can be stuck by a suitable pressing force applied from the needle tube 5 and the stabilizer 13, so as to inject the drug into the skin. Thus, the force for the stabilizer 13 to press against the skin becomes a predetermined value. As a result, it is possible to guide the pressing force of the stabilizer 13 for the user as well as to press the stabilizer 13 against the skin with a proper pressing force, and therefore the first needle tip 8 and the contact surface 14a of the needle tube 5 can be reliably positioned in the upper layer of the skin.

As shown in FIG. 24, when the lid 182 is pressed so as to rotate until the contact surface 14a of the guide portion 14 contacts the skin, the force for the convex spherical surface 187a of the lid 182 to press the drug container 173 is larger than the penetration resistance of the seal member 192 to the second needle tip 9. Thus, the seal member 192 is penetrated by the second needle tip 9, so that the drug container 173 is moved toward the side of the mounting surface 22 of the injection needle assembly 172. Thus, the drug container 173 is pressed against the mounting surface 22, and the second needle tip 9 is penetrated through both the seal member 192 and the second member 196 so as to be inserted into the container body 191.
Thus, liquid passage of the drug M enclosed in the drug container 173 into the needle tube 5 is performed.

Next, the lid 182 is pressed so that the lid 182 is further rotated toward the side of the lid receiver 181. Thus, the convex spherical surface 187a of the lid 182 presses the first member 195 of the container body 191 toward the side of the second member 196, so that the inner pressure of the container body 191 increases, and therefore the drug M enclosed into the container body 191 is discharged from the first needle tip 8 into the living body through the second needle tip 9 of the needle tube 5.

Further, as shown in 25, the convex spherical surface 187a of the lid 182 fits the concave spherical surface 16a of the mounting surface 22 when the lid 182 is closed with respect to the lid receiver 181, and the locking portion 182a of the lid 182 is locked by the locked portion 184 of the lid receiver 181. Thus, since the first member 195 of the container body 191 is brought into close contact with the second member 196, the drug M in the container body 191 is completely discharged, and the administration of the drug M is completed.

Incidentally, when the lid 182 has been completely closed to the lid receiver 181, the second needle tip 9 projecting above the mounting surface 22 of the drug container mount 15 will be housed in the needle housing portion 195a of the first member 195, and the second needle tip 9 and the needle housing portion 195a will be housed in the contact avoiding portion 189 of the lid 182.

In the drug injection apparatus 171 of the present embodiment, the direction in which the drug injection apparatus 171 is pressed to stick the first needle tip 8 into the living body is identical to the direction in which the drug container 173 is pressed to discharge the drug M from the first needle tip 8. Further, it is possible to press the drug container 173 by the convex spherical surface 187a of the lid 182 while pressing the drug injection apparatus 171 against the living body by the force for pressing the pressing surface 188 of the lid 182 to rotate the lid 182. Thus, it is possible to securely stick the first needle tip 8 into the living body, and discharge the drug M while maintaining stable pressing force. Thus, the user can administer the drug in a stable manner.

In the above description, although the method for using the drug injection apparatus 171 is described on a step-by-step basis, the entire operation from the step of sticking the first needle tip 8 into the living body to the step of discharging the drug M can be successively performed by pressing the drug container 173 in a direction from the first member 195 to the second member 196. Thus, time for performing a sequence of operations can be reduced.

Further, in the drug injection apparatus 171 of the present embodiment, since the drug M filled in the flexible container body 191 can be administered by rotating the lid 182, the user does not directly touch the drug container 173. Thus, when using the drug injection apparatus 171, accidental sticking to the user can be prevented even in the case where the flexible container body 191 has been penetrated by the needle tube 5. Further, after the lid 182 has been completely closed to the lid receiver 181, the lid 182 is locked by the lid receiver 181 so as to become impossible to be opened again. Thus, the injection needle assembly 172 can be prevented from being reused.

Further, in the drug injection apparatus 171 of the present embodiment, the needle housing portion 195a is formed in the first member 195 of the container body 191, so that when the lid 182 is completely closed to the side of the lid receiver 181, the second needle tip 9 is housed in the needle housing portion 195a, and the needle housing portion 195a is housed in the contact avoiding portion 189 of the lid 182. Thus, the first member 195 will not be penetrated by the second needle tip 9 even when the lid 182 is completely closed. Thus, the discharge of the drug M in the drug container 173 from the first needle tip 8 will not be inhibited.

Further, in the present embodiment, the mounting surface 22 is formed in a curved shape, and the lid 182 facing the mounting surface 22 is formed in a curved shape so as to fit the mounting surface 22. Thus, when the lid 182 is rotated so as to be closed with respect to the lid receiver 181, the first member 195 of the container body 191 is brought into close contact with the second member 196. Thus, the drug M filled in the container body 191 can be sufficiently discharged, and therefore residual liquid can be reduced.

In the present embodiment, the drug M is filled in the drug container 173 formed of a flexible material, and the drug M can be administered by pressing the drug container 173 with a finger. Thus, the configuration of the drug injection apparatus 171 of the present embodiment is simpler than that of a syringe, such as a conventional prefilled syringe, in which a drug is filled in an inflexible drug container, and the drug in the inflexible drug container is discharged by using a pusher and a gasket. Thus, with the present embodiment, cost can be reduced compared to the conventional prefilled syringe, and the present embodiment is particularly effective to be applied to the case where the drug injection apparatus is filled with a small amount of drug.

### 7. Seventh embodiment

### [Drug injection apparatus]

Next, a drug container according to a seventh embodiment of the present invention, and a drug injection apparatus which uses the drug container will be described below.
FIG. 26 is a cross-sectional view showing the configuration of a drug injection apparatus 201 of the seventh embodiment, wherein a drug container 173 and an injection needle assembly 202 are separated from each other.

The drug injection apparatus 201 of the present embodiment differs from the drug injection apparatus 171 of the sixth embodiment in that the configuration of a lid 212, and a lid receiver 211 of a needle hub 206, which constitutes the injection needle assembly 202, of the drug injection apparatus 201 are different from the configuration of the lid 182 and the lid receiver 181 of the drug injection apparatus 171.
Note that, in the following paragraphs, description will only be given to the portions whose configuration and function are different from those of the drug injection apparatus 171 of the sixth embodiment, and description of other portions will be omitted.

### [Injection needle assembly]

In the present embodiment, the lid receiver 211, which constitutes the needle hub 206, has substantially the same configuration as that of the lid receiver 181 (see FIG. 22) of the drug injection apparatus 171. The lid receiver 211 differs from the lid receiver 181 in that, in the lid receiver 211, a locked portion 214 for locking the lid 212 has a plurality of locked claws 214a formed from the upper end of a side peripheral portion 211b in the depth direction.

Further, the lid 212 has substantially the same configuration as that of the lid 182 of the drug injection apparatus 171. The lid 212 differs from the lid 182 in that a locking portion 219 extending in the rotation direction perpendicular to a flat surface 217b is formed in the end portion of the lid 212 corresponding to the locked portion 214 when the lid 212 is closed to the side of the lid receiver 211, wherein the locking portion 219 has a plurality of locking claws 219a arranged in the rotation direction.

With such an arrangement, when closing the lid 212 with respect to the lid receiver 211, the plurality of locking claws 219a of the lid 212 overleap a plurality of locked claws 214a of the lid receiver 211 along with the rotation of the lid 212, so that the locking portion 219 is locked step-by-step by the locked portion 214, and the rotation of the lid 212 is stopped when the lid 212 is completely closed to the side of the lid receiver 211.

### [Method for using drug injection apparatus]

Next, a method for using the drug injection apparatus 201 of the present embodiment will be described below. Note that, in the method for using the drug injection apparatus 201, the description of the part identical to that of the drug injection apparatus 171 of the sixth embodiment will not be omitted.
FIG. 27 is a cross-sectional view showing a state where administration of the drug M performed by the drug injection apparatus 201 has been completed.

The drug injection apparatus 201 can be used in the same manner as the drug injection apparatus 171. In the present embodiment, when the lid 212 is rotated to discharge the drug, the plurality of locking claws 219a of the locking portion 219 of the lid 212 are locked step-by-step with respect to the plurality of locked claws 214a of the locked portion 214 of the lid receiver 211, so that the user can feel and/or hear the click caused when the locking claws 219a overleap the locked claws 214a, and therefore the user can know the state of the administration of the drug (for example, the completion of the administration) without relying on visual confirmation.
Further, the same advantages as those of the sixth embodiment can be achieved.

### 8. Eighth embodiment

### [Drug injection apparatus]

Next, a drug container according to an eighth embodiment of the present invention, and a drug injection apparatus which uses the drug container will be described below.
FIG. 28 is a cross-sectional view showing the configuration of a drug injection apparatus 241 of the seventh embodiment, wherein a drug container 173 and an injection needle assembly 242 are separated from each other.

The present embodiment differs from the sixth embodiment in that the configuration of a lid 252 of a needle hub 246, which constitutes the injection needle assembly 242, is different from the configuration of the lid 182 of the sixth embodiment. In FIG. 28, components corresponding to those of FIG. 22 are denoted by the same reference numerals, and the explanation thereof will not be repeated.

### [Injection needle assembly]

In the present embodiment, the lid 252, which constitutes the needle hub 246, includes a convex portion 252a having a dome-like shape bulging toward the side opposite to the drug container mount 15, and a flat portion 252b formed in the outer circumferential portion of the convex portion 252a. The pressing-side housing body 252 is flexible, and is formed so that the convex portion 252a thereof will yield so as to be reversed (i.e., be buckled) when subjected to a pressure equal to or higher than a certain level. When using the drug injection apparatus 241, the convex portion 252a of the lid 252 is convex toward the side opposite to the drug container mount 15; however, when the lid 252 is pressed so as to be buckled, the lid 252 will be reversed so as to become convex toward the side of the drug container mount 15. The shape of the lid 252 is formed so that when the lid 252 becomes convex toward the side of the drug container mount 15, the convex surface of the lid 252 will fit the concave spherical surface 16a of the mounting surface 22.

Further, in the present embodiment, a contact avoiding portion 259 is formed at the center of the lid 252, wherein the contact avoiding portion 259 is a recessed portion formed continuously from a surface of the lid 252 on the side of the mounting surface 22. The strength of the contact avoiding portion 259 is such that when to the lid 252 is buckled, the contact avoiding portion 259 will not be buckled. Thus, when the lid 252 is buckled, the needle housing portion 195a, in which the second needle tip 9 is housed, is housed in the contact avoiding portion 259. Thus, the second needle tip 9 will not be prevented from being penetrated through the first member 195 to administer the drug.

In the present embodiment, the surface of the lid 252 on the side opposite to the mounting surface 22 serves as a pressing surface 258. Further, when the lid 252 is closed with respect to the lid receiver 181, the end portion of the lid 252 corresponding to the locked portion 184 of the lid receiver 181 serves as a locking portion 252c locked by the locked portion 184.

The lid 252 is formed of a polymer material that it will yield so as to be reversed when subjected to a pressure equal to or higher than a certain level. Examples of the material of the lid 252 include metals, such as iron, stainless steel and the like, and synthetic resins, such as polypropylene (PP), polyethylene (PE), polystyrene (PS), ABS resin and the like.

### [Method for using drug injection apparatus]

Next, a method for using the drug injection apparatus 241 of the present embodiment will be described below. FIG. 29 is a cross-sectional view showing a state where the drug container 173 is mounted on the injection needle assembly 202. FIG. 30 is a cross-sectional view showing a state where the first needle tip 8 is stuck into a living body. FIG. 31 is a cross-sectional view showing a state where administration of the drug M performed by the drug injection apparatus 241 has been completed.

Before being used, the drug injection apparatus 241 is in a state where the injection needle assembly 242 and the drug container 173 are separated from each other (see FIG. 28). Thus, when using the drug injection apparatus 241, first, the drug container 173 is mounted on the mounting surface 22 of the drug container mount 15 so that the seal member 192 of the drug container 173 is abutted against the second needle tip 9. Since the seal member 192 has a certain level of resistance to the force for the second needle tip 9 to try to penetrate through the seal member 192, the seal member 192 will not be penetrated by the second needle tip 9 at the stage where the drug container 173 has been mounted on the mounting surface 22. Further, at this time, since the flange piece 199 of the drug container 173 is locked by the locking pieces 23 formed on the edge of the mounting surface 22, the drug container 173 is positioned, so that the drug container 173 is prevented from being displaced in the surface direction of the mounting surface 22.

Next, as shown in FIG. 29, the user rotates the lid 252 of the injection needle assembly 242 toward the side of the lid receiver 181 to completely close the lid 252 with respect to the lid receiver 181, so that the locking portion 252c of the lid 252 is locked by the locked portion 184 of the lid receiver 181. At this time, in the convex portion 252a of the lid 252, since the surface facing the mounting surface 22 is a recessed surface, the drug container 173 will not be pressed by the lid 252.
By performing the above operation, the drug container 173 is set to the injection needle assembly 242, and thereby the preparation of the drug injection apparatus 241 is completed.

Next, the user grips the needle hub 246 of the drug injection apparatus 241, whose lid 252 has been closed, to cause the end face 13a of the stabilizer 13 to face the skin and cause the first needle tip 8 of the needle tube 5 to face the skin to be stuck. Next, as shown in FIG. 30, the drug injection apparatus 241 is substantially perpendicularly moved toward the side of the skin, so that the first needle tip 8 is stuck into the skin and the end face 13a of the stabilizer 13 is pressed against the skin.

Next, as shown in FIG. 31, after the stabilizer 13 is pressed until the contact surface 14a of the guide portion 14 contacts the skin, the pressing surface 258 of the lid 252 is pressed toward the side of the lid receiver 181 by a predetermined force, and thereby the lid 252 is buckled and reversed, so that the surface of the lid 252 facing the drug container 173 becomes a convex surface. Thus, the second needle tip 9 is penetrated through the seal member 192, and the container body 191 is compressed, so that the inner pressure of the container body 191 increases, and therefore the drug M filled in the container body 191 is discharged to the living body through the needle tube 5, thereby the administration of the drug M is performed.

After the drug M has been discharged, the second needle tip 9 exposed from the mounting surface 22 of the drug container mount 15 is housed in the needle housing portion 195a of the first member 195 of the container body 191, and the needle housing portion 195a is housed in the contact avoiding portion 259 of the lid 252. Thus, the second needle tip 9 will not be penetrated through the first member 195, and the drug will not be prevented from being discharged.

In the present embodiment, since the drug container 173 is caused to deform by causing the lid 252 to be buckled, the period from the liquid passage into the needle tube 5 to discharging the drug M can be reduced. Thus, time for administering the drug M can be reduced, and therefore the burden of the user can be reduced. Further, after the lid 252 is buckled and reversed, even if the pressing force is reduced, a predetermined pressure will be applied to the drug until the deformation is completed, and therefore the injection of the drug can be continued in a stable manner.
Further, the same advantages as those of the sixth embodiment can be achieved.

### 9. Ninth embodiment

### [Drug injection apparatus]

Next, a drug container according to a ninth embodiment of the present invention, and a drug injection apparatus which uses the drug container will be described below.
FIG. 32 is a cross-sectional view showing the configuration of a drug injection apparatus 271 of the ninth embodiment, wherein a drug container 103 and an injection needle assembly 272 are separated from each other.

The present embodiment is an example in which a lid receiver 181 and a lid 282 are provided to the injection needle assembly 102 (see FIG. 13) of the fourth embodiment. In FIG. 32, components corresponding to those of FIG. 13 are denoted by the same reference numerals, and the explanation thereof will not be repeated.

### [Injection needle assembly]

The injection needle assembly 272 includes a needle tube 5 having a hollow needle hole, and a needle hub 276 by which the needle tube 5 is fixed. The needle hub 276 includes a needle-holding portion 11, an adjusting portion 12, a stabilizer 13, a guide portion 14, a drug container mount 15, a lid receiver 181, and a lid 282.
Since the lid receiver 181 of the needle hub 276 is identical to that of the sixth embodiment (see FIG. 22), the description thereof will be omitted.

The lid 282 of the needle hub 276 is formed in a circular plate shape whose outer diameter is equal to or smaller than the inner diameter of the side peripheral portion 181b of the lid receiver 181, and is rotatably attached to the lid receiver 181 by the hinge 185. The lid 282 includes an abutting surface 287 and a pressing surface 288, where the abutting surface 287 is a surface facing the mounting surface 22 of the drug container mount 115 and is also a surface abutting the drug container 103 when using the drug injection apparatus 271, and the pressing surface 288 is a surface opposite to the abutting surface 287 and is also a surface to be pressed by the user when using the drug injection apparatus 271.

The abutting surface 287 is formed so as to curve toward the side opposite to the side facing the mounting surface 22 from the center toward the outer side, and includes a convex spherical surface 287a and a flat surface 287b flatly formed in the outer circumferential portion of the convex spherical surface 187a, wherein the diameter of the convex spherical surface 287a is identical to the diameter of the concave spherical surface 16a of the mounting surface 22. Further, the curvature of the convex spherical surface 287a of the abutting surface 287 of the lid 282 is substantially equal to the curvature of the concave spherical surface 16a of the mounting surface 22 of the drug container mount 115.

### [Method for using drug injection apparatus]

Next, a method for using the drug injection apparatus of the present embodiment will be described below.
FIG. 33 is a cross-sectional view showing a state where the drug container 103 is mounted on the injection needle assembly 272, and the first needle tip 8 is stuck into a living body. FIG. 34 is a cross-sectional view showing a state where the second needle tip 9 is penetrated through the seal member 133 of the drug container 103 so as to be inserted into the needle puncture portion 132. FIG. 35 is a cross-sectional view showing a state where administration of the drug M performed by the drug injection apparatus 271 has been completed.

Before being used, the drug injection apparatus 271 is in a state where the injection needle assembly 272 and the drug container 103 are separated from each other (see FIG. 32). Thus, when using the drug injection apparatus 271, first, the drug container 103 is mounted on the mounting surface 22 of the drug container mount 115 so that the seal member 133 of the drug container 103 is abutted against the second needle tip 9. Since the seal member 133 has a certain level of resistance to the force for the second needle tip 9 to try to penetrate through the seal member 133, the seal member 133 will not be penetrated by the second needle tip 9 at the stage where the drug container 103 has been mounted on the mounting surface 22. Further, at this time, since the flange piece 139 of the drug container 103 is locked by the locking pieces 23 formed on the edge of the mounting surface 22, the drug container 103 is positioned, so that the drug container 103 is prevented from being displaced in the surface direction of the mounting surface 22.
By performing the above operation, the drug container 103 is set to the injection needle assembly 272, and thereby the preparation of the drug injection apparatus 271 is completed.

Next, the lid 282 is rotated toward the side of the lid receiver 181, so that the abutting surface 287 of the lid 282 is abutted against the first member 135 of the drug container 103. Next, the user grips the drug injection apparatus 271 to cause the end face 13a of the stabilizer 13 to face the skin and cause the first needle tip 8 of the needle tube 5 to come close to the skin to be stuck.

Next, as shown in FIG. 33, the pressing surface 288 of the lid 282 is pressed to further rotate the lid 282 toward the side of the lid receiver 181, and thereby the injection needle assembly 272 is substantially perpendicularly moved toward the side of the skin, so that the first needle tip 8 is stuck into the skin and the end face 13a of the stabilizer 13 is pressed against the skin.

At this time, since the seal member 133 is formed of a material so that the resistance of the seal member 133 to the second needle tip 9 is larger than the resistance of the living body to the first needle tip 8, the first needle tip 8 is stuck into the living body before the second needle tip 9 penetrates through the seal member 133.

Next, as shown in FIG. 34, the user presses the pressing surface 288 of the lid 282 to further rotate the lid 282 toward the side of the lid receiver 181, and thereby the drug injection apparatus 271 is pressed toward the side of the living body, so that the contact surface 14a of the guide portion 14 is brought into contact with the skin.

As shown in FIG. 34, when the lid 282 is pressed to be rotated until the contact surface 14a of the guide portion 14 contacts the skin, the force for the convex spherical surface 287a of the lid 282 to press the drug container 103 is larger than the penetration resistance of the seal member 133 to the second needle tip 9. Thus, the seal member 133 is penetrated by the second needle tip 9, and the needle puncture portion 132 of the drug container 103 is inserted into the insertion-hole 117 formed in the drug container mount 115. At this time, since the depth of the insertion-hole 117 is substantially equal to the protruding length of the needle puncture portion 132, the needle puncture portion 132 is inserted into insertion-hole 117 until the tip end of the needle puncture portion 132 abuts the bottom of the insertion-hole 117, and thereby the second needle tip 9 is inserted into the needle puncture portion 132. Since the protruding length of the second needle tip 9 within the insertion-hole 117 is set to a value smaller than the depth of the insertion-hole 117, the insertion length of the second needle tip 9 into the needle puncture portion 132 is smaller than the length of the tube hole 132a.
By performing the above operation, the liquid passage of the drug M enclosed in the drug container 103 into the needle tube 5 is completed.

Next, the user presses the pressing surface 288 of the lid 282 so that the lid 282 is further rotated toward the side of the lid receiver 181. Consequently, the convex spherical surface 287a of the lid 282 presses the first member 135 toward the side of the second member 136 while maintaining a state while the drug injection apparatus 271 is pressed against the living body. Thus, the inner pressure of the container body 131 increases, and therefore the drug M enclosed in the container body 131 is discharged into the living body from the first needle tip 8 through the second needle tip 9 of the needle tube 5.

Further, as shown in 35, the convex spherical surface 287a of the lid 282 fits the concave spherical surface 16a of the mounting surface 22 when the lid 282 is closed with respect to the lid receiver 181, and the locking portion 282a of the lid 282 is locked by the locked portion 184 of the lid receiver 181. Thus, since the first member 135 of the container body 131 is brought into close contact with the second member 136, the drug M in the container body 131 is completely discharged, and the administration of the drug M is completed.

In the present embodiment, the second needle tip 9 does not project above the mounting surface 22 of the drug container mount 115, and the liquid passage of the drug M into the needle tube 5 is performed by causing the needle puncture portion 132, which projects from the drug container 103, to be inserted into the insertion-hole 117 of the drug container mount 115. Thus, even if the first member 135 of the container body 131 bows toward the side of the second member 136 due to closing the lid 282, the second needle tip 9 will not penetrate through the first member 135 of the container body 131. In other words, the insertion-hole 117 functions as a contact avoiding portion for avoiding contact between the second needle tip 9 of the needle tube 5 and the convex spherical surface 287a of the lid 282. Thus, the drug will not be inhibited from being discharged from the drug container.
Further, the same advantages as those of the sixth embodiment can be achieved.

### 10. Tenth embodiment

### [Drug injection apparatus]

Next, a drug container according to a tenth embodiment of the present invention, and a drug injection apparatus which uses the drug container will be described below.
FIG. 36 is a cross-sectional view showing the configuration of a drug injection apparatus 301 of the ninth embodiment, wherein a drug container 143 and an injection needle assembly 272 are separated from each other.

The present embodiment is constituted by the injection needle assembly 272 of the ninth embodiment (see FIG. 32) and the drug container 143 of the fifth embodiment (see FIG. 18). In FIG. 36, components corresponding to those of FIG. 32 and FIG. 18 are denoted by the same reference numerals, and the explanation thereof will not be repeated.

### [Method for using drug injection apparatus]

Next, a method for using the drug injection apparatus 301 of the present embodiment will be described below.
FIG. 37 is a cross-sectional view showing a state where the drug container 143 is mounted on the injection needle assembly 272, and where the second needle tip 9 is penetrated through the seal member 153 of the drug container 143 so as to be inserted into the needle puncture portion 152. FIG. 38 is a cross-sectional view showing a state where the first needle tip 8 is stuck into a living body. FIG. 39 is a cross-sectional view showing a state where administration of the drug M performed by the drug injection apparatus 301 has been completed.

Before being used, the drug injection apparatus 301 is in a state where the injection needle assembly 272 and the drug container 143 are separated from each other (see FIG. 36). Thus, when using the drug injection apparatus 301, first, the drug container 143 is mounted on the mounting surface 22 of the drug container mount 115 so that the seal member 153 of the drug container 143 is abutted against the second needle tip 9. Since the seal member 153 has a certain level of resistance to the force for the second needle tip 9 to try to penetrate through the seal member 153, the seal member 153 will not be penetrated by the second needle tip 9 at the stage where the drug container 143 has been mounted on the mounting surface 22.

Further, the user presses the flange piece 159 of the drug container 143 from the side of the first member 135, and thereby the drug container 143 is moved toward the side of the mounting surface 22 so that the needle puncture portion 152 of the drug container 143 is inserted into the insertion-hole 117. Thus, as shown in FIG. 37, the second needle tip 9 is penetrated through the seal member 153 so as to be inserted into the needle puncture portion 152 of the drug container 143.
Thus, the drug container 143 is set to the injection needle assembly 272, and thereby the preparation of the drug injection apparatus 301 is completed.

At this time, since the depth of the insertion-hole 117 is substantially equal to the protruding length of the needle puncture portion 152, the tip end of the needle puncture portion 152 abuts the bottom of the insertion-hole 117, and the second member 155 of the drug container 143 also abuts the mounting surface 22. Since the protruding length of the second needle tip 9 within the insertion-hole 117 is set to a value smaller than the length of the tube hole 152a, the insertion length of the second needle tip 9 into the needle puncture portion 152 is smaller than the length of the tube hole 152a of the needle puncture portion 152, and therefore the second needle tip 9 will not project from the surface of the second member 155 on the side of the first member 135. Thus, the drug injection apparatus 301 is in a state where liquid passage of the drug M enclosed in the drug container 143 into the needle tube 5 is performed.

However, when the liquid passage of the drug M is into the needle tube 5 is being performed, since the user presses the flange piece 159 of the drug container 143 to move the drug container 143 toward the side of the mounting surface 22, the liquid chamber 156 is not pressed. Thus, the drug M of the liquid chamber 156 can be prevented from being discharged from the first needle tip 8. Further, in the present embodiment, since the second member 155 of the drug container 143 is formed of an inflexible material, the drug container 143 can be simply moved toward the side of the mounting surface 22 by pressing the flange piece 159.

Next, the end face 13a of the stabilizer 13 is caused to face the skin and the first needle tip 8 of the needle tube 5 is brought close to the skin to be stuck. Next, the user grips a portion other than the drug container 143 (for example, the side portion of the needle-holding portion 11) to press the drug injection apparatus 301 in the direction in which the first needle tip 8 is stuck into the living body. The injection needle assembly 272 is caused to substantially perpendicularly move toward the side of the skin, so that the first needle tip 8 is stuck into the skin, and the stabilizer 13 is pressed until the contact surface 14a of the guide portion 14 contacts the skin.
Thus, as shown in FIG. 38, the first needle tip 8 is stuck into the living body.

Next, the user presses the pressing surface 288 of the lid 282 so that the lid 282 is further rotated toward the side of the lid receiver 181. Thus, the first member 135 of the container body 151 is pressed toward the side of the second member 155 by the convex spherical surface 287a of the lid 282. Consequently, the inner pressure of the container body 151 increases, so that the drug M enclosed in the container body 151 is discharged into the living body from the first needle tip 8 through the second needle tip 9 of the needle tube 5.

Further, as shown in 39, the convex spherical surface 287a of the lid 282 fits the concave spherical surface 16a of the mounting surface 22 when the lid 282 is closed with respect to the lid receiver 181, and the locking portion 282a of the lid 282 is locked by the locked portion 184 of the lid receiver 181. Thus, since the first member 135 of the container body 151 is brought into close contact with the side of the second member 155, the drug M in the container body 151 is completely discharged, and the administration of the drug M is completed.

In the drug injection apparatus 301 of the present embodiment, since the surface of the drug container 143 on the side facing the drug container mount 115 is formed of an inflexible material in a curved shape, it is unstable on the mounting surface 22 before the second needle tip 9 penetrates through the seal member 153. Thus, liquid passage has been completed before the first needle tip 8 is stuck into the living body, and thereafter, administration of the drug can be stably performed by sticking the first needle tip 8 into the living body.
Further, the same advantages as those of the sixth to ninth embodiments can be achieved.

In the sixth to tenth embodiments, although the mounting surface of the drug container mount in the injection needle assembly is formed in a curved shape, the mounting surface may also be formed as a flat surface. In such a case, the surface of the lid facing the mounting surface is also formed as a flat surface, and thereby after administering drug, the residual liquid in the drug container can be reduced. Further, since the mounting surface of the drug container mount is formed in a curved shape, when mounting the drug container on the drug container mount, the drug container can be more stably mounted, and therefore safety in use can be ensured.

Although the sixth to tenth embodiments are described based on an example in which the lid has a disk-like shape and the lid receiver has a circular tube shape; however, the lid and the lid receiver are not limited thereto, but may respectively have polygonal plate shape and polygonal tube shape in plan view. Further, although the lid receiver is formed extending from the needle-holding portion, the lid receiver may also be formed extending from the drug container mount.

The embodiments of the drug injection apparatus of the present invention, including the advantages thereof, have been described above. However, the drug injection apparatus of present invention is not limited to the embodiments described above, and various modifications can be made without departing from the spirit and scope of the present invention.

The first to tenth embodiments are described based on an example in which the drug injection apparatus is the one used in the case where a drug is injected into the upper layer of the skin. However, the drug injection apparatus of the present invention may also be used to inject a drug into other sites of the living body such as subcutaneous tissue, muscle and the like, depending on the setting of the protruding length L on the side of the first needle tip 8.

Further, in the first to tenth embodiments, although the needle tube 5 is fixed to the needle-holding portion 11 by using an adhesive, the needle tube of the present invention may also be fixed to the needle-holding portion by other fixing methods such as high-frequency fusion, press fitting and the like.

Further, in the first to tenth embodiments, although the stabilizer 13, the guide portion 14 and the drug container mount are formed integrally with the needle-holding portion, the stabilizer, the guide portion and the drug container mount may also be respectively formed separately from the needle-holding portion 11, so that the needle hub is formed by assembling the separated components.

Further, in the first to tenth embodiments, although the adjusting portion 12 is formed integrally with the needle-holding portion 11, the adjusting portion may also be formed separately from the needle-holding portion and fixed to the needle tube.

### Explanation of Reference Numerals

1, 41, 71, 101, 141, 171, 201, 241, 271, 301 drug injection apparatus
10 gripping portion
2, 42, 72, 102, 172, 202, 242, 272 injection needle assembly
3, 43, 73, 103, 143, 173 drug container
5 needle tube
6, 46, 76, 106, 176, 206, 246, 276 needle hub
8 first needle tip
9 second needle tip
11 needle-holding portion
12 adjusting portion
12a needle-protruding surface
13 stabilizer
13a end face
4 guide portion
14a contact surface
15, 55, 85, 115 drug container mount
22, 82 mounting surface
23, 83 locking piece
31, 61, 91, 131, 151, 191 container body
32, 133, 153, 192 seal member
33 protective plate (penetration avoiding portion)
96a, 195a needle housing portion (penetration avoiding portion)
177 insertion-hole
132, 152 needle puncture portion
181, 211 lid receiver
182, 212, 252, 282 lid
M drug

## Claims

1. A drug injection apparatus comprising:
a drug container having a drug filled therein;
a needle tube having a first needle tip to be stuck into a living body and a second needle tip to be inserted into the drug container;
a needle-holding portion which holds a central portion of the needle tube; and
a drug container mount which is arranged on the second needle tip side of the needle-holding portion and from which the second needle tip projects,
wherein the drug container comprises:
a container body having a contact portion to be penetrated by the second needle tip and to be brought into contact with the drug container mount, and a pressing portion that faces the contact portion, wherein the pressing portion is to be pressed toward the side of the contact portion so as to deform;
a seal member attached to a portion of the contact portion to be penetrated by the second needle tip, and adapted to be brought into close contact with the circumferential surface of the needle tube in a liquid-tight manner; and
a penetration avoiding portion adapted to avoid the pressing portion from being penetrated by second needle tip inserted into the container body.

2. The drug injection apparatus according to claim 1,
wherein the pressing portion of the container body is formed of a flexible material, and
wherein the penetration avoiding portion is either an abutting portion arranged in the pressing portion and adapted to abut the second needle tip inserted into the container body or a recessed portion arranged in the pressing portion and adapted to house the second needle tip.

3. The drug injection apparatus according to claim 1 or 2,
wherein the resisting force of the seal member against the insertion of the second needle tip is larger than the resisting force of the living body against the insertion of the first needle tip.

4. The drug injection apparatus according to claim 2,
wherein the pressing portion is formed so that when a predetermined pressure is applied to the pressing portion, the pressing portion will be buckled so as to become convex toward the side of the contact portion.

5. The drug injection apparatus according to claim 1,
wherein the drug container mount has a spherical shaped mounting surface to be contacted by the contact portion of the drug container.

6. The drug injection apparatus according to claim 1,
wherein the contact portion of the container body is formed of a flexible material, and
wherein the pressing portion is formed of a material so that even the pressing portion abuts the second needle tip, the pressing portion will not be penetrated the second needle tip, and also serves as the penetration avoiding portion.

7. The drug injection apparatus according to claim 1,
wherein the contact portion of the container body is inflexible, is formed in a vessel-like shape opening toward the side of the pressing portion, and has a through-hole sealed by the seal member, and
wherein the pressing portion is formed of a flexible material.

8. The drug injection apparatus according to claim 1,
wherein the drug container mount has a locking piece to prevent the drug container from being moved in directions perpendicular to the pressing direction.

9. The drug injection apparatus according to claim 1, further comprising:
an adjusting portion arranged around the side of the first needle tip and having a needle-protruding surface to abut the skin when the first needle tip is stuck into the living body.

10. The drug injection apparatus according to claim 9,
wherein the adjusting portion is formed integrally with the needle-holding portion.

11. A drug injection apparatus comprising:
a drug container having a drug filled therein;
a needle tube having a first needle tip to be stuck into a living body and a second needle tip to be inserted into the drug container;
a needle-holding portion which holds a central portion of the needle tube; and
a drug container mount which is arranged on the second needle tip side of the needle-holding portion and on which the drug container is to be mounted,
wherein the drug container comprises:
a container body in which a liquid chamber for being filled with the drug is formed by joining a sheet-like first member and a sheet-like second member;
a needle puncture portion which projects outward from the second member and into which the second needle tip is to be inserted; and
a seal member attached to an end portion of the needle puncture portion and adapted to be penetrated by the second needle tip,
wherein the first member is pressed toward the second member, and thereby the drug filled in the liquid chamber is discharged through the needle tube penetrated through the seal member and inserted into the needle puncture portion.

12. The drug injection apparatus according to claim 11,
wherein the needle tube is held by the needle-holding portion so that when the second needle tip is inserted into the needle puncture portion, the second needle tip will stop in the needle puncture portion.

13. The drug injection apparatus according to claim 11 or 12,
wherein the drug container mount has a mounting surface on which the drug container is to be mounted, and an insertion-hole which is formed in the central portion of the mounting surface so as to surround the second needle tip of the needle tube and into which the needle puncture portion is inserted, and
wherein the second needle tip is held by the needle-holding portion so as not to reach the mounting surface.

14. The drug injection apparatus according to claim 11,
wherein the second member is formed of a flexible material, and
wherein the needle puncture portion has an inflexible guard portion that surrounds a portion to which the seal member is not attached.

15. The drug injection apparatus according to claim 14,
wherein, when the drug container is mounted on the drug container mount, the second needle tip will abut but not penetrate through the seal member.

16. The drug injection apparatus according to claim 15,
wherein the resisting force of the seal member against the insertion of the second needle tip is larger than the resisting force of the living body against the insertion of the first needle tip.

17. The drug injection apparatus according to claim 11,
wherein the first member is formed of a flexible material, and the second member is formed of an inflexible material.

18. The drug injection apparatus according to claim 11,
wherein the direction in which the first member is pressed toward the second member coincides with the axial direction of the needle tube inserted into the needle puncture portion.

19. The drug injection apparatus according to claim 1 or 11,
wherein the drug container mount has a mounting surface on which the drug container is to be mounted, and
wherein the drug injection apparatus further comprises a lid which is formed so as to be able to be rotated with respect to the mounting surface of the drug container mount, and which is either adapted to be rotated toward the side of the mounting surface to thereby press the drug container mounted on the mounting surface, or adapted to be rotated toward the side of the mounting surface so as to be closed with respect to the drug container mount and then deformed to thereby press the drug container.

20. The injection needle assembly according to claim 19,
either the drug container mount or the lid is provided with a contact avoiding portion adapted to avoid the lid from being contacted by the second needle tip of the needle tube, wherein the contact avoiding portion is formed in a recessed shape continuing from either the drug container mount or the lid.

21. The drug injection apparatus according to claim 20,
wherein, when the contact avoiding portion is formed in the lid, the drug container has a penetration avoiding portion which projects toward the side of the lid and which is to be inserted into the contact avoiding portion.

22. The drug injection apparatus according to claim 20,
wherein, when the contact avoiding portion is formed in the drug container mount, the drug container has a needle puncture portion which projects toward the side of the drug container mount and into which the second needle tip is to be inserted, the seal member is attached to an end portion of the needle puncture portion, the drug container further has an inflexible guard portion that surrounds a portion to which the seal member is not attached, and the needle puncture portion is to be inserted into the contact avoiding portion.

23. The injection needle assembly according to claim 19, further comprising:
a lid receiver for receiving the rotated lid, the lid receiver being arranged around the drug container mount,
wherein, when a locking portion arranged in the lid is locked by a locked portion arranged in the lid receiver, the locking portion is locked step-by-step by the locked portion along with the rotation of the lid.

24. The injection needle assembly according to claim 19,
wherein the mounting surface of the drug container mount is formed so as to curve toward the side of the lid from the central portion toward the outer side,
wherein the lid is formed so as to curve toward the side opposite to the side of the mounting surface from the central portion toward the outer side, and
wherein, when the lid is closed with respect to the drug container mount, the lid fits the drug container mount.

25. The injection needle assembly according to claim 19,
wherein the lid is formed so that when a predetermined pressure is applied to the lid, the lid will be buckled so as to become convex toward the side of the drug container mount.

26. A drug container comprising:
a container body in which a liquid chamber for being filled with a drug is formed by joining a sheet-like first member and a sheet-like second member;
a needle puncture portion which projects from the second member and into which a needle tube is to be inserted; and
a seal member attached to an end portion of the needle puncture portion and adapted to be penetrated by the needle tube,
wherein the first member is pressed toward the second member, and thereby the drug filled in the liquid chamber is discharged through the needle tube penetrated through the seal member and inserted into the needle puncture portion.

27. The drug container according to claim 26,
wherein the second member is formed of a flexible material, and
wherein the needle puncture portion has an inflexible guard portion that surrounds a portion to which the seal member is not attached.

28. The drug container according to claim 26,
wherein the first member is formed of a flexible material, and the second member is formed of an inflexible material.

29. The drug container according to claim 26,
wherein the direction in which the first member is pressed toward the second member coincides with the direction in which the axial direction of the needle tube inserted into the needle puncture portion.

30. The drug container according to claim 26,
wherein both the portion of the first member and the portion of the second member corresponding to the liquid chamber are each formed in a dome-like shape and are arranged so as to face each other.
